# EUROPEAN PATENT APPLICATION

(11) **EP 2 230 243 A1**
(43) Date of publication of application: **22.09.2010**
(21) Application number: 10154458.3
(22) Date of filing: 24.02.2010
(51) Int. Cl.: C07D 495/04, A61K 31/197, A61P 25/00

(54) **Pharmaceutical compounds**

(30) Priority: 25.02.2009 IT MI20090261
(71) Applicant: Neuroscienze Pharmaness S.C. A R.L., 09010 Pula (CA) (IT)
(72) Inventor: Lazzari, Paolo, 09010, Pula (Gagliari) (IT); Loriga, Giovanni, 07100, Sassari (IT); Manca, Ilaria, 07100, Sassari (IT); Pinna, Gerard Aime, 07100, Sassari (IT)
(74) Representative: Sama, Daniele

(57) **Abstract**

Condensed tricyclic pyrazole compounds having affinity for the CB1 and/or CB2 cannabinoidergic receptors, with activity both on the peripheral and central nervous system, of formula (I) : 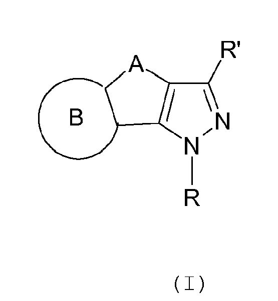 wherein:
A represents a group selected from -(CH₂)ₜ-, -(CH₂)ᵣ-O-(CH₂)ₛ- and -(CH₂)ᵣ-S(O)ₚ-(CH₂)ₛ-
B is an heteroaryl,
R is a group selected from heteroaryl, heteroarylalkyl, aryl, arylalkyl, arylalkenyl or bivalent aliphatic chain,
R' is a group selected from the following:
R'₁: a substituent bearing a keto group of formula -C(O)-(Z')ᵥ-Z"
R'₂: a substituent having an hydroxylic function of formula -CH(OH)-(Z')ᵥ-Z",
R'₃: an amide substituent of formula -C(O)-NH-(Z')ᵥ-T'.

## Description

The present invention relates to pyrazole derivatives having affinity for the CB1 and/or CB2 cannabinoidergic receptors, the correspondig solvates and pharmaceutically acceptable salts and the pharmaceutical compositions containing them.

More specifically the present invention relates to condensed tricyclic pyrazole derivatives having affinity for the CB1 and/or CB2 cannabinoidergic receptors, acting both on the peripheral and central nervous system, and which can also be formulated under the form of microemulsions.

It is well known that the microemulsions are widely requested by users since they provide formulation systems which are stable in time. Besides, when in microemulsions a phase separation occurs, the initial system can be restored by mild stirring when the pharmaceutical compound is formulated in a microemulsion.

Cannabinoids are compounds derived from *Cannabis sativa,* commonly known as marijuana. Among the at least 66 cannabinoid compounds characterizing marijuana, tetrahydrocannabinols (THC) and Δ⁹-tetrahydrocannabinol (Δ⁹-THC) in particular, are considered to be those most active. The properties which have brought to the use of marijuana as therapeutic agent of natural origin both in mammals and in human beings have been correlated to said compounds. Said properties are the following: the analgesic effect, the antiinflammatory activity, the reduction of the blood and intraocular pressure, the antiemetic activity. To tetrahydrocannabinols the negative effects which are associated to the marijuana use have furthermore been correlated, with particolar reference to the psychological distortion of perception, to the motory coordination loss, to the euphoria, to the sedative effect. The pharmacological action of cannabinoids appears directly correlated to their affinity towards two different classes of specific receptors belonging to the receptor family "G protein-coupled": the CB1 receptors, located in the central nervous system besides that in peripheral tissues, and the CB2 receptors, found in the cerebellum (Q.J.Lu et al.; Visual Neurosci.; 2000, 17,9 1-95) but which are mostly found in peripheral tissues (M.Glass; Progr. Neuro-Psychopharmacol. & Biol. Psychiat.; 2001, 25, 743-765). In the brain, the CB1 receptors are abundantly expressed in the hippocampus, in the cortical regions, in the cerebellum and inside the basal ganglia. Among the peripheral tissues wherein the CB1 receptors have been found, the testicles, the small intestine, the vesica, the deferent duct can be mentioned. The CB1 receptors have been furthermore identified both in the rat eye and in the human eye, both in the retina and in the iris and in the ciliary body (A. Porcella et al.; Molecular Brain Research; 1998, 58, 240-245; A. Porcella et al.; European Journal of Neuroscience; 2000, 12, 1123-1127). The CB2 receptors are on the contrary prevailingly located in the marginal zones of the spleen, in tonsils, besides in several cells of the immune system, as macrophages, monocytes, the cells of the bone marrow, of thymus and of pancreas. Other cells of the immune system wherein the CB2 receptors are significantly present are T4 and T8 cells, the polymorphonucleated leucoocytes, in particular the cells called "natural killers" and lymphocytes B.

The compounds able to interact, as agonists or antagonists, with the CB2 receptors can therefore be used in the treatment of diseases wherein immune system cells or immune disorders are involved. The activation (modulation) of the CB2 receptors is also important in the treatment of other diseases, such as in the treatment of osteoporosis, renal ischaemia, pain, neuropathic pain, post-surgery pain, inflammatory conditions, amyotrophic sclerosis.

The compounds with affinity for the CB1 receptors can be used in the treatment of eye diseases such as glaucoma, pulmonary diseases, as asthma and chronic bronchitis, inflammations as arthritis, allergies and allergic reactions, such as allergic rhinitis, contact dermatitis, allergic conjunctivitis. Said compounds can also be used in the treatment of pain, in conditions of anxiety, behaviour disorders, delirium conditions, psychotic problems in general, furthermore for the treatment of schizophrenia, depression and in the treatment of drug and/or alcohol abuse, (for example alcoholism and tabagism). The same compounds can also be used to combat vomit, nausea, vertigoes, especially in the case of patients subjected to chemotherapy; in the treatment of neuropathies, hemicrania, stress, psychosomatic origin diseases, epilepsy, Tourette syndrome, Parkinson disease, Huntington disease, Alzheimer disease, senile dementia and in the case of cognition disorders and memory loss. Further applications of the compounds having affinity towards the CB1 receptors are the treatment of pathologies related to appetite disorders (obesity, bulimia), pathologies of the gastrointestinal tract and of the bladder, cardiovascular diseases, urinary and fertility disorders, neuroinflammatory pathologies, such as multiple sclerosis, Guillain-Barré syndrome, viral encefalitis. For example some CB1 agonist active principles are successfully used in the treatment of nausea and vomit associated to chemotherapy and in the appetite whetting in AIDS patients. Compounds having antagonist activity towards the CB1 receptors can be used for example in the treatment of psychosis, anxiety, depression, schizophrenia, obesity, neurological diseases (for example: dementia, Parkinson disease, Alzheimer disease, epilepsy, Tourette syndrome), in conditions of memory loss, of central nervous system diseases involving the neurotransmission of cannabinoids, in fertility and erectile disorders, in the treatment of gastrointestinal and/or cardiovascular disorders.

The compounds which are effective in activating cannabinoid receptors show immunosuppressive activity and are used in the treatment of eye inflammatory conditions and autoimmune diseases, for instance uveitis and uveoretinitis (H. Xu et al., J. Leukocyte Biology, 82, 2007, 532-541). They are used also for treating retina neurodegeneration (G. Pryce et al., Brain 126 2003 2191-2202).

With reference to the wide pharmacological applications of cannabinoids, in the latest years several studies have been carried out for finding endocannabinoids and for the synthesis of new compounds capable of selectively interacting with the two subclasses of CB1 and CB2 cannabinoidergic receptors. Researches have led on the one hand to the identification of anandamide endocannabinoids (arachidonyl ethanolamide) and 2-arachidonyl glycerol, on the other hand to the preparation of different classes of synthesis compounds, agonist or antagonist towards the CB1 or CB2 receptors.

The class of the compounds having agonist activity towards the CB1 receptors (cannabimimetic activity) comprises both synthesis compounds with a basic structure directly derived from that of Δ⁹-THC, as (-)-11-OH-Δ⁸THC-dimethylheptyl (HU210) and nabilone, and compounds structurally different from Δ⁹-THC, as aminoalkylindols of the WIN 55,212-2 series (M. Pacheco et al.; J. Pharmacol. Exp. Ther.; 1991, 257, 1701-183) or as bicyclic cannabinols (non classic cannabinoids) which refer to the compound CP 55,940 (M. Glass; Progr. Neuro-Psychopharmacol. & Biol. Psychiat.; 2001, 25, 743-765). The compounds having cannabimimetic activity show in vivo the following effects: hypoactivity, hypothermia, analgesia and catalepsy (B.R. Martin et al.; Pharmacol. Biochem. Behav.; 1991, 40, 471-478; P.B. Smith et al.; J. Pharmacol. Exp. Ther.; 1994, 270, 219-227).

Clinical data have shown thah the CB1 antagonist pyrazole compound Rimonabant is effective in reducing both weight and metabolic and/or cardiovascular risk factors in patients with metabolic syndrome and/or dyslipidemia (J. P. Després et al., the New England Journal of Medicine, 2005, 353, 2121-2134, D. Tonstad, Nutrition, Metabolism and Cadiovascular Diseases 2006, 16, 156-162. The effectiveness of Rimonabant in reducing metabolic and/or cardiovascular risk factors has been shown also in patients with type 2 diabetes (A. J.Scheen et A1., Lancet 2006, 368 1660-1672).

Condensed tricyclic compounds having a high affinity for the cannabinoidergic receptors and, above all, a high selectivity for the CB1 receptors, are described in EP 1,230,244. Said compounds have the following general structure: 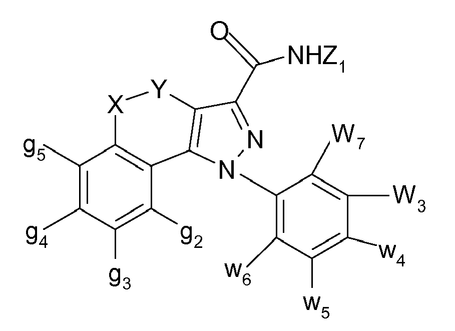 wherein Z₁, W₇, W₃, W₄, W_{5,} W_{6,} g₂, g_{3,} g₄, g₅ have different meanings; X-Y- represent a group selected from:
-(CH₂)_{d}-CH₂-, -CH₂-S(O)_{g}-, -S(O)_{g}-CH₂-, with d equal to 1 or 2, g equal to zero, 1 or 2. In the document no mention is made that the described compounds could be formulated under the form of microemulsion.

Condensed tricyclic compounds having a high affinity for the cannabinoidergic receptors and, above all, high selectivity for the CB2 receptors, are described in EP 1,230,222. The compounds described in this patent have the following general structure: 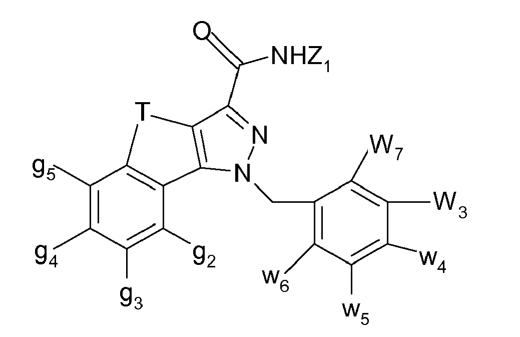 wherein: -T- represents a group -(CH₂)ₘ-, with m equal to 1 or 2; Z₁, W₇, W₃, W₄, W₅, W₆, g₂, g_{3,} g₄, g₅ have different meanings.

Also in this patent no mention is made that the compounds described could be formulated under the microemulsion form.

Another class of compounds having affinity towards the CB1 and/or CB2 receptors with a pyrazole basic structure wherein the pyrazole ring forms a part of a condensed tricyclic structure,is represented by the derivatives described in US 2005/0282,798. These derivatives act only on the CB1 and/or CB2 cannabinoidergic receptors at a peripheral level. The compounds are unable to show any activity on the central nervous system. They are not able to pass the haematoencephalic barrier.

One class of benzopyranopyrazolyl derivatives is described in USP 5,547,975 and shows the following general formula: 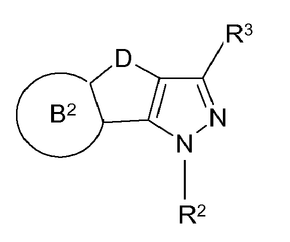 wherein B² and D have different meanings, R² is a group selected between aryl and heteroaryl, optionally substituted with different substituents, R³ is a group selected from hydrogen, halogen, haloalkyl, cyano, nitro, formyl, alkoxycarbonyl, carboxyl, carboxyalkyl, alkoxycarbonylalkyl, amidino, cyanoamidino, aminocarbonyl, alkoxy, alkoxyalkyl, aminocarbonylalkyl, N-alkylaminocarbonyl, N-arylamino-carbonyl, N,N-dialkylaminocarbonyl, N-alkyl-N-arylamino-carbonyl, alkylcarbonyl, alkylcarbonylalkyl, hydroxyalkyl, alkylthio, alkylsulphinyl, alkylsulphonyl, alkylthioalkyl, alkylsulphinylkyl, alkylsulphonylalkyl, N-alkylsulphamyl, N-arylsulphamyl, arylsulphonyl, N,N-dialkylsulphamyl, N-alkyl-N-arylsulphamyl, heterocycle.

These compounds are for the treatment of the inflammation or disorders related to inflammation. No mention is made that these compounds have affinity for the CB1 and/or CB2 cannabinoid receptors. In this patent no mention is made that the formulations of said compounds could be in the form of a microemulsion.

A further class of condensed tricyclic compounds containing a pyrazole ring is described in WO 03/070706. The described compounds have general formula: 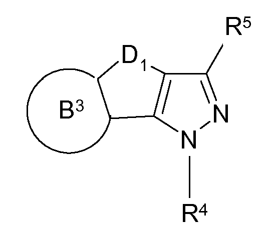
wherein D₁ has various meanings, B³ is heteroaryl, R⁴ is aryl or heteroaryl with a 5 or 6 atom ring, R⁵ is a group selected from amidine, alkylamino, aminoalkyl, NH₂, CONHR¹⁶, NHCOR⁶, CH₂-NH-COR⁶, being:
R¹⁶ a group selected from hydrogen, aryl, arylalkyl, alkyl, haloalkyl, alkenyl, alkynyl, hydroxyalkyl, aminoalkyl, alkoxy, alkoxyalkyl,
R⁶ is a group selected from hydrogen aryl, heteroaryl, alkyl, haloalkyl, alkenyl, alkynyl, hydroxyalkyl, aminoalkyl, alkylammonialkyl, alkoxy, alkoxyalkyl, heterocycloalkyl, heterocycle.

These compounds are described for the use in the treatment of cancer, inflammation and disorders related thereto. No indication is reported as to the affinity of the compounds for the CB1 and/or CB2 cannabinoid receptors. In this patent no mention is made that the formulations of said compounds could be in the form of microemulsions.

The need was felt to have available compounds having affinity for the CB1 and/or CB2 cannabinoidergic receptors having the following combination of properties:
- activity in vitro and in vivo on peripheral and on central nervous system,
- improved activity at a peripheral level compared to the prior art compounds, in particular for compounds having affinity for the CB1 cannabinoidergic receptors, said improved activity being shown at least in intraocular pressure reduction,
- suitable for being formulated into microemulsions.

An object of the present invention are condensed tricyclic pyrazole compounds having affinity for the CB1 and/or CB2 cannabinoidergic receptors, having activity both on the peripheral and on central nervous system, having formula (I): 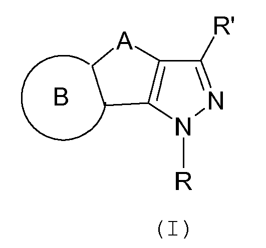 wherein:
A represents a group selected from -(CH₂)ₜ-, -(CH₂)ᵣ-O-(CH₂)ₛ- and -(CH₂)ᵣ-S(O)ₚ-(CH₂)ₛ- wherein
   t is an integer equal to 1, 2 or 3,
   p is an integer equal to 0, 1 or 2,
   r and s, equal to or different from each other, are integers equal to 0, 1 or 2 with the proviso that r+s is equal to 0, 1, 2 or 3,
B is an heteroaryl,
R is a group selected from heteroaryl, heteroarylalkyl, aryl, arylalkyl, arylalkenyl or a bivalent C₁-C₁₀ aliphatic chain, linear or branched when possible, wherein the chain end not linked to the nitrogen atom is linked to W, W being a group selected from hydrogen, halogen, isothiocyanate, CN, OH, OCH₃, NH₂, SO₂NH₂ or -CH=CH₂,
R' is a group selected from the following:
   R'₁: a substituent bearing a keto group of formula -C(O)-(Z')ᵥ-Z"
   wherein Z' is a bivalent C₁-C₈ aliphatic chain, Z" is selected from C₃-C₁₅ cycloalkyl, saturated or unsaturated heterocycle, aryl, heteroaryl,
   v is an integer equal to 1,
   when A is -(CH₂)ᵣ-O-(CH₂)ₛ- v=0, r and s being as defined above,
   R'₂: a substituent bearing an hydroxylic function, of formula -CH(OH)-(Z')ᵥ-Z",
   Z', v and Z" being as defined above,
   R'₃: an amide substituent of formula -C(O)-NH-(Z')ᵥ-T',
   Z' and v being as defined above and T' a group selected from:
      - C₃-C₁₅ cycloaklyl,
      - NR₁R₂,
         wherein R₁ and R₂, equal to or different from each other, have the following meanings: hydrogen, C₁-C₇ alkyl, C₁-C₇ haloalkyl, heteroaryl, heteroarylalkyl, aryl, arylalkyl or arylalkenyl,
         or
         R₁ and R₂ with the nitrogen atom form a saturated or unsaturated heterocycle, containing from 5 to 10 atoms,
      - C₃-C₁₅ heterocycloalkyl containing one or more heteroatoms, equal to or different from each other selected from N, O, S, with the proviso that Z' is linked to one carbon atom of the heterocycloalkyl ring, and excluding from the above formula when Z' is C₁-C₃ linear alkylene chain and T' has the following formula (IB): 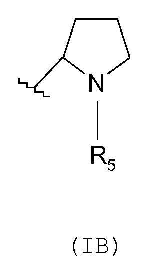 wherein R₅ represents a linear or branched when possibile C₁-C₃ alkyl,
      - aryl or heteroaryl, with the proviso that A is a
      - (CH₂)ᵣ-O-(CH₂)ₛ- group, r and s being as defined above. Preferably from formula (I) the compound (δα) is excluded

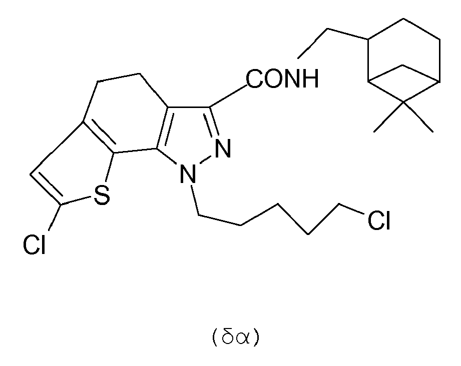

The compounds of formula (I) comprise also both the corresponding geometrical isomers and the stereoisomers and mixtures thereof. Furthermore the different atoms present in the compounds of formula (I) can be also in different isotopic forms, to allow the radiolabelling of said compounds.

B can be substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, SO₂NH₂, cyano, nitro, isothiocyanate, phenyl, cycloalkyl, saturated or unsaturated heterocycle, heteroaryl, amino optionally mono- or bisubstituted with a C₁-C₇ alkyl chain. The substituents phenyl, cycloalkyl, saturated or unsaturated heterocycle and heteroaryl are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, SO₂NH₂, cyano, isothiocyanate, phenyl, cycloalkyl, saturated or unsaturated heterocycle, heteroaryl, nitro, amino optionally mono- or bisubstituted with a C₁-C₇ alkyl chain.

When R is heteroaryl, heteroarylalkyl, aryl, arylalkyl or arylalkenyl, R can have one or more substituents, equal to or different from each other, selected from halogen, C₁-C₇ alkyl, C₁-C₇ alkylthio, C₁-C₇ alkoxy, C₁-C₇ haloalkyl, C₁-C₇ haloalkoxy, SO₂NH₂, cyano, nitro, phenyl, saturated or unsaturated heterocycle, amino optionally mono- or bisubstituted with a C₁-C₇ alkyl chain.

Z" can have one or more substituents, equal to or different from each other, selected from halogen, SO₂NH₂, C₁-C₇ alkyl, C₁-C₇ haloalkyl, C₁-C₇ haloalkoxy, C₁-C₇ alkylthio, C₁-C₇ alkoxy.

When T' is aryl, heteroaryl, C₃-C₁₅ cycloalkyl or C₃-C₁₅ heterocycloalkyl, T' can be substituted with one or more groups, equal to different from each other, selected from halogen, SO₂NH₂, C₁-C₇ alkyl, C₁-C₇ haloalkyl, C₁-C₇ haloalkoxy, C₁-C₇ alkylthio, C₁-C₇ alkoxy, phenyl, benzyl, the phenyl and benzyl substituents are optionally substituted with one or more groups, equal to or different from each other, selected from halogen, SO₂NH₂, C₁-C₇ alkyl, C₁-C₇ alkylthio, C₁-C₇ alkoxy, C₁-C₇ haloalkyl, C₁-C₇ haloalkoxy, cyano, nitro, isothiocyanate, phenyl, cycloalkyl, saturated or unsaturated heterocycle, amino optionally mono- or bisubstituted with a C₁-C₇ alkyl chain.

When R₁ and R₂ are heteroaryl, heteroarylalkyl, aryl, arylalkyl or arylalkenyl, or R₁ and R₂ with the nitrogen atom form one heterocycle, the aromatic rings or the heterocycle can be substituted with one or more groups equal to or different from each other, selected from halogen, SO₂NH₂, C₁-C₇ alkyl, C₁-C₇ haloalkyl, C₁-C₇ haloalkoxy, C₁-C₇ alkylthio, C₁-C₇ alkoxy, phenyl, benzyl, cyano, nitro, amino optionally mono- or bisubstituted with a C₁-C₇ alkyl chain, the phenyl and benzyl substituents are optionally substituted with one or more groups, equal to or different from each other, selected from halogen, SO₂NH₂, C₁-C₇ alkyl, C₁-C₇ alkylthio, C₁-C₇ alkoxy, C₁-C₇ haloalkyl, C₁-C₇ haloalkoxy, cyano, isothiocyanate, phenyl, cycloalkyl, saturated or unsaturated heterocycle, heteroaryl, nitro, amino optionally mono- or bisubstituted with a C₁-C₇ alkyl chain.

Where not otherwise specified, the following meanings are meant in the present invention:
by alkyl or alkyl chain it is meant a saturated C₁-C₂₀ hydrocarbon chain, linear or branched when possible,
by alkenyl or alkenyl cahin it is meant a mono- or polyunsaturated C₂-C₂₀ hydrocarbon chain, preferably mono-unsaturated, linear or branched when possible,
by cycloalkyl, wherein the ring or the rings do not contain unsaturations, it is meant an aliphatc monocyclic ring, having from 3 to 10, preferably from 4 to 9 carbon atoms, or a polycyclic structure from 7 to 19 carbon atoms,
by heterocyclalkyl and saturated heterocycle it is meant a cycloalkyl as defined above wherein one or more carbon atoms are substituted by heteroatoms, equal to or different from each other, selected from S, O, N; when the ring is monocyclic, preferably the heteroatoms are no more than 2,
by unsaturated heterocycle it is meant a cyclalkyl as defined above with one or more double bonds, with the proviso that the ring structure is not aromatic, and wherein at least one carbon atom is substituted by one heteroatom selected from S, O, N,
by halogen it is meant one atom selected from fluorine, chlorine, bromine, iodine,
by haloalkyl or haloalkyl chain it is meant an alkyl as defined above, wherein one or more hydrogen atoms are substituted with halogen atoms. Examples of haloalkyl are trifluoromethyl, 1-bromo-n-butyl, pentachlorethyl, etc.
by aryl it is meant an aromatic monocyclic radical, or a condensed aromatic polycyclic radical having from 6 to 20 carbon atoms,
by heteroaryl it is meant an aryl as defined above, except that the monocyclic radical is C₅-C₆ wherein at least one or more carbon atoms are substituted with one or more heteroatoms, equal to or different from each other, selected from S, O, N, when the radical is monocyclic preferably the heteroatoms are no more than 2,
by arylalkyl it is meant an alkyl as defined above, preferably C₁-C₇, linked to an aryl as defined above, for example benzyl,
by arylalkenyl it is meant an alkenyl as defined above linked to an aryl as defined above,
by heteroarylalkyl it is meant an alkyl as defined abve, preferably C₁-C₇, linked to an heteroaryl as defined above,
by bivalent aliphatic chain it is meant a C₁-C₂₀ aliphatic chain, preferably C₁-C₈, saturated or unsaturated, linear or branched when possible, having at each end a free valence, one or more hydrogen atoms of the chain can optionally be substituted with halogen atoms,
by compound having affinity towards the receptors it is meant a compound having in vitro and/or in vivo and/or in ex-vivo agonist, or antagonist, or partial agonist, or partial antagonist, or inverse agonist, or inverse antagonist, or inverse partial agonist activity towards the receptors. The meaning of said terms is well known to the skilled in the field.

The preferred compounds of formula (I) are those wherein A, R and R' are as defined above and B is a monocyclic heteroaryl.

The more preferred compounds of formula (I) are those wherein:
A is as defined above,
B is an heteroaryl with a 5 or 6 atom membered ring, optionally substituted with one or more groups, equal to or different from each other, selected from the following: halogen, C₁-C₇ alkyl, C₁-C₇ alkylthio, C₁-C₇ alkoxy, C₁-C₇ haloalkyl, C₁-C₇ haloalkoxy, SO₂NH₂, phenyl, heteroaryl, wherein said phenyl and heteroaryl substituents are optionally substituted with one or more groups, equal to or different from each other, selected from halogen, C₁-C₇ alkyl, C₁-C₇ alkoxy, SO₂NH₂, phenyl, heteroaryl,
R is selected from the following groups:
   - monocyclic heteroaryl, heteroarylalkyl, aryl, arylalkyl,
   - bivalent C₁-C₁₀ aliphatic chain, linear or branched when possible, wherein the chain end not linked to the nitrogen atom is linked to W, W being as defined above,
R' is a R'₁ or R'₃ group as defined above.

The still more preferred compounds of formula (I) are those wherein:
A is as defined above but excluding the meaning -(CH₂)ᵣ-S(O)ₚ-(CH₂)ₛ-,
B is an heteroaryl selected from the following: thiophene, pyridine, furan, oxazole, thiazole, imidazole, pyrazole, isoxazole, isothiazole, triazole, pyridazine, pyrimidine, pyrazine, triazine, pyrrole, said heteroaryls optionally substituted with one or more groups, equal to or different from each other, selected from: halogen, C₁-C₃ alkyl, C₁-C₃ alkylthio, C₁-C₃ alkoxy, C₁-C₃ haloalkyl, C₁-C₃ haloalkoxy, SO₂NH₂, phenyl, heteroaryl, amino optionally mono- or bisubstituted with a C₁-C₃ alkyl chain, wherein said phenyl and heteroaryl are optionally substituted with one or more groups, equal to or different from each other, selected from halogen, C₁-C₃ alkyl, C₁-C₃ alkoxy, SO₂NH₂, phenyl, heteroaryl,
R is selected from the following groups:
   - monocyclic aryl or monocyclic arylalkyl as defined above, optionally substituted with one or more substituents, equal to or different from each other, selected from halogen, C₁-C₃ alkyl, SO₂NH₂, C₁-C₃ alkoxy, C₁-C₃ alkylthio, C₁-C₃ haloalkyl, C₁-C₃ haloalkoxy, amino optionally mono- or bisubstituted with a C₁-C₃ alkyl chain,
   - bivalent C₄-C₁₀ aliphatic chain, linear or branched, wherein the chain end not linked to the nitrogen atom is linked to W, W being as defined above,
R' is selected from R'₁ and R'₃ with the proviso that Z' is a bivalent C₁-C₃ aliphatic chain, Z" a group selected from C₃-C₁₅ cycloalkyl, saturated or unsaturated heterocycle, monocyclic aryl or monocyclic heteroaryl, v and T' are as defined above.

The particularly preferred compounds of formula (I) are those wherein:
A is as defined above but excluding the meaning of -(CH₂)ᵣ-S(O)ₚ-(CH₂)ₛ-,
B is thiophene, optionally substituted with one or more groups, equal to or different from each other, selected from the following: halogen, C₁-C₃ alkyl, C₁-C₃ alkylthio, C₁-C₃ alkoxy, C₁-C₃ haloalkyl, C₁-C₃ haloalkoxy, SO₂NH₂, phenyl, heteroaryl, amino optionally mono- or bisubstituted with a C₁-C₃ alkyl chain, wherein said phenyl and heteroaryl are optionally substituted with one or more groups, equal to or different from each other, selected from halogen, C₁-C₃ alkyl, C₁-C₃ alkoxy, SO₂NH₂, phenyl, heteroaryl,
R is selected from the following groups:
   - phenyl or benzyl, optionally substituted with one or more substituents, equal to or different from each other, selected from halogen, C₁-C₃ alkyl, SO₂NH₂, C₁-C₃ alkoxy, C₁-C₃ alkylthio, C₁-C₃ haloalkyl, C₁-C₃ haloalkoxy, amino optionally mono- or bisubstituted with a C₁-C₃ alkyl chain,
   - bivalent C₄-C₁₀ aliphatic chain, linear or branched, wherein the chain end not linked to the nitrogen atom is linked to W, W being as defined above,
R' is selected between R'₁ and R'₃ with the proviso that:
Z' is selected between -CH₂- or -CH(C_{H}3)-,
Z" is a group selected from C₃-C₁₅ cycloalkyl, saturated or unsaturated heterocycle, monocyclic aryl, monocyclic heteroaryl,
T' and v are as defined above.

Examples of the compounds of the invention are the compounds of formula (I), having the following formulae from (X') to (XXXIII'): 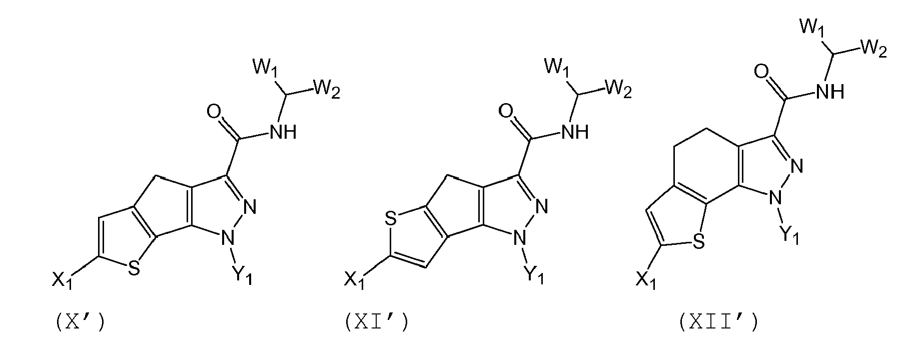 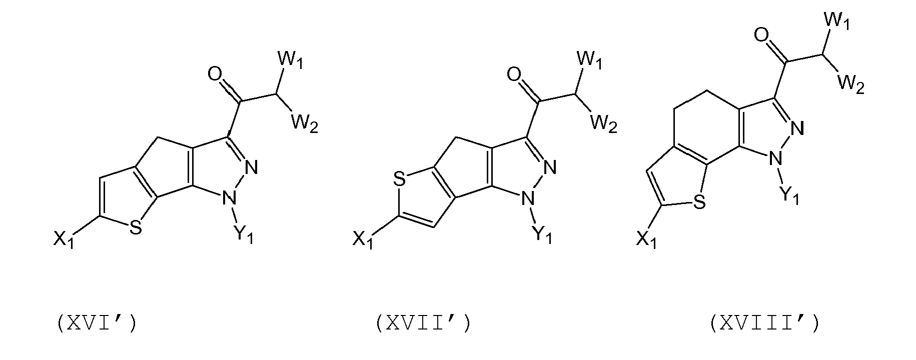 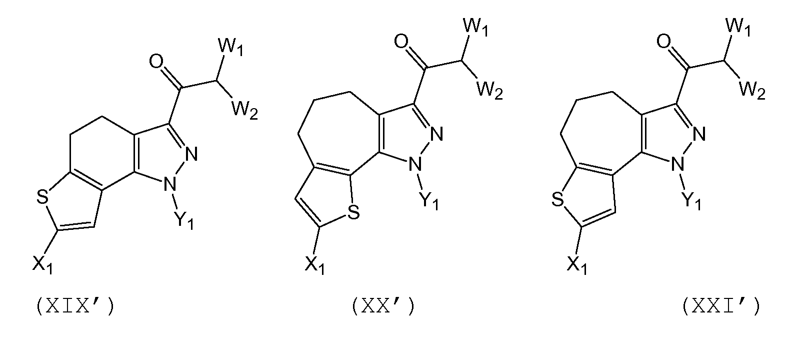 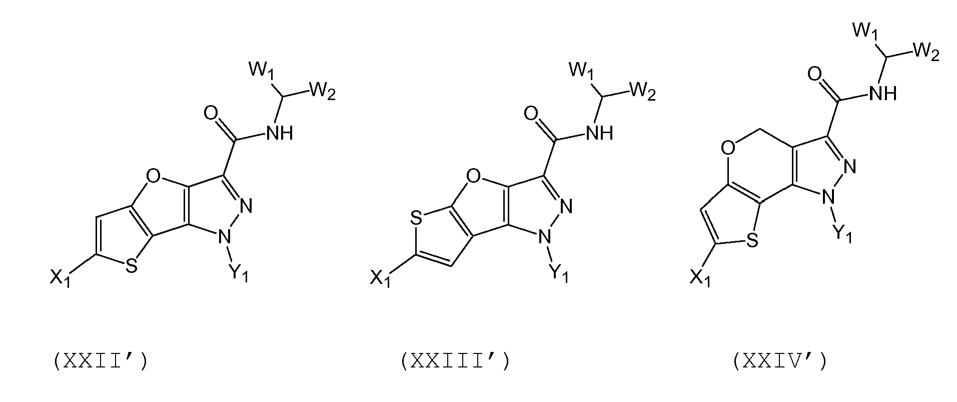 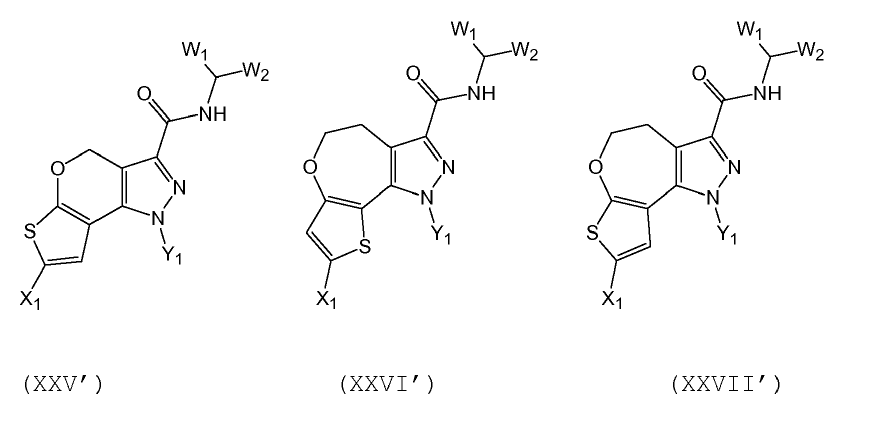 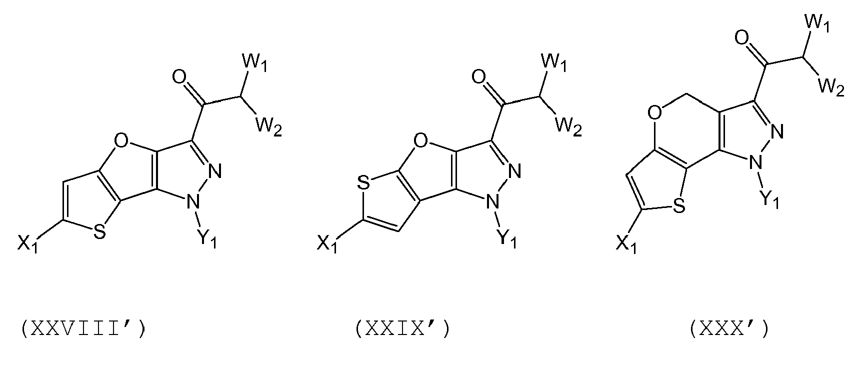 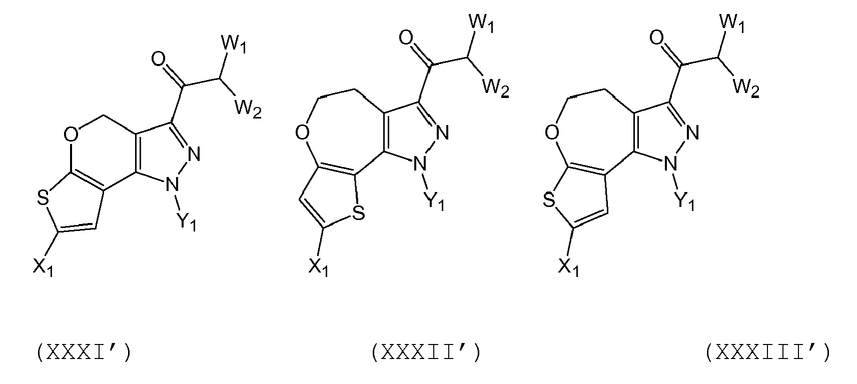 wherein in formulae (X') to (XXXIII'):
Y₁ is selected from: 2,4-dichlorophenyl, 2,4-difluorophenyl, 4-methylbenzyl or 5-chloropentyl,
X₁ is selected from: methyl, chlorine, bromine, fluorine, phenyl, 4-methylphenyl, 4-chlorophenyl, 4-bromophenyl, 4-fluorophenyl, thiophene,
W₁ is hydrogen or methyl,
W₂ is a group selected from the following: 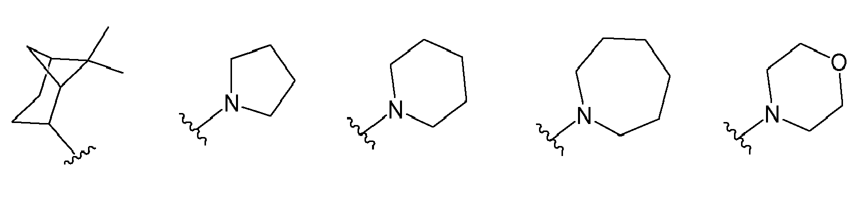 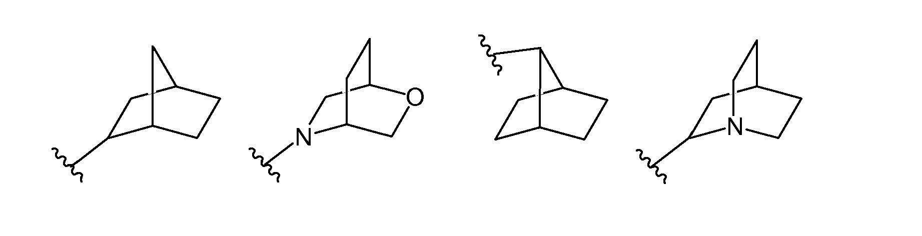 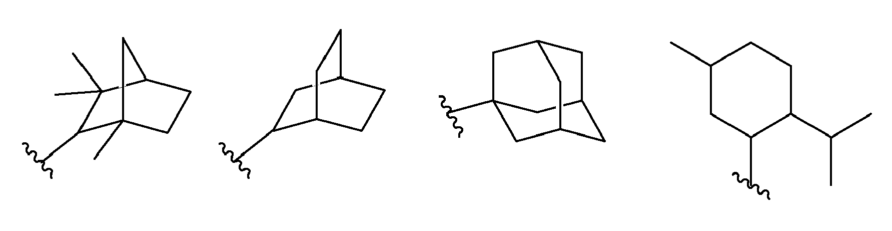 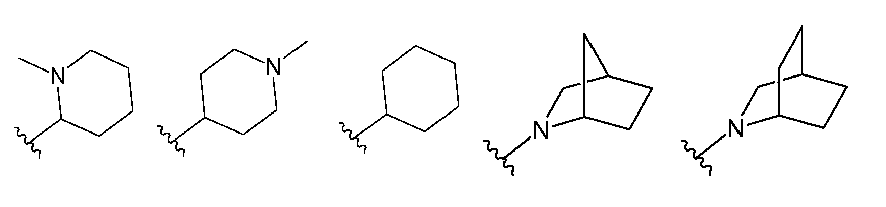 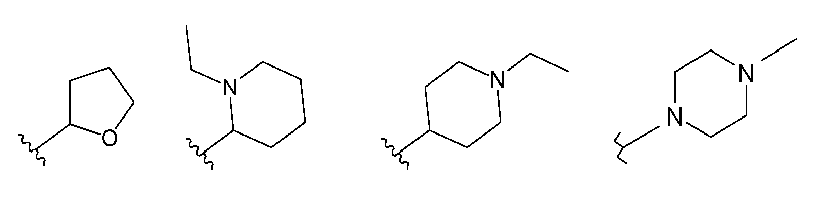 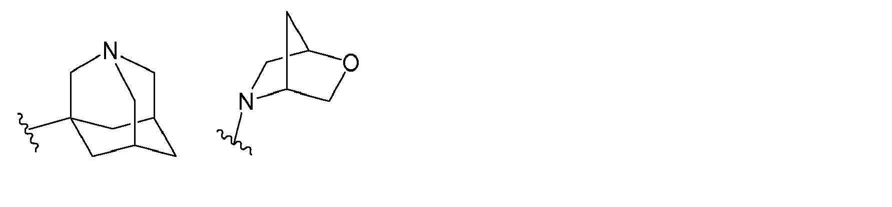 wherein in formulae (X') to (XXXIII') all the geometrical isomers and/or stereoisomers are comprised.

The still more preferred compounds of the invention are reported hereunder:

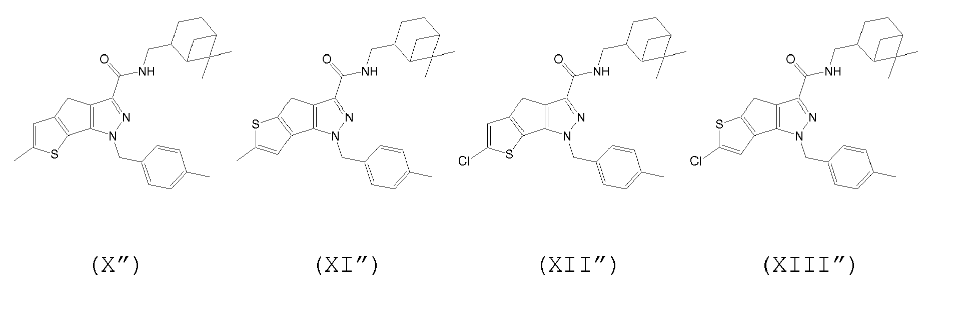

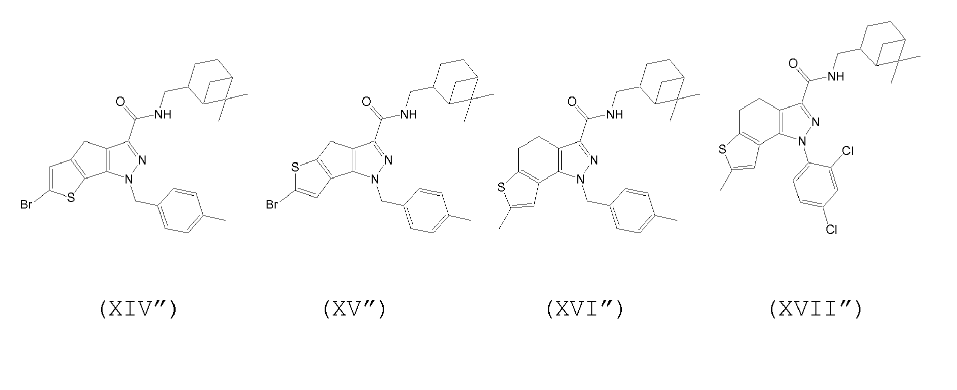

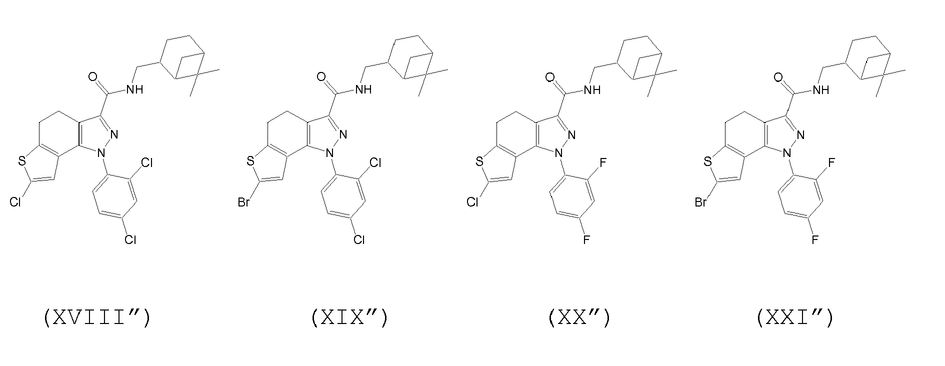

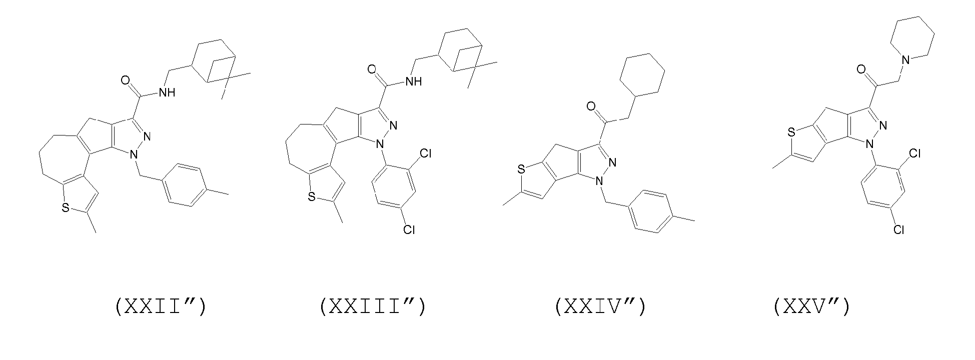

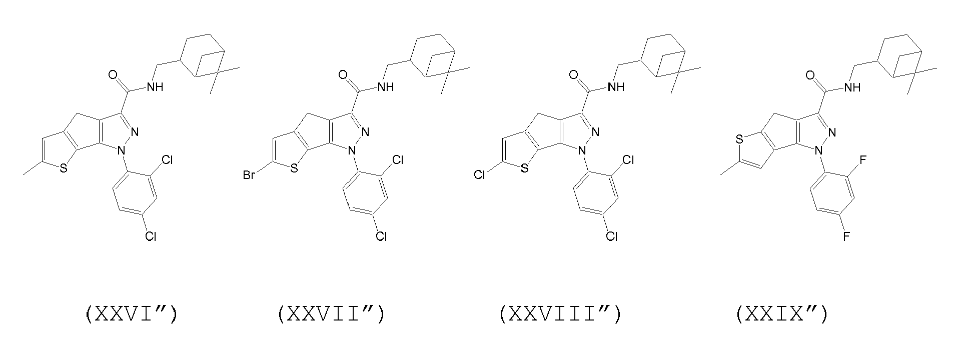

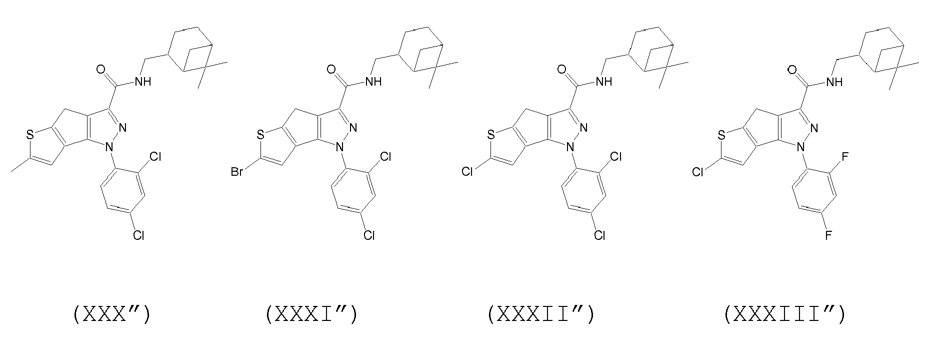

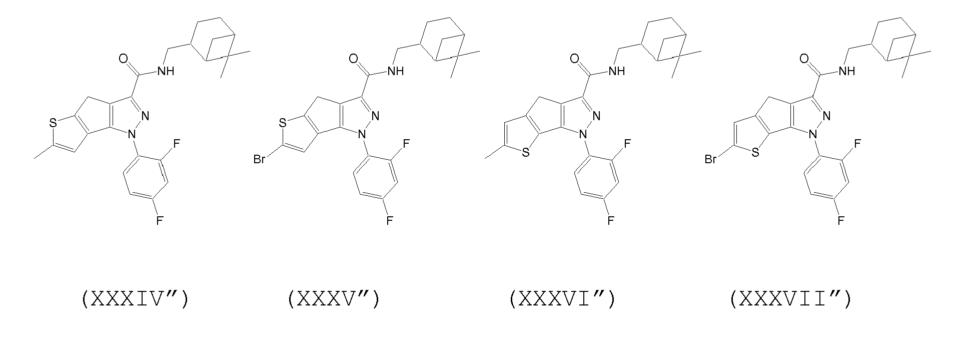

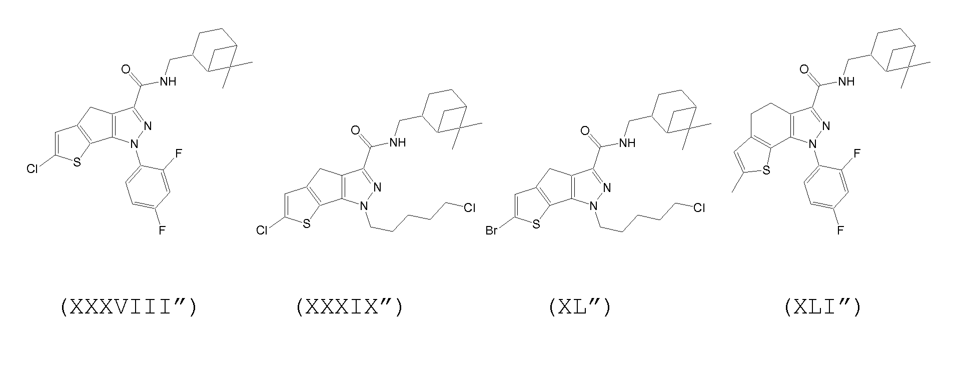

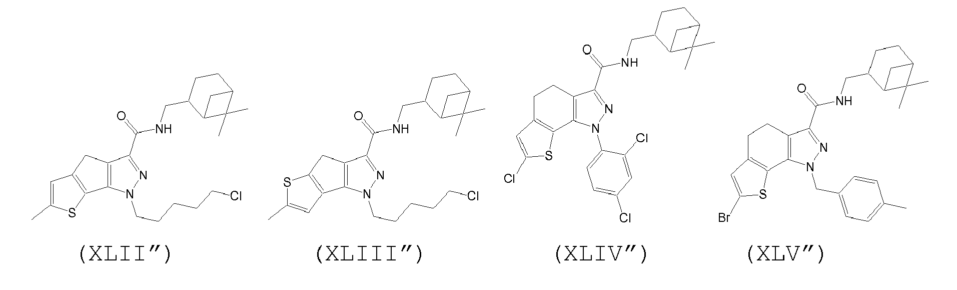

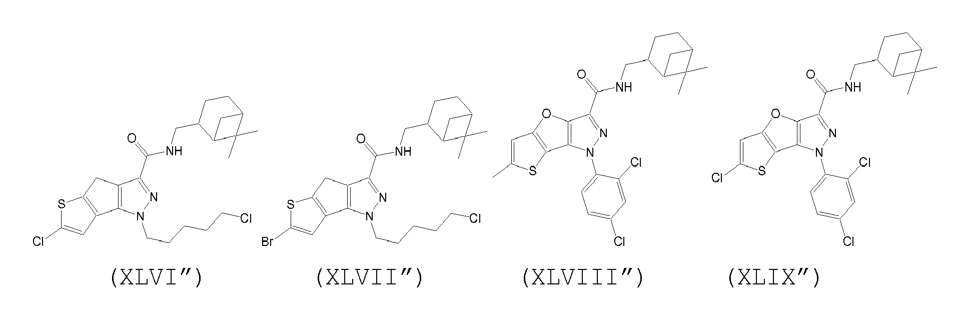

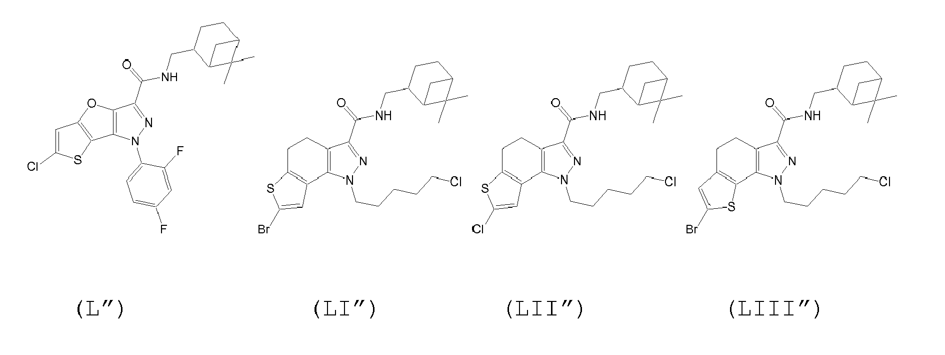

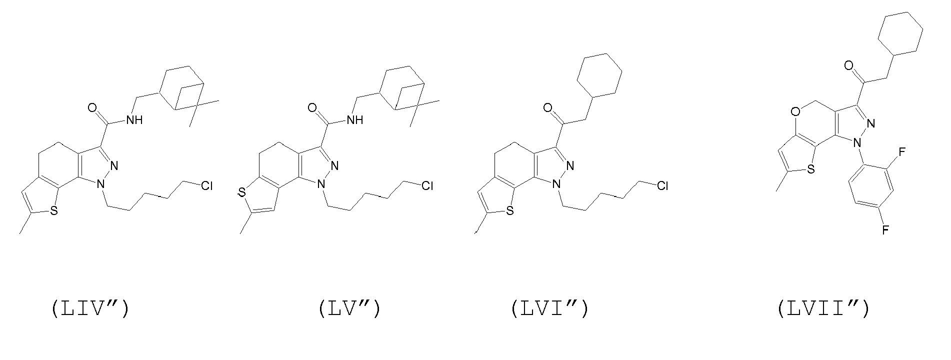

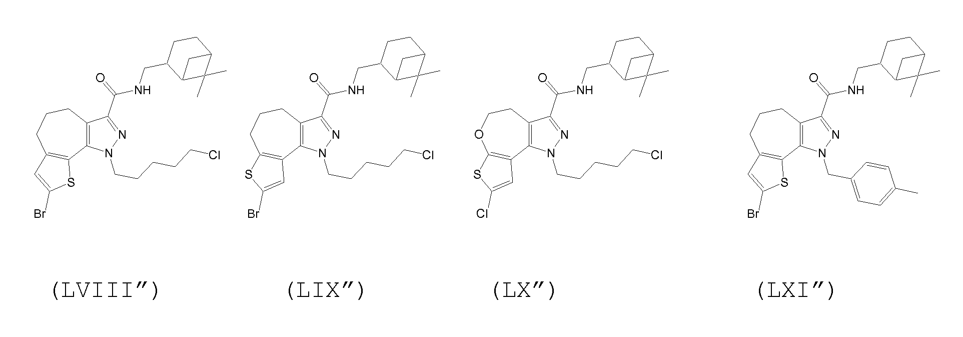

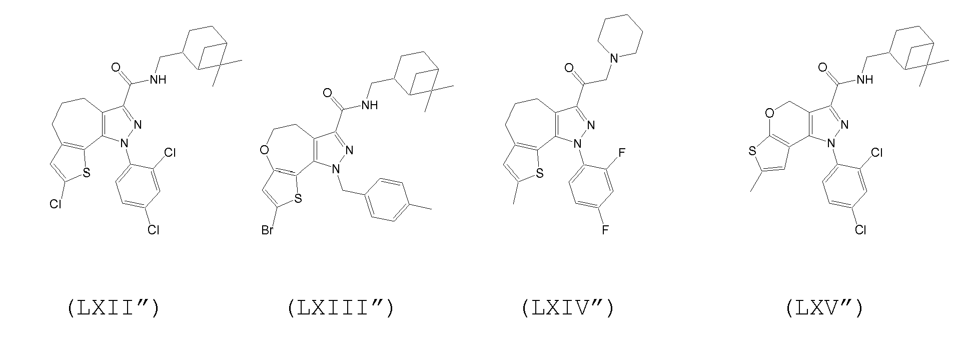

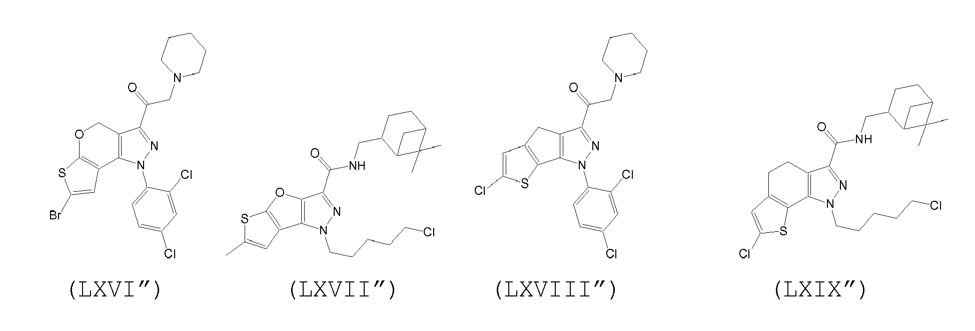

The compounds of formula (I) of the present invention, in dependence on the substituents, can contain in their structure chiral centres. All the various isomers of the compounds of formula (I), and the corresponding mixtures, are included in the present invention. In the compounds of formula (I) both geometrical isomers, for example cis-trans, and optical isomers (stereoisomers) can also be present.

Surprisingly and unexpectedly the Applicant has found that the compounds of formula (I) have affinity for the CB1 and/or CB2 cannabinoidergic receptors, and are able to act both at peripheral level and on central nervous system.

Furthermore it has been surprisingly and unexpectedly found by the Applicant that the compounds of the present invention show an impoved activity at a peripheral level compared to the prior art compounds. In particular, as regards the compounds having affinity for the CB1 cannabinoidergic receptors with agonist activity, the compounds of the present invention show an improved activity at a peripheral level, compared to the prior art compounds, at least in reducing the intraocular pressure. Furthermore it has been, surprisingly and unexpectedly found by the Applicant that the compounds of the present invention showing affinity for the CB1 receptors have an improved activity at a peripheral level on an ex-vivo pharmacological model based on the deferent vessel (vas deferens).

As said, the compounds of the invention are active also on central nervous system, for example the CB1 agonist compounds of the invention can show activity in lowering the body temperature in the laboratory animal. See the examples.

The corresponding hydrates, solvates and the pharmaceutically acceptable salts of the above defined compounds of formula (I), including all the various isomers, stereoisomers and the corresponding mixtures, are a further object of the present invention.

The meaning of the terms hydrate and solvate is well known to the skilled in the field. In particular by hydrate it is meant the compound containing one or more molecules of hydration water, in general from 1 to 10 molecules of water. By solvate it is meant that the compound contains one or more molecules of a solvent different from water.

A further object of the present invention is a process for preparing the compounds of general formula (I) comprising:
i) synthesis of the acid of the following general formula (II), or optionally of one reactive derivative thereof, selected from acyl halides, anhydrides, mixed anhydrides, imidazolides, ester-amide adducts, linear or branched C₁-C₄ alkyl esters: 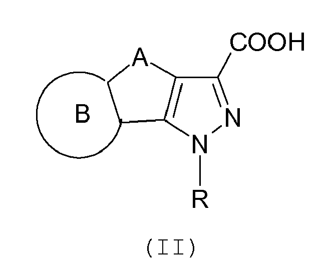 said synthesis comprising the following steps:
   - synthesis of α-hydroxy-γ- ketoesters of formula (IV), wherein A and B are as previously defined, by reacting a compound of formula (III) with sodium alkoxide (RONa) and diethyloxalate in a C₁-C₃ alcoholic solvent at reflux (Claisen condensation): 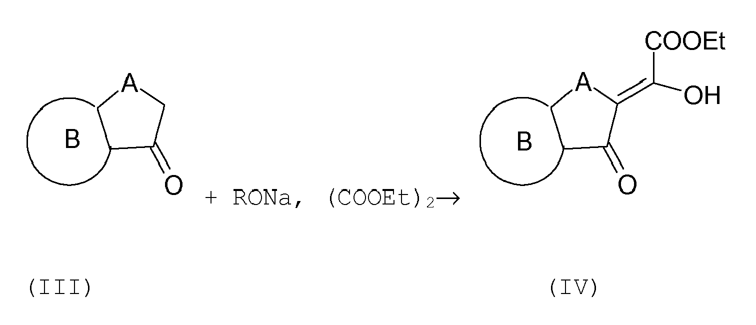
   - reaction of the compounds of formula (IV) with an hydrazine of formula (V), wherein R is as previously defined, said compound (V) optionally being in the form of a hydrochloride salt in an alcoholic solvent or in acetic acid, at reflux, to obtain the tricyclic compound of formula (VI):

      (IV) + NH₂-NH-R → (V) 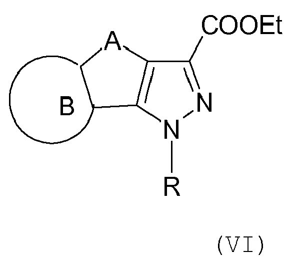
   - base hydrolysis with alkaline hydroxides in an hydroalcoholic solution of the compound of formula (VI) at reflux, to obtain the acid of formula (II);
   - optionally, formation of a reactive derivative of the acid of general formula (II), said reactive derivative being as defined above.
ii) When R'=R'₁ as defined above, the compounds of formula (I) can be prepared according to one of the following two processes:
   first process
      - reaction of an ester of the acid of general formula (II), with trialkylaluminum and with the hydrochloride salt of an ammine in an inert solvent under the reaction conditions until the ester has completely reacted, and subsequent addition to the reaction mixture of [Z"-(Z')ᵥ]MgBr, wherein Z', v and Z" are as defined above, and following reaction at room temperature until obtaining the compound of formula (I) wherein R'=R'₁, or
   second process
      - reaction of the acid of formula (II), or a reactive derivative thereof, with a metallorganic salt of formula [Z"-(Z')ᵥ]⁻ Me⁺, wherein Me⁺ is an alkaline metal cation, in a solvent inert under the reaction conditions, obtaining the compound of formula (I) wherein R'=R'₁.
iii) When in general formula (I) R'= R'₂ the compounds of formula (I) are prepared as follows:
   - formation of the compound of formula (I) wherein R'=R'₁ by using one of the two synthesis routes described above in ii);
   - reduction at room temperature of the compound of formula (I) wherein R'=R'₁, obtaining the final product of formula (I) wherein R'=R'₂.
iiii) When in general formula (I) R'= R'₃ the compounds of formula (I) are prepared by reaction of the acid of formula (II), in the form of a corresponding reactive derivative as defined in i), with a compound of general formula:

   H₂N-(Z')ᵥ-T' (VII)

   wherein Z', v and T' are as defined above. The reaction is carried out in a solvent inert under the reaction conditions at room temperature.

In i), when R has the meaning of bivalent C₁-C₁₀ aliphatic chain, linear or branched when possible, wherein the end of the main chain not linked to the nitrogen atom is linked to W, W being as defined above, compound (VI) is prepared by reacting the compound (IV) with hydrated hydrazine in an alcoholic solvent, at reflux, obtaining the compound (IV'): 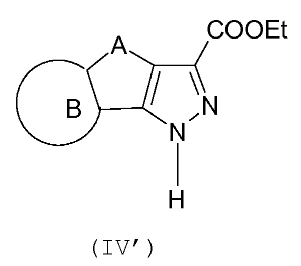 and subsequent alkylation of compound (IV') by means of the compound W-R-T" in an inert solvent at reflux, preferably in the presence of a base, obtaining compound (VI), W and R being as defined above and T" a leaving group, for example mesyl, tosyl, bromine.

In ii) the first of the two synthesis processes is the preferred one.

In ii), preferably in the first reaction of the first of the two synthesis processes for obtaining the compounds of general formula (I) with R'=R'₁, the ethyl ester of the acid of general formula (II), Al(CH₃)₃, HN(OCH₃)CH₃.HCl and, as reaction solvent, dichloromethane, are used. Preferably both the reactions of the first of the two synthesis processes described in ii) are initially carried out at a temperature of 0°C and then at room temperature (20-25°C).

In ii), in the second of the two synthesis processes, preferably Me⁺ is lithium cation.

In iii) preferably the reduction reaction is carried out with lithium hydride and aluminum or with sodium borohydride.

The compounds of formula (III) and (VII) are available on the market or otherwise are described in organic chemistry publications.

By pharmaceutically acceptable salts, all the salts are meant which are obtained by treating the compounds of formula (I) with organic or inorganic acids acceptable from a pharmaceutical point of view. For example hydrochlorides, sulphates, fumarates, oxalates, citrates, hydrogensulphates, succinates, paratoluensulphonates can be mentioned. See the volume: "Remington, The Science and Practice of Pharmacy", vol. II, 1995, page 1457.

A further object of the present invention is represented by pharmaceutical compositions containing the compounds of general formula (I), the isomers and the corresponding mixtures, the corresponding hydrates or solvates or pharmaceutically acceptable salts included.

By pharmaceutical compositions preparations are meant formulations wherein the active principles of formula (I) comprising all the different isomers and mixtures thereof, or the corresponding hydrates or solvates or pharmaceutically acceptable salts, are admixed with excipients, carriers, dyestuffs, preservatives, aromas, and other additives the use of which in the pharmaceutical field is known.

The pharmaceutical compositions can be administered by oral, subcutaneous, sublingual, intramuscular, intravenous, topic, transdermal, rectal, ophthalmic, intranasal route. Said pharmaceutical compositions include for example dispersions, solutions, emulsions, microemulsions, powders, capsules, aerosol, suppositories, tablets, syrups, elixirs, creams, gels, ointments, plasters.

They can be obtained according to known processes of pharmaceutical technique, for example they are obtainable by starting from emulsions and microemulsions wherein the active principles of formula (I), all the different isomers and mixtures thereof, or the corresponding hydrates or solvates or pharmaceutically acceptable salts included, are mixed in the presence of surfactants and other additives, with an aqueous phase and optionally with an oil phase.

It is a further object of the present invention pharmaceutical compositions formed of microemulsions or emulsions or comprising microemulsions or emulsions, comprising the following components (% by weight), the sum of the components being 100%:
S) from 0.01 to 95% of one or more pharmaceutically acceptable compounds, selected from the following classes:
   - surfactants selected from non-ionic, anionic, cationic and amphotheric, optionally containing fluorine atoms,
   - polymers forming organized structures such as aggregates, micelles, liquid crystals, vesicles, in the liquid in which they are solubilized,
O) from 0 to 95% of one or more oils selected from the following classes of pharmaceutically acceptable compounds:
   - esters of C₄-C₃₂ acids, the acid optionally containing one or more unsaturations of ethylene type,
   - C₄-C₃₂ acids optionally containing one or more unsaturations of ethylene type, that can be included when the final composition has a pH such that the acid is not converted into the salt thereof,
PA) from 0.001 to 90% of compounds of formula (I), all the different isomers and mixtures thereof included, or the corresponding hydrates or solvates or pharmaceutically acceptable salts,
AD) from 0 to 60% by weight of one or more compounds selected from the following classes:
   - modifiers of the water and/or oil polarity,
   - modifiers of the film curvature of component S),
   - co-surfactants,
WA) from 0.001 to 99.9% of water or of a saline aqueous solution, optionally buffered,
the sum of the components of the microemulsions or emulsions being 100%.

In the microemulsions of the present invention it is preferred that the amount of component O) ranges, as % by weight, from 0.01 to 95%, preferably from 0.01 to 90%, still more preferably from 0.01 to 70% and in particular from 0.01 to 50%.

The compositions of the invention in the form of microemulsions are limpid and transparent, preferably liquid. When the viscosity is very high, the microemulsions of the invention are in the gel form, optionally formed of liquid crystals.

In component S) the surfactants containing fluorine atoms can have (per)fluorinated chains, for example (per)fluoropolyether chains.

The liquids wherein the polymers of component S) are solubilized to form the organized structures are water and/or oil. The kinds of usable oils are mentioned later on and can be of both natural and synthetic origin.

By microemulsion a system is meant, formed of two or more immiscible phases among each other, transparent, isotropic, comprising at least one aqueous phase and at least one oil phase, wherein the various phases are stabilized by component S), optionally in the presence of one or more compounds AD), for example co-surfactants. See for example R.K. Mitra, Physicochemical investigations of microemulsification of eucalyptus oil and water using mixed surfactants (AOT+ Brij-35) and butanol, J. Colloid and Interface Science, 283 (2005) 565-577. Sometimes the oil phase in the microemulsions for pharmaceutical use is formed of the active principle as such, when it is lipophilic and therefore insoluble in water or in aqueous phase.

By emulsion it is meant a system formed of the same components of the microemulsion but that it has an opalescent or milky appearance, or it can be in the form of a cream.

The processes for preparing the microemulsions of the invention or the emulsions of the invention are described hereinafter.

Preferred microemulsions or emulsions according to the present invention have the following composition (% by weight):
- Component S) from 0.01 to 90%,
- One or more oils of component O) from 0 to 90%,
- Compounds component PA) from 0.001 to 50%,
- Component AD) from 0 to 30%,
- Component WA) from 0.1 to 99.9%,
the sum of the components being 100%.

More preferred microemulsions or emulsions have the following composition (% by weight):
- Component S) from 0.01 to 80%,
- One or more oils of component O) from 0 to 70%,
- Compounds component PA) from 0.05 to 40%,
- Component AD) from 0 to 20%,
- Component WA) from 10 to 99.9%,
the sum of the components being 100%

Still more preferred microemulsions or emulsions have the following composition (% by weight):
- Component S) from 0.01 to 70%,
- One or more oils of component O) from 0 to 50%,
- Compounds component PA) from 0.05 to 30%,
- Component AD) from 0 to 15%,
- Component WA) from 20 to 99.9%,
the sum of the components being 100%.

The preferred surfactants component S) are those non-ionic and anionic ones. Among the non-ionic surfactants, the most preferred are those containing polyoxyalkylene chains, preferably polyoxyethylene chains. The following ones can for example be mentioned:
polyoxyl 35 castor oil, known for example by the trademark Cremophor^{®} EL (BASF), produced by ethoxylation of castor oil,
polyoxyl 40 hydrogenated castor oil, known for example by the trademark Cremophor^{®} RH40 (BASF), prepared by ethoxylation of hydrogenated castor oil,
polyethylenglycol 15 hydroxystearate, known for example by the trademark Solutol^{®} HS15 (BASF), prepared by reaction of 15 moles of ethylene oxide with 1 mole of 12-hydroxystearic acid,
polyoxyethylene polysorbate, such as Tween^{®} 80, Tween^{®} 20, Tween^{®} 60, Tween^{®} 85,
sorbitan esters of fatty acids, as sorbitan monolaurate and sorbitan monostearate, commercialized for example by the name Span^{®} 20 and Span^{®} 60, respectively,
vitamin E/TPGS: tocopheryl propylenglycol 1000 succinate, polyoxyethylen ethers of fatty acids, as those of the series Brij^{®}, quali Brij^{®} 35, Brij^{®} 76, Brij^{®} 98,
PEG-12-acyloxy-stearates, see for example C.E. McNamee et al. in "Physicochemical Characterization of PEG 1500-12-acyloxystearate micelles and liquid cristalline phases", Langmuir, 2005, 21, 8146-8154, among these the following can for example be mentioned:
   - PEG 1500 mono-12-capryloyloxy stearate (PEG 1500-C₁₈C₈)
   - PEG 1500 mono-12-caproyloxy stearate (PEG 1500-C₁₈C₁₀)
   - PEG 1500 mono-12-lauroyloxy stearate (PEG 1500-C₁₈C₁₂)
   - PEG 1500 mono-12-myristoyloxy stearate (PEG 1500-C₁₈C₁₄)
   - PEG 1500 mono-12-palmitoyloxy stearate (PEG 1500-C₁₈C₁₆).

Among the anionic surfactants the following can for example be mentioned: soya lecithin, for example known by the trademark Epikuron^{®} 200, bis-2-ethylhexylsulphosuccinate (AOT), sodium taurocholate.

Among cationic surfactants, hexadecyltrimethylammonium bromide (CTAB) and didodecylammonium bromide (DDAB) can for example be mentioned.

The polymers which can be used as component S) must be soluble in the aqueous phase and/or in the oily phase. By soluble it is meant that the polymers must reach in the phase in which they are soluble concentrations at least equal to those allowing the formation of organized structures as aggregates, micelles, liquid crystals, vesicles. The presence of said organized structures may be detected by specific techniques of the physical chemistry of the dispersed systems. Laser Light Scattering (LLS), Neutron Scattering, microscopy can be for example employed.

As said, the polymers component S) can be used also in combination with the above mentioned surfactants. Also in this case the concentration of the solubilized polymer in the liquid phase must be such as to lead to the formation of the above mentioned organized structures.

The polymers component S) are for example polyvinylpyrrolidone and vinylpyrrolidone/vinyl acetate copolymers, commercialized for example with the name Kollidon^{®}, as Kollidon^{®} 12PF and Kollidon^{®} 17PF (BASF), and the block copolymers containing polyoxyalkylene chains, more preferably containing polyoxyethylene chains (PEO), as for example the block copolymers PEO with polyoxypropylene chains (PPO) characterized by PEO-PPO-PEO structures, commercially available for example by the trademark Pluronic^{®} or Poloxamer^{®} or Lutrol^{®}, as Lutrol^{®} F68 and Lutrol^{®} F127 commercialized by Basf.

In component O) the organic acid esters are preferably obtained by esterification of the corresponding acid, preferably aliphatic carboxylic acid, with an alcohol having an aliphatic chain, preferably C₁-C₅, or having a polyoxyethylene chain, or with glycerine. In this case mono-, di- or triglycerides are obtained.

The following can for example be mentioned:
oleoyl macrogol 6 glyceride (unsaturated polyglycosylated glyceride), commercialized for example with the trademark Labrafil^{®} 1944 CS, (Gattefossé),
propylenglycol caprylate caprate, known for example by the traademark Labrafac^{®} PG (Gattefossé),
propylenglycol monoester of the caprylic acid, commercialized for example by the trademark Capmul^{®} PG-8 (Abitec),
glycerol oleate (for example Peceol^{®} (Gattefossé)),
medium chain mono- and diglycerides, for example capric and caprylic acid glycerides (for example Capmul^{®} MCM (Abitec), Imwitor^{®} 308 (Sasol)),
polyglycerol oleate (for example Pluro^{®} oleic (Gattefossé)), capric/caprylic acid triglycerides (for example Miglyol^{®} 812 and Miglyol^{®} 810 (Sasol), Labrafac^{®} CC CS (Gattefossé)),
ethyl butyrate, ethyl caprylate, ethyl oleate,
tripalmitine, commercialized for example by the trademark DY-NASAN^{®} 116 by Sasol.

Vegetable oils for a pharmaceutical use, containing one or more of the above mentioned esters can also be used. Soya oil can for example be mentioned.

The acids component O) are preferably aliphatic carboxylic acids. Among the acids component O), the stearic acid, the omega-3 and omega-6 acids can be mentioned.

In component AD) as modifiers of the water and/or oil polarity can be cited for example polyethylenglycols. Lutrol^{®}E300 and Lutrol^{®}E400 (BASF) can also be mentioned. Aliphatic alcohols, for example ethanol, can also be used.

In component AD) the modifiers of the film curvature of component S) are for example aliphatic alcohols, preferably C₂-C₅.

In component AD) the co-surfactants can be for example surfactant compounds as defined above, or aliphatic alcohols, preferably having a chain with at least 6 carbon atoms. There can be mentioned for example:
propylen glycol monolaurate, known for example by the trademark Capmul^{®} PG12 (Gattefossé) or Lauroglycol^{®} 90 (Gattefossé),
caprylocaproyl macrogol 8 glyceride (saturated ethyldiglycosylated glyceride) commercialized for example by the trademarks Labrasol^{®}, Gelucire 44-14 (Gattefossé),
diethylenglycol monoethyl ether, known for example by the trademark Transcutol^{®} (Gattefossé).

The compositions formed of microemulsions are stable in a wide range of temperature, generally from 0°C to 80°C, preferably from 4°C to 45°C.

The microemulsions of the present invention can be prepared with a a process comprising the following steps:

| | |
|---|---|
| (IP) | optionally, solubilization of the compound component PA) in oil, obtaining an oily solution of component PA) |
| (IIP) | addition of component S) to component PA), or to the corresponding oily solution obtained in (IP), obtaining a liquid phase comprising components S) and PA) |
| (IIIP) | optionally, addition of component AD) to the liquid phase obtained in (IIP), obtaining a liquid phase comprising components AD)+S)+PA) |
| (IVP) | addition, under stirring, of water or of a saline aqueous solution to the liquid phases obtained in (IIP) or in the optional step (IIIP), obtaining a limpid solution that is the microemulsion. |

The steps of the process can be carried out at temperatures in the range 0°C - 80°C.

It is possible to obtain a microemulsion in the form of a limpid solution also by varying the order of performance of the above mentioned steps, or, for example, by proceeding as follows:

| | |
|---|---|
| (IP') | optionally solubilization of the compound component PA) in oil, obtaining an oily solution of component PA) |
| (IIP') | addition of component S) to water or to a saline aqueous solution, obtaining an aqueous phase comprising components S), |

| | |
|---|---|
| (IIIP') | optionally, addition of component AD) to the aqueous phase, obtaining an aqueous phase comprising components S) and AD) |
| (IVP') | mixing under stirring of component PA) or of the oily solution of step (IP') with the aqueous phase of step (IIP') or optionally step (IIIP'), obtaining the microemulsion. |

The temperature range at which one operates is the same as indicated above.

The emulsions of the present invention can be prepared by a process comprising the following steps:

| | |
|---|---|
| (IP") | optionally, solubilization of the compound component PA) in oil, optionally in the presence of component AD), |
| (IIP") | heating of component PA), or of the oily solution obtained in the optional step (IP") at temperatures in the range 35°C- 80°C, more preferably 45-70°C, |
| (IIIP") | addition of component S) to water or to a saline aqueous solution, optionally including component AD), |
| (IVP") | heating of the aqueous phase of step (IIIP") at temperatures in the range 35°C-80°C, more preferably 45-70°C, |
| (VP") | addition, under stirring of the liquid phase obtained in step (IIP") to the aqueous phase obtained in step (IVP"), obtaining an emulsion, |
| (VIP") | cooling of the emulsion at temperatures comprised between 0°C and 30°C. |

Step (VP") preferably is performed by using turboemulsifiers.

The liquid phases (emulsions) obtained in steps (VP") and (IVP") can optionally be subjected to a further homogeneization step at high pressure.

The emulsions can also be prepared by dilution of microemulsions with water or with aqueous solutions or with component O). Component AD) can be included in each of the following liquid phase: water, the aqueous solutions and component O). Additional pharmaceutical compositions can be obtained according to the procedures described in USP 6,028,084, herein incorporated by reference.

The pharmaceutical compositions can also be prepared by using the methods and the additives indicated in patent application US 2003/0003145. In these formulations sodium alkylsulphate, or another surfactant conventionally used in the pharmaceutical field can be used. For example pharmaceutical compositions usable for the oral administration of the compounds of formula (I), their isomers or of the correspondidng hydrates or solvates or pharmaceutically acceptable salts, comprise (% by weight):
0.5-20% of one or more compounds of formula (I),
0.05-0.5% of sodium alkylsulphate or another surfactant, 2.5-10% of a disgregating agent, for example cellulose, sodium carboxymethylcellulose or other cellulose derivatives, the difference 100% being conventional adjuvants for oral administration dosage forms.

Pharmaceutical formulations usable for both the oral and intraocular administration comprise the compounds of formula (I), and hydroxypropylmethylcellulse. In particular they comprise (% by weight) :
0.1 to 20% of the compounds of formula (I)
0.5 to 10% of hydroxypropylmethylcellulose (HPMC).

Specific pharmaceutical formulations for the oral administration in the form of capsules or tablets comprise compounds of formula (I), hydroxypropylmethylcellulose, other excipients, such as monohydrate lactose, magnesium stearate, microcristalline cellulose, titanium oxide. In these preparations HPMC can be present in the capsule or tablet core, and/or in the tablet shell, when the latter is present in the formulation.

The compounds of formula (I) and the related pharmaceutical compositions have a high affinity in vitro for the CB1 and/or CB2 cannabinoidergic receptors (see the examples). More specifically, the compounds of the invention have a Ki value for the CB1 and/or CB2 receptors lower than 0.5 µM.

The present invention relates also to the use of compounds of formula (I) or their pharmaceutical compositions, for preparing products for the treatment in mammals and in an individual of diseases and disorders involving the CB1 and/or CB2 receptors.

In particular the compounds of formula (I) having affinity towards the CB2 receptors, and the corresponding pharmaceutical compositions, can therefore be used in the treatment of diseases wherein immune system cells or immune disorders are involved or in the treatment of other pathologies, as osteoporosis, renal ischaemia, pain, neuropathic pain, post-surgery pain, inflammatory conditions, lateral amyotrophic schlerosis.

The compounds of the present invention having affinity towards the CB2 receptors and the related pharmaceutical compositions can furthermore be used in the treatment of diseases associated to organ transplants and therapies for preventing allogenic transplant rejection, in the treatment of transplant rejection also in patients undergoing other immunosuppressive therapies, in the treatment and prophylaxis of GVHD (Graft Versus Host Disease), in the treatment of diseases such as: systemic erithematous lupus, ankilosant spondylitis, reumathoid polyarthritis, haemolytic autoimmune anaemia, Behcet disease, Sjögren syndrome, undifferentiated spondylarthritis, reactive arthritis, dermatomyositis.

The compounds of formula (I) having affinity towards the CB1 receptors and the related pharmaceutical compositions can be used in the treatment of ocular diseases, glaucoma or ocular hypertension, pulmonary diseases, asthma and chronic bronchitis, allergies and allergic reactions, (for example allergic rhinitis, contact dermatitis, allergic conjunctivitis), inflammations such as arthitis.

The compounds of formula (I) having affinity towards the CB1 receptors and the corresponding pharmaceutical compositions can be used as analgesics in the treatment of pain, anxiety, behaviour disorders, delirium conditions, psychotic disorders in general, for the treatment of schizophrenia, depression, treatment of drug and/or alcohol abuse, tabagism.

The compounds of formula (I) having affinity towards the CB1 receptors and the corresponding pharmaceutical compositions can also be used in the treatment of vomit, nausea, vertigoes, especially in patients undergone to chemiotherapy, in the treatment of neuropathies, hemicrania, stress, psychosomatic origin diseases, epilepsy, Tourette syndrome, Parkinson disease, Huntington disease, Alzheimer disease, senile dementia, in the case of recognition disorders and memory loss, in the treatment of appetite disorders (obesity, bulimia), in the treatment of pathologies of the gastrointestinal tract and of the bladder, cardiovascular diseases, urinary, fertility and erectile disorders, in the treatment of neuroinflammatory pathologies, as multiple sclerosis, Guillain-Barré syndrome, viral encefalitis, syndrome associated to demineralization, osteoporosis.

The compounds and the pharmaceutical compositions of the present invention can also be used for reducing metabolic and/or cardiovascular risk factors, also in patients with metabolic syndrome and/or dyslipidemia and in patients with type 2 diabetes.

The compounds of formula (I) and thereof pharmaceutical compositions can also be used for the treatment of eye inflammatory conditions, eye autoimmune diseases, uveitis, uveoretinitis and retina neurodegeneration.

The use of the compounds of formula (I) and the related pharmaceutical compositions for the treatment of the various pathologies wherein the CB1 and/or CB2 receptors are involved, can be made by carrying out the known methods used for said treatments. In particular the administration of the compounds must be performed in a sufficiently effective amount for the specific treatment. Similarly, the dosages, the administration route and the posology will be determined depending on the disease typology, on the pathology severity, on the physical conditions and characteristics of the patient (for example age, weight, drug response), on the pharmacokinetics and toxicology of the selected compounds of formula (I) for the specific treatment.

The preferred daily dosage is of 0.01-1,000 mg of compound of formula (I) of the invention per Kg of body weight of the mammal or individual to be treated. In human beings, the preferred daily dosage range is 0.01-1,000 mg of compound for Kg of body weight, still more preferred from 1 to 800 mg.

A further object of the present invention is the use of radiolabelled compounds of formula (I) for identifying and labelling the CB1 or CB2 cannabinoidergic receptors in mammals or in human beings.

The following examples are reported for a better understanding of the present invention but are not meant to be limitative of the scope of the invention.

### EXAMPLES

### EXAMPLE 1.1

### Preparation of ethyl 6-methyl-l-(4'-methylbenzyl)-1,4-dihydrothieno [3',2':4,5]cyclopenta[1,2-c]pyrazol-3-carboxylate

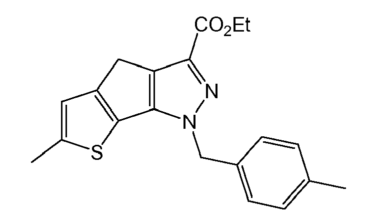

### 1.1a Preparation of ethyl α-(2-methyl-6-oxo-4H-5,6-dihydro cyclopenta [b]thiophen-5-yl)-α-oxo-acetate

Metal sodium (0.60 g) was added in small portions to absolute ethanol (15 ml) by stirring up to complete solubilization. To this mixture diethyloxalate (1.92 g) and then, by dripping, a solution of 2-methyl-4*H*-5,6-dihydrocyclopenta[*b*]thiophen-6-one (2.00 g, corresponding to 13.14 mmoles) in absolute ethanol (40 ml), were added. The reaction mixture was kept under stirring at room temperature for 5 hours and then poured into a mixture of ice and HC1 1N. A white precipitate was formed, that was filtered, washed with water and dried in the air. 2.32 g (yield 70%) of the compound ethyl α-(2-methyl-6-oxo-4*H*-5,6-dihydro-cyclopenta [b]thiophen-5-yl)-α-oxo-acetate were recovered. Rf = 0.47 (CHC1₃); IR (nujol) (λ= cm⁻¹) 1725, 1680; ¹H-NMR (CDCl₃) δ 1.41 (t, 3H, J = 6.8 Hz); 2.63 (s, 3H); 3.81 (s, 2H); 4.39 (q, 2H, J = 7.0 Hz); 6.84 (s, 1H); 12.80 (bs, 1H). Anal. calc. for C₁₂H₁₂O₄S: C, 57.13; H, 4.79; S, 12.71. Found: C, 57.14; H, 4.78; S, 12.70.

### 1.1b Preparation of ethyl 6-methyl-1-(4'-methylbenzyl)-1,4-dihydro thieno[3',2':4,5]cyclopenta[1,2-c]pyrazol-3-carboxylate

A mixture formed of the compound prepared in example 1.1a (1.0 g corresponding to 3.95 mmoles) and of 4-methylbenzyl hydrazine hydrochloride (0.78 g, corresponding to 4.55 mmoles) in glacial acetic acid (8 ml) was heated at reflux for 8 hours, then cooled at room temperature. A precipitate was obtained, which was filtered, washed with water and dried in the air to give 1.16 g (yield 83%) of the compound ethyl 6-methyl-1-(4'-methylbenzyl)-1,4-dihydrothieno[3',2':4.5] cyclopenta[1.2-*c*] pyrazol-3-carboxylate. IR (nujol) (λ= cm⁻¹) 1725, ¹H-NMR (CDC1₃) δ 1,44 (t, 3H, J = 7.0 Hz); 2.34 (s, 3H); 2.48 (s, 3H); 3.52 (s, 2H); 4.44 (q, 2H, J = 7.0 Hz); 5.49 (s, 2H); 6.76 (s, 1H); 7.18-7.32 (m, 4H). Anal. calc. for C₂₀H₂₀N₂O₂S: C, 68.16; H, 5.72; N, 7.95; S, 9.10. Found: C, 68.10; H, 5.70; N, 7.94; S, 9.08.

### EXAMPLE 1.2

### Preparation of ethyl 1-(2',4'-difluorophenyl)-6-methyl-1,4-dihydro thieno[3',2':4.5]cyclopenta[1,2-c]pyrazol-3-carboxylate

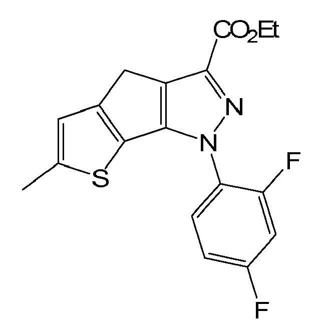

The same procedure described in example 1.1b is used. The compound prepared in example 1.1a (3.95 mmoles) is reacted with 2,4-difluorophenylhydrazine hydrochloride (4.55 mmoles). The yield is quantitative. Rf = 0.60 (oil ligroin/AcOEt 8:2); IR (nujol) (λ= cm⁻¹) 1725; ¹H-NMR (CDCl₃) δ 1.43 (t, 3H, J = 7.3 Hz); 2.52 (s, 3H); 3.63 (s, 2H); 4.45 (q, 2H, J = 7.0 Hz); 6.82 (s, 1H); 7.00-7.12 (m, 2H); 7.68-7.79 (m, 1H). Anal. calc. for C₁₈H₁₄F₂N₂O₂S: C, 59.99; H, 3.92; F, 10.54; N, 7.77; S, 8.90. Found: C, 59.85; H, 3.90; F, 10.51; N, 7.75; S, 8.88.

### EXAMPLE 1.3

### Preparation of ethyl 1-(2',4'-dichlorophenyl)-7-methyl-4,5-dihydro-1H-thieno[2.3-g]indazol-3-carboxylate

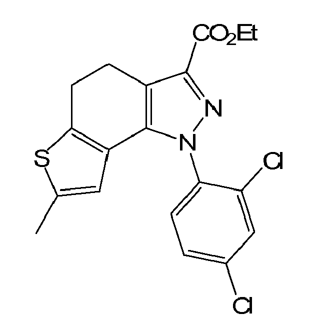

### 1.3a Preparation of ethyl α-(2-methyl-4-oxo-4,5,6,7-tetrahydro-benzothiophen-5-yl)-α-oxo-acetate

The same procedure of example 1.1a was repeated, but using 2-methyl-5*H*-6,7-dihydro-benzo[*b*]thiophen-4-one (13.14 mmoles) instead of 2-methyl-4H-5,6-dihydro-cyclopenta[b]-thiophen-6-one. Yield: 76%. Rf = 0.44 (CHCl₃); IR (nujol) (λ= cm⁻¹) 1724, 1680; ¹H-NMR (CDC1₃) δ 1.35 (t, 3H, J = 6.8 Hz); 2.30 (t, 2H, J = 7.4 Hz); 2.41 (s, 3H); 2.59 (t, 2H, J = 7.4 Hz); 4.28 (q, 2H, J = 6.9 Hz); 6.56 (s, 1H); 12.84 (bs, 1H). Anal. calc. for C₁₃H₁₄O₄S: C, 58.63; H, 5.30; S, 12.04. Found: C, 58.46; H, 5.29; S, 12.01.

### 1.3b Preparation of ethyl 1-(2',4'-dichlorophenyl)-7-methyl-4,5-dihydro-1H-thieno[2,3-g]indazol-3-carboxylate

The same procedure of example 1.1b was repeated but reacting the compound prepared in example 1.3a (3.95 mmoles) with 2,4-dichlorophenylhydrazine hydrochloride (4.55 mmoles). Yield: 71%; Rf = 0.44 (CHC1₃); IR (nujol) (λ= cm⁻¹) 1725; ¹H-NMR (CDCl₃) δ 1.41 (t, 3H, J = 6.9 Hz); 2.41 (s, 3H); 2.80-2.90 (m, 4H); 4.35 (q, 2H, J = 6.9 Hz); 6.60 (s, 1H); 7.39-7.44 (m, 2H); 7.55 (d, 1H, J = 2.0 Hz). Anal. calc. for C₁₉H₁₆C1₂N₂O₂S: C, 56.03; H, 3.96; Cl, 17.41; N, 6.88; S, 7.87. Found: C, 55.88; H, 3.95; Cl, 17.38; N, 6.86; S, 7.85.

### EXAMPLE 1.4

### Preparation of ethyl 7-chloro-1-(2',4'-difluorophenyl)-4,5-dihydro-1H-thieno[2,3-g]indazol-3-carboxylate

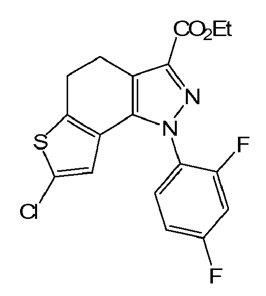

### 1.4a Preparation of ethyl α-(2-chloro-4-oxo-4,5,6,7-tetrahydro-benzothiophen-5-yl)-α-oxo-acetate

The same procedure of example 1.1a was repeated, but using 2-chloro-5*H*-6,7-dihydro-benzo[*b*]thiophen-4-one in place of 2-methyl-4*H*-5,6-dihydro-cyclopenta[*b*]thiophen-6-one. The reaction yield is 82%. Rf = 0.42 (CHC1₃); IR (nujol) (λ= cm⁻¹) 1723, 1681; ¹H-NMR (CDC1₃) δ 1.36 (t, 3H, J = 6.9 Hz); 2.32 (t, 2H, J = 7.5 Hz); 2.57 (t, 2H, J = 7.5 Hz); 4.27 (q, 2H, J = 6.9 Hz); 6.55 (s, 1H); 12.77 (bs, 1H). Anal. calc. for C₁₂H₁₁ClO₄S: C, 50.27; H, 3.87; Cl, 12.36; S, 11.18. Found: C, 50.15; H, 3.86; Cl, 12.33; S, 11.16.

### 1.4b Preparation of ethyl 7-chloro-1-(2',4'-difluorophenyl)-4,5-dihydro-1H-thieno[2,3-g]indazol-3-carboxylate

The same procedure of example 1.1b was repeated, but reacting the compound prepared in example 1.4a (3.95 mmoles) with 2,4-difluorophenylhydrazine hydrochloride (4.55 mmoles). The reaction yield is 97%. Rf = 0.22 (oil ligroin/AcOEt 9:1 volume/volume); IR (nujol) (λ= cm⁻¹) 1724; ¹H-NMR (CDCl₃) δ 1.41 (t, 3H, J = 7.0 Hz); 3.01 (t, 2H, J = 7.9 Hz); 3.16-3.37 (m, 2H); 4.43 (q, 2H, J = 7.0 Hz); 6.11 (s, 1H); 6.98-7.17 (m, 2H); 7.48-7.58 (m, 1H). Anal. calc. for C₁₈H₁₃ClF₂N₂OS: C, 54.76; H, 3.32; Cl, 8.98; F, 9.62; N, 7.10; S, 8.12. Found: C, 54.68; H, 3.31; Cl, 8.96; F, 9.61; N, 7.09; S, 8.10.

### EXAMPLE 1.5

### Preparation of ethyl 7-bromo-1-(2',4'-dichlorophenyl)-4,5-dihydro-1H-thieno[2,3-g]indazol-3-carboxylate

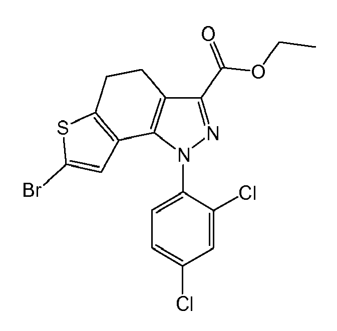

### 1.5a Preparation of 2-bromo-5H-6,7-dihydrobenzo[b]thiophen-4-one

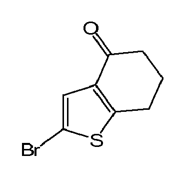

1 g of 6,7-dihydrobenzo[*b*]thiophen-4-one (6.57 mmoles) was solubilized in an aqueous solution of acetic acid at 50% by weight at a temperature of 4°C. A solution of molecular bromine (Br₂) (0.34 ml, 6.57 mmoles) in concentrated acetic acid (6 ml) was added, dropwise, to the obtained solution. The so obtained mixture was reacted under stirring at room temperture for one hour, then poured into an aqueous solution of sodium acetate AcONa (3 ml). A white precipitate was obtained which was successively filtered, washed some times with water and then dried in the air. 1.27 grams (84% yield) of the compound 2-bromo-5*H*-6,7-dihydrobenzo[*b*]thiophen-4-one were separated. IR (KBr) (λ=cm⁻¹) 1708; ¹H-NMR (CDCl₃) δ 1.80-2.00 (m, 2H); 2.40 (t, 2H, J = 7.4 Hz); 2.55 (t, 2H, J = 7.4 Hz); 7.14 (s, 1H). Anal. calc. for C₈H₇BrOS: C, 41.58; H, 3.05; S, 13.87. Found: C, 41.60; H, 3.03; S, 13.91.

### 1.5b Preparation of ethyl 2-(2-bromo-4,5,6,7-tetrahydro-4-oxobenzo[b]thiophen-5-il)-2-oxoacetate

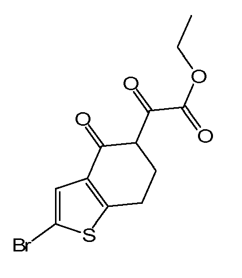

Metal sodium in pieces (0.25 g; 10.82 mmoles) was added to 6 ml of absolute ethanol. It is reacted under stirring at room temperature up to a complete metal dissolution. To the thus obtained solution diethyloxalate (1.18 g; 8.11 mmoles) is added and then, dropwise, a solution of 2-bromo-5*H*-6,7-dihydrobenzo[*b*]thiophen-4-one (1.25 g; 5.41 mmoles) obtained in example 1.5a, dissolved in absolute ethanol (15 ml). The reaction mixture is kept under stirring at room temperature for 22 hours, then poured into a mixture of ice and HC1 1N. A pale yellow precipitate is obtained, which is filtered under vacuum, washed with water and dried in the air. 1.70 grams (95% yield) of the compound ethyl 2-(2-bromo-4,5,6,7-tetrahydro-4-oxobenzo[*b*]thiophen-5-yl)-2-oxoacetate were thus isolated. IR (KBr) (λ=cm⁻¹) 1724 (COOEt), 1682 (C=O); 1677 (C=O); ¹H-NMR (CDCl₃) δ 1.30 (t, 3H, J = 6.9 Hz); 2.20-2.37 (m, 2H); 2.57 (t, 2H, J = 7.5 Hz); 3.20 (t, 1H, J = 7.0 Hz); 4.20(q, 2H J = 6.9 Hz); 6.10 (s, 1H). Anal. calc. for C₁₂H₁₁BrO₄S: C, 43.52; H, 3.35; S, 9.68. Found: C, 43.58; H, 3.31; S, 9.70.

### 1.5c Preparation of ethyl 7-bromo-1-(2',4'-dichlorophenyl)-4,5-dihydro-1H-thieno[2,3-g]indazol-3-carboxylate

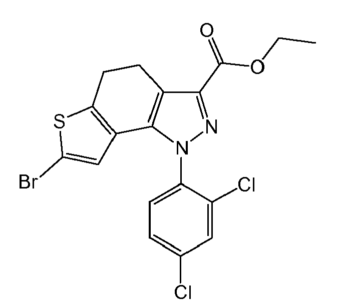

A mixture formed of ethyl 2-(2-bromo-4,5,6,7-tetrahydro-4-oxobenzo[b]thiophen-5-yl)-2-oxoacetate (1.5 g; 3.18 mmoles) obtained in example 1.5c, and of 2,4-dichlorophenylhydrazine hydrochloride (0.75 g; 3.49 mmoles) in absolute ethanol (15 ml) is heated overnight at reflux, then poured into cold water. A white solid is obtained which is filtered, washed with water and dried in the air. The compound ethyl 7-bromo-1-(2',4'-dichlorophenyl)-4,5-dihydro-1*H*-thieno[2,3-*g*]indazol-3-carboxylate was thus obtained. The reaction yield was of 75%. IR (nujol) (λ= cm⁻¹) 1723 (COOEt); ¹H-NMR (CDCl₃) δ 1.36 (t, 3H, J = 6.9 Hz); 2.32 (t, 2H, J = 7.5 Hz); 2.57 (t, 2H, J = 7.5 Hz); 4.27 (q, 2H, J = 6.9 Hz); 6. 10 (s, 1H); 7.70-7.75 (m, 2H); 8.01 (s, 1H). Anal. calc. for C₁₈H₁₃BrCl₂N₂O₂S: C, 45.79; H, 2.78; N, 5.93; S, 6.79. Found: C, 45.75; H, 2.77; N, 5.92; S, 6.80.

### EXAMPLE 1.6

### Preparation of ethyl 1-(2',4'-dichlorophenyl)-5-methyl-1,4-dihydro-thieno[3',2':4,5]cyclopenta[1,2-c]pyrazol-3-carboxylate

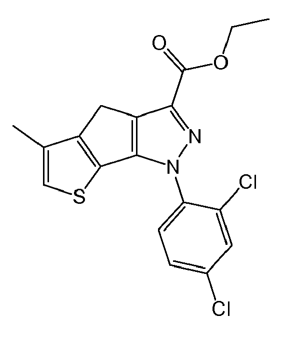

### 1.6a Preparation of 3-chloro-1-(4'-methylthiophen-2'-yl)-propan-1-one

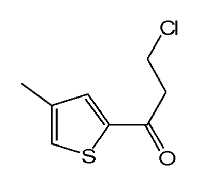

To a solution of 3-methylthiophene (0.50 g; 5.1 mmoles) and 3-chloropropionylchloride (0.49 ml; 5.1 mmoles) in CH₂Cl₂ (4 ml), cooled at a 0°C, AlCl₃ (0.77 g; 5.81 mmoles) was added. The so obtained mixture was stirred at room temperature for 14 hours, then poured on ice (10 grams) and extracted with diethyl ether (3 extractions each with 10 ml). The combined organic phases were anhydrified on anhydrous sodium sulphate and concentrated under vacuum. The oil residue was purified by flash chromatography (oil ligroin/diethyl ether 8.5:1.5 v/v) to give 0.39 grams of 3-chloro-1-(4'-methythiophen-2'-yl)propan-1-one (41% yield). Rf = 0.37 (oil ligroin/diethyl ether 8.5:1.5 v/v); IR (nujol) (λ= cm⁻¹) 1714; ¹H-NMR (CDCl₃) δ 2.31 (s, 3H); 3.17 (t, 2H, J = 8.1 Hz); 3.57 (t, 2H, J = 8.2 Hz); 7.03 (bs, 1H); 7.31 (bs, 1H). Anal. calc. for C₈H₉ClOS: C, 50.93; H, 4.81; Cl, 18.79; S, 16.99. Found: C, 50.81; H, 4.80; Cl, 18.76; S, 16.97.

### 1.6b Preparation of 1-(4'-methylthiophen-2'-yl)prop-2-en-1-one

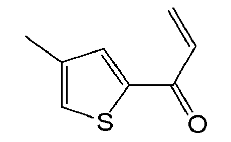

2.00 grams of 3-chloro-1-(4'-methylthiophen-2'-yl)propan-1-one obtained in example 1.6a were solubilized in 14 ml of anhydrous diethyl ether. The thus prepared solution was dripped at room temperature under stirring into a solution, formed of Et₃N (1.77 ml) and anhdrous diethyl ether (16 ml). The mixture was kept under stirring at room temperature for 40 hours. At the end the reaction mixture was washed with a 5% weight HCl aqeuous solution. The organic phase was then separated and anhydrified on anhydrous sodium sulphate. After solvent evaporation under vacuum, it was obtained enone 1-(4'-methylthiophen-2'-yl)prop-2-en-1-one with quantitative yield. Rf = 0.39 (oil ligroin/diethyl ether 8.5:1.5 v/v); IR (nujol) (λ= cm⁻¹) 1708; ¹H-NMR (CDCl₃) δ 2.31 (s, 3H); 5.99 (dd, 1H, J = 3.5 and 8.1 Hz); 6.16 (dd, 1H, J = 3.5 and 13.4 Hz); 6.31 (dd, 1H, J = 8.2 and 13.4 Hz); 7.20 (bs, 1H); 7.39 (bs, 1H). Anal. calc. for C₈H₈OS: C, 63.13; H, 5.30; S, 21.07. Found: C, 63.08; H, 5.29; S, 21.05.

### 1.6c Preparation of 4,5-dihydro-3-methyl-6H-cyclopenta[b] thiophen-6-one

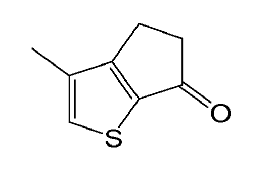

1,4 grams of 1-(4'-methylthiophen-2'-yl)prop-2-en-1-one obtained in example 1.6b were solubilized in 14 ml of 1,2-dichloroethane. The solution was dripped in 14 ml of a mixture of H₂SO₄ 98%/1,2-dichloroethane 1:1 (v/v). The obtained mixture was kept under stirring at 80°C for 75 minutes, then cooled at room temperature and poured on ice (50 grams). The organic phase was separated, washed with a 5% NaHCO₃ aqueous solution, anhydrified by Na₂SO₄ and concentrated under vacuum. It was left an oil, which was purified by flash chromatography (oil ligroin/diethyl ether 7:3 v/v). At the end of the purification process 0.49 grams of 4,5-dihydro-3-methyl-6*H-*cyclopenta[*b*]thiophen-6-one (35% yield) were obtained. Rf = 0.36 (oil ligroin/diethyl ether 8:2 v/v); IR (nujol) (λ= cm⁻¹) 1711; ¹H-NMR (CDCl₃) δ 2.31 (s, 3H); 2.74 (t, 2H, J = 8.0 Hz); 3.22 (t, 2H, J = 8.0 Hz); 6.89 (s, 1H). Anal. calc. for C₈H₈OS: C, 63.13; H, 5.30; S, 21.07. Found: C, 63.10; H, 5.28; S, 21.04.

### 1.6d Preparation of 2-(3-methyl-6-oxo-5,6-dihydro-4H-cyclopenta[b]thiophen-5-yl)-2-ethyl oxoacetate

1.53 grams of diethyloxalate, a solution formed of 1.60 grams of 4,5-dihydro-3-methyl-6*H*-cyclopenta[*b*]thiophen-6-one obtained in example 1.6c and lastly 32 ml of absolute ethanol were added, in sequence, to a solution of sodium ethylate, prepared by solubilizing 0.48 grams of metal sodium in 12 ml of absolute ethanol. The reaction mixture was stirred at room temperature for 5 hours and then poured on ice and aqueous HC1 1N. A white precipitate was obtained which was then filtered, washed with water and dried in the air. 1.96 grams of 2-(3-methyl-6-oxo-5,6-dihydro-4*H*-cyclopenta[*b*]-thiophen-5-yl)-2-ethyl oxoacetate (74% yield) were recovered. Rf = 0.45 (CHCl₃); IR (nujol) (λ= cm⁻¹) 1724, 1677; ¹H-NMR (CDCl₃) δ 1.40 (t, 3H, J = 6.9 Hz); 2.35 (s, 3H); 3.82 (s, 2H); 4.41 (q, 2H, J = 7.0 Hz); 6.89 (s, 1H); 12.75 (bs, 1H). Anal. calc. for C₁₂H₁₂O₄S: C, 57.13; H, 4.79; S, 12.71. Found: C, 57.06; H, 4.77; S, 12.68.

### 1.6e Preparation of ethyl 1-(2',4'-dichlorophenyl)-5-methyl-1,4-dihydro-thieno[3',2':4,5]cyclopenta[1,2-c]pyrazol-3-carboxylate

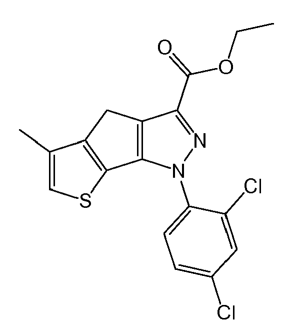

A mixture, containing the diketoester compound obtained in example 1.6d (1.6 grams), 2,4-dichlorophenylhydrazine hydrochloride (1.49 grams) and glacial acetic acid (13 ml), was heated at reflux for 8 hours, and then cooled at room temperature. A suspension was obtained which was afterwards diluted with 100 ml of distilled water. The solid was recovered by filtration, washed with water and dried in the air. 1.99 grams of 1-(2',4'-dichlorophenyl)-5-methyl-1,4-dihydro-thieno-[3',2':4,5] cyclopenta[1,2-c]pyrazol-3-ethyl carboxylate (80% yield) were obtained. IR (nujol) (λ= cm⁻¹) 1726; ¹H-NMR (CDCl₃) δ 1.41 (t, 3H, J = 7.0 Hz); 2.34 (s, 3H); 3.75 (s, 2H); 4.43 (q, 2H, J = 7.0 Hz); 6.91 (s, 1H); 7.39 (dd, 1H, J = 2.0 and 8.2 Hz); 7.48-7.59 (m, 2H); Anal. calc. for C₁₈H₁₄C1₂N₂O₂S: C, 54.97; H, 3.59; Cl, 18.03; N, 7.12; S, 8.15. Found: C, 54.83; H, 3.58; Cl, 18.02; N, 7.11; S, 8.14.

### EXAMPLE 1.7

### Preparation of ethyl 1-(2',4'-dichlorophenyl)-6-methyl-1,4-dihydro-thieno[3',2':4,5]cyclopenta[1,2-c]pyrazole-3-carboxylate

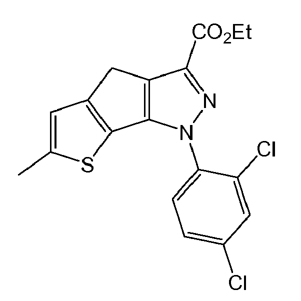

The same procedure described in ex. 1.1b was repeated but the compound prepared in ex. 1.1a was reacted with 2,4-dichlorophenylhydrazine hydrochloride instead of 4-methylbenzyl hydrazine hydrochloride. Yield 64%. IR (nujol) (λ = cm⁻¹) 1725; ¹H-NMR (CDCl₃) δ 1.43 (t, 3H, J = 7.0 Hz); 2.52 (s, 3H); 3.66 (s, 2H); 4.46 (q, 2H, J = 7.0 Hz); 6.83 (s, 1H); 7.40 (dd, 1H, J = 2.2 and 8.4 Hz); 7.54-7.61 (m, 2H). Anal. calc. for C₁₈H₁₄Cl₂N₂O₂S: C, 54.97; H, 3.59; Cl, 18.03; N, 7, 12; S, 8.15. Found: C, 54.79; H, 3.58; Cl, 18.01; N, 7,10; S, 8.13.

### EXAMPLE 1.8

### Preparation of ethyl 6-bromo-1-(2',4'-difluorophenyl)-1,4-dihydro-thieno[3',2':4,5]cyclopenta[1,2-c]pyrazole-3-carboxylate

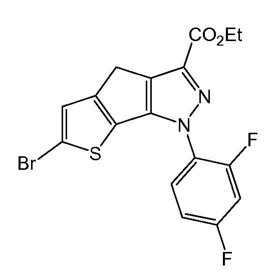

### 1.8a Preparation of ethyl 2-(6-oxo-5,6-dihydro-4H-cyclopenta [b]thiophen-5-yl)-2-oxoacetate

The same procedure described in ex. 1.1a was repeated, but using 4,5-dihydrocyclopenta[*b*]thiophen-6-one instead of 2-methyl-4H-5,6-dihydrocyclopenta[b]thiophen-6-one. Yield 55%. Rf=0.28 (petroleum ether/ethyl acetate 8/2 v/v); IR (nujol) (λ = cm⁻¹) 1724, 1682; ¹H-NMR (CDCl₃) δ 1.41 (t, 3H, J = 7.0 Hz); 3.87 (s, 2H); 4.38 (q, 2H, J = 7.0 Hz); 7.12 (d, 1H, J = 4.8 Hz); 7.93 (d, 1H, J = 4.8 Hz). Anal. calc. for C₁₁H₁₀O₄S: C, 55.45; H, 4.23; S, 13.46. Found: C, 55.26; H, 4.22; S, 13.44.

### 1.8b Preparation of ethyl 1-(2',4'-difluorophenyl)-1,4-dihydro-thieno[3',2':4,5]cyclopenta[1,2-c]pyrazole-3-carboxylate

The same procedure described in ex. 1.1b was repeated but using the compound obtained in ex. 1.8a instead of that obtained in ex. 1.1a. Besides, instead of 4-methylbenzyl hydrazine hydrochloride, 2,4-difluorophenylhydrazine hydrochloride was used. Yield 90%. Rf=0.69 (petroleum ether/ethyl acetate 7/3 v/v); IR (nujol) (λ = cm⁻¹) 1724; ¹H-NMR (CDCl₃) δ 1.46 (t, 3H, J = 7.0 Hz); 3.71 (s, 2H); 4.46 (q, 2H, J = 7.0 Hz); 7.02-7.12 (m, 2H); 7.14 (d, 1H, J = 4.8 Hz); 7.31 (d, 1H, J = 4.8 Hz); 7.71-7.81 (m, 1H). Anal. calc. for C₁₇H₁₂F₂N₂O₂S: C, 58.95; H, 3.49; F, 10.97; N, 8.09; S, 9.26. Found: C, 58.90; H, 3.47; F, 10.95; N, 8.08; S, 9.24.

### 1.8c Preparation of ethyl 6-bromo-1-(2',4'-difluorophenyl)-1,4-dihydro-thieno[3',2':4,5]cyclopenta[1,2-c]pyrazole-3-carboxylate

To a suspension of the compound prepared in ex. 1.8b (1.50 g, 4.33 mmol) in CH₃CN (35 ml) N-bromosuccinimide (NBS) (0.92 g, 5.20 mmol) in small portions, under nitrogen at 0°C, was added. The resulting mixture was then warmed to room temperature and stirred at said temperature for 16 hours. At the end the reaction was quenched with a saturated aqueous sodium thiosulfate solution. A precipitate was obtained. This precipitate was filtered, washed with water, and dried under vacuum to obtain the compound ethyl 6-bromo-1-(2',4'-difluorophenyl)-1,4-dihydro-thieno[3',2':4,5]cyclo penta[1,2-c]pyrazole-3-carboxylate as a white solid (99% yield). Rf=0.63 (petroleum ether/ethyl acetate 8/2 v/v); IR (nujol) (λ. = cm⁻¹) 1725; ¹H-NMR (CDCl₃) δ 1.44 (t, 3H, J = 6.7 Hz); 3.70 (s, 2H); 4.46 (q, 2H, J = 6.0 Hz); 7.01-7.13 (m, 2H); 7.16 (s, 1H); 7.70-7.81 (m, 1H). Anal. calc. for C₁₇H₁₁BrF₂N₂O₂S: C, 48.01; H, 2.61; Br, 18.79; F, 8.94; N, 6.59; S, 7.54. Found: C, 47.93; H, 2.60; Br, 18.77; F, 8.92; N, 6.58; S, 7.53.

### EXAMPLE 1.9

### Preparation of ethyl 6-bromo-1-(2',4'-dichlorophenyl)-1,4-dihydro-thieno[3',2':4,5]cyclopenta[1,2-c]pyrazole-3-carboxylate

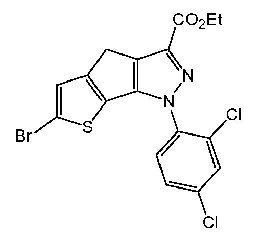

### 1.9a Preparation of ethyl 1-(2',4'-dichlorophenyl)-1,4-dihydro-thieno[3',2':4,5]cyclopenta[1,2-c]pyrazole-3-carboxylate

The same procedure described in ex. 1.1b was repeated but using the compound of ex. 1.8a instead of that of ex. 1.1a, and with 2,4-dichlorophenylhydrazine hydrochloride instead of 4-methyl benzylhydrazine hydrochloride. Yield 68%. Rf=0.55 (petroleum ether/ethyl acetate 8/2 v/v); IR (nujol) (λ = cm⁻¹) 1720; ¹H-NMR (CDCl₃) δ 1.42 (t, 3H, J = 8.0 Hz); 3.7 (s, 2H); 4.45 (q, 2H, J = 8.0 Hz); 7.15 (d, 1H, J= 8.0 Hz); 7.30 (d, 1H, J= 8.0 Hz); 7.41 (dd, 1H, J = 2.2 and 8.0 Hz); 7.54 (d, 1H, J = 8.0 Hz); 7.60 (d, 1H, J = 2.2 Hz). Anal. calc. for C₁₇H₁₂Cl₂N₂O₂S: C, 53.84; H, 3.19; Cl, 18.70; N, 7.39; S, 8.45. Found: C, 53.81; H, 3.20; Cl, 18.68; N, 7.37; S, 8.48.

### 1.9b Preparation of ethyl 6-bromo-1-(2',4'-dichlorophenyl)-1,4-dihydro-thieno[3',2':4,5]cyclopenta[1,2-c]pyrazole-3-carboxylate

The same procedure of ex. 1.8c was repeated, but using the compound obtained in ex. 1.9a instead of that prepared in ex. 1.8b. Yield: 99% . Rf = 0.58 (petroleum ether/ethyl acetate 8/2 v/v); IR (nujol) (λ = cm⁻¹) 1720; ¹H-NMR (CDCl₃) δ 1.42 (t, 3H, J = 8.0 Hz); 3.7 (s, 2H); 4.45 (q, 2H, J = 8.0 Hz); 7.15 (s, 1H); 7.41 (dd, 1H, J = 2.2 and 8.0 Hz); 7.54 (d, 1H, J = 8.0 Hz); 7.60 (d, 1H, J = 2.2 Hz). Anal. calc. for C₁₇H₁₁BrCl₂N₂O₂S: C, 44.57; H, 2.42; Br, 17.44; Cl, 15.48; N, 6.11; S, 7.00. Found: C, 44.55; H, 2.40; Br, 17.46; F, 15.47; N, 6.10; S, 7.55.

### EXAMPLE 2.1

### Preparation of 6-methyl-1-(4'-methylbenzyl)-1,4-dihydro thieno[3',2':4,5]cyclopenta[1,2-c]pyrazol-3-carboxylic acid

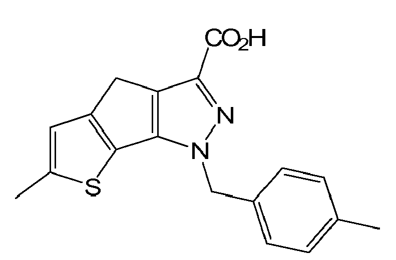

1.1 g (3.15 mmoles) of the ester compound obtained in example 1.1 were solubilized in 16 ml of an EtOH/H₂O solution (1:1 volume/volume). To the obtained solution 2.30 g of solid KOH were added. The so obtained mixture was kept under stirring at the reflux temperature for 4 hours, then poured on ice and HCl 1N. The separated white precipitate was filtered, washed with water and dried in the air. 1.00 g (98% yield) of the 6-methyl-1-(4'-methylbenzyl)-1,4-dihydrothieno[3',2':4,5]-cyclo-penta [1,2-*c*]pyrazol-3-carboxylic acid are obtained. IR (nujol) (λ= cm⁻¹) 3410, 1678; ¹H-NMR (CDCl₃+DMSO) δ 2.29 (s, 3H); 2.47 (s, 3H); 3.41 (bs, 1H); 3.47 (s, 2H); 5.41 (s, 2H); 6.87 (s, 1H); 7.10-7.30 (m, 4H). Anal. calc. for C₁₈H₁₆N₂O₂S: C, 66.64; H, 4.97; N, 8.64; S, 9.88. Found: C, 66.49; H, 4.95; N, 8,62; S, 9.86.

### EXAMPLE 2.2

### Preparation of 1-(2',4'-difluorophenyl)-6-methyl-1,4-dihydro-thieno[3',2':4,5]cyclopenta[1,2-c]pyrazol-3-carboxylic acid

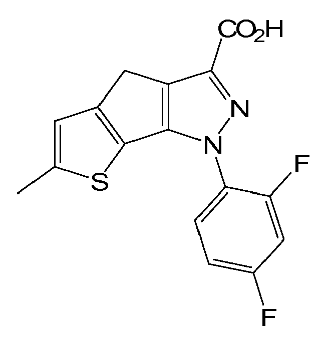

The same procedure described in example 2.1 was followed but using the ester compound of example 1.2 instead of the compound of example 1.1. The yield was quantitative. IR (nujol) (λ= cm⁻¹) 3410, 1678; ¹H-NMR (CDCl₃+DMSO) δ 2.48 (s, 3H); 3.44 (bs, 1H); 3.65 (s, 2H); 6.96 (s, 1H); 7.30-7.39 (m, 1H); 7.60-7.68 (m, 1H); 7.76-7.85 (m, 1H). Anal. calc. for C₁₆H₁₀F₂N₂O₂S: C, 57.83; H, 3.03; F, 11.43; N, 8.43; S, 9.65. Found: C, 57.77; H, 3,02; F, 11.42; N, 8.42; S, 9.63.

### EXAMPLE 2.3

### Preparation of 1-(2',4'-dichlorophenyl)-7-methyl-4,5-dihydro-1H-thieno[2,3-g]indazol-3-carboxylic acid

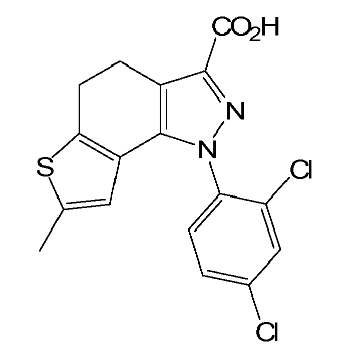

The same procedure described in example 2.1 was followed but using the ester compound obtained in example 1.3 instead of the compound synthesized in example 1.1. The yield was 95%. IR (nujol) (λ= cm⁻¹) 3400, 1677; ¹H-NMR (CDCl₃+DMSO) δ 2.41 (s, 3H); 3.49 (bs, 1H); 2.75-2.92 (m, 4H); 6.61 (s, 1H); 7.40-7.45 (m, 2H); 7.53 (d, 1H, J = 2.2 Hz). Anal. calc. for C₁₇H₁₂Cl₂N₂O₂S: C, 53.84; H, 3.19; Cl, 18.70; N, 7.39; S, 8.45. Found: C, 53.69; H, 3.18; Cl, 18.68; N, 7.37; S, 8.43.

### EXAMPLE 2.4

### Preparation of 7-chloro-1-(2',4'-difluorophenyl)-4,5-dihydro-1H-thieno[2,3-g]indazol-3-carboxyilc acid

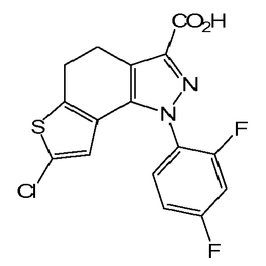

The same procedure of example 2.1 was followed but using the ester compound of example 1.4 in place of the compound of example 1.1. The yield was 96%. IR (nujol) (λ= cm⁻¹) 3410, 1679; ¹H-NMR (CDCl₃+DMSO) δ 1.39 (bs, 1H); 2.89-3,15 (m, 4H); 6.18 (s, 1H); 6.75-8.26 (m, 3H). Anal. calc. for C₁₆H₉ClF₂N₂O₂S: C, 52.40; H, 2.47; Cl, 9.67; F, 10.36; N, 7.64; S, 8.74. Found: C, 52.33; H, 2.46; Cl, 9.65; F, 10.35; N, 7.63; S, 8.75.

### EXAMPLE 2.5

### Preparation of 7-bromo-1-(2',4'-dichlorophenyl)-4,5-dihydro-1H-thieno[2,3-g]indazol-3-carboxylic acid

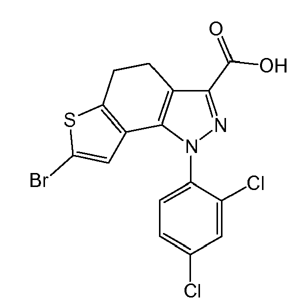

A mixture of ethyl 7-bromo-1-(2,4-dichlorophenyl)-4,5-dihydro-1*H*-thieno[2,3-*g*]indazol-3-carboxylate (0.32 g, 0.68 mmoles) and KOH (0.15 g; 2.71 mmoles) in methanol (7 ml) is heated at reflux for 20 hours, obtaining finally a solution. The solution was then cooled to room temperature and poured on ice and HCl 1N. A white solid is formed which is filtered, washed with water and dried in the air. 0.29 g (97% yield) of 7-bromo-1-(2,4-dichlorophenyl)-4,5-dihydro-1H-thieno [2,3-g]indazol-3-carboxylc acid were thus obtained. IR (KBr) (λ = cm⁻¹) 3360 (OH), 1675 (C=O); ¹H-NMR (DMSO) δ 3.03 (t, 2H, J=8.4 Hz); 3.10 (t, 2H, J=8.7 Hz); 6.10 (s, 1H); 7.69-7.76 (m, 2H); 8.00 (s, 1H). Anal. calc. for C₁₆H₉BrCl₂N₂O₂S: C, 43.27; H, 2.04; N, 6.31; S, 7.22. Found: C, 43.25; H, 2.01; N, 6.33; S, 7.25.

### EXAMPLE 2.6

### Preparation of 1-(2',4'-dichlorophenyl)-5-methyl-1,4-dihydro-thieno[3',2':4,5]cyclopenta[1,2-c]pyrazol-3-carboxylic acid

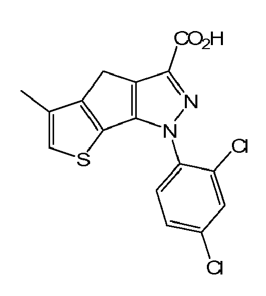

To a solution of the ester compound obtained in example 1.6 (1.70 g; 4.32 mmoles) in 25 ml of EtOH/H₂O 1:1 (v/v), 2.42 grams of solid KOH were added under stirring. The mixture was heated at reflux for 4 hours, then cooled at room temperature, then poured on a mixture of ice and 1N aqueous HCl. A white precipitate was formed which was filtered, washed with water and dried in the air. 1.51 grams of the 1-(2',4'-dichlorophenyl)-5-methyl-1,4-dihydro-thieno[3',2':4,5] cyclopenta[1,2-*c*]pyrazol-3-carboxylic acid (96% yield) were thus obtained. IR (nujol) (λ = cm⁻¹) 3400, 1677; ¹H-NMR (DMSO) δ 2.36 (s, 3H); 3.74 (s, 2H); 6.99 (s, 1H); 7.56-7.80 (m, 3H); 8.10 (bs, 1H); Anal. calc. for C₁₆H₁₀Cl₂N₂O₂S: C, 52.62; H, 2.76; Cl, 19.41; N, 7.67; S, 8.78. Found: C, 52.48; H, 2.75; Cl, 19.39; N, 7.65; S, 8.77.

### EXAMPLE 2.7

### Preparation of 1-(2',4'-dichlorophenyl)-6-methyl-1,4-dihydro-thieno[3',2':4,5]cyclopenta[1,2-c]pyrazole-3-carboxylic acid

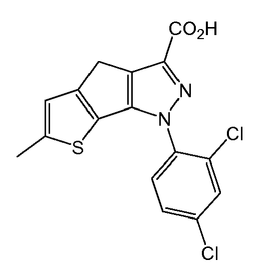

The same procedure described in ex. 2.1 was repeated but the starting compound used in the synthesis was the ethyl ester obtained in ex. 1.7. Yield 83%. IR (nujol) (λ_{.}= cm⁻¹) 3410, 1678; ¹H-NMR (DMSO) δ. 2.48 (s, 3H); 3.64 (s, 2H); 7.00 (s, 1H); 7.62-7.82 (m, 3); 8.03 (bs, 1H). Anal. calc. for C₁₆H₁₀Cl₂N₂O₂S: C, 52.62; H, 2.76; Cl, 19.41; N, 7.67; S, 8.78. Found: C, 52.48; H, 2.75; Cl, 19.39; N, 7.65; S, 8.77.

### EXAMPLE 2.8

### Preparation of 6-bromo-1-(2',4'-difluorophenyl)-1,4-dihydro-thieno [3',2':4,5]cyclopenta[1,2-c]pyrazole-3-carboxylic acid

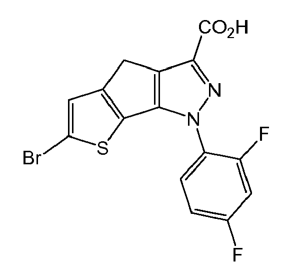

The same procedure described in ex. 2.1 was repeated but the starting compound used in the synthesis was the ethyl ester obtained in ex. 1.8 instead of the compound obtained in ex. 1.1. Yield 99%. Rf=0.29 (chloroform/methanol 9/1 v/v). IR (nujol) (λ_{.}= cm⁻¹) 3400, 1679; ¹H-NMR (CDCl₃+DMSO) δ_{.} 3.72 (s, 2H); 7.16-7.27 (m, 2H); 7.41 (s, 1H); 7.92-8.01 (m, 1H); 11.30 (bs, 1H). Anal. calc. for C₁₅H₇BrF₂N₂O₂S: C, 45.36; H, 1.78; Br, 20.12; F, 9.57; N, 7.05; S, 8.07. Found: C, 45.28; H, 1.77; Br, 20.08; F, 9.55; N, 7.04; S, 8.06.

### EXAMPLE 2.9

### Preparation of 6-bromo-1-(2',4'-dichlorophenyl)-1,4-dihydro-thieno [3',2':4,5]cyclopenta[1,2-c]pyrazole-3-carboxylic acid

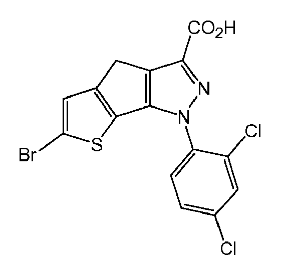

The same procedure described in ex. 2.1 was repeated but the starting compound used in the synthesis was the ethyl ester obtained in ex. 1.9 instead of the compound obtained in ex. 1.1. Yield 99%. Rf=0.25 (chloroform/methanol 9/1 v/v). IR (nujol) (λ_{.}= cm⁻¹) 3392, 1680; ¹H-NMR (DMSO) δ 3.72 (s, 2H) ; 7.44 (s, 1H); 7.68 (dd, 1H, J= 2.2 and 8.5 Hz); 7.74 (d, 1H, J= 8.5 Hz); 8.01 (d, 1H, J= 2.2 Hz); 12.0 (bs, 1H). Anal. calc. for C₁₅H₇BrCl₂N₂O₂S: C, 40.39; H, 1.58; Br, 17.91; Cl, 15.89; N, 6.28; S, 7.19. Found: C, 40.38; H, 1.56; Br, 17.90; Cl, 15.90; N, 6.26; S, 7.21.

### EXAMPLE 3.1

### Preparation of N-myrtanyl-6-methyl-1-(4'-methylbenzyl)-1,4-dihydrothieno[3',2':4,5]cyclopenta[1,2-c]pyrazol-3-carboxamide

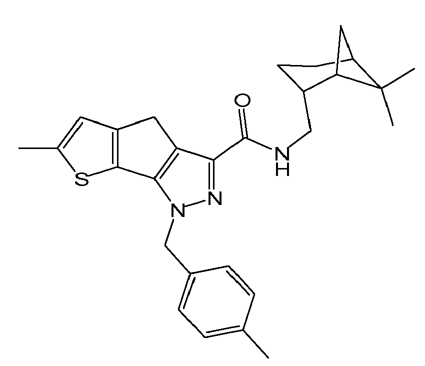

A mixture comprising the acid compound obtained in example 2,1 (0.62 g, corresponding to 1.91 mmoles), *N*-(3-dimethylamino propyl)-*N*'-ethylcarbodiimide hydrochloride (EDC) (1.2 equivalents), 1-hydroxybenzotriazole hydrate (HOBt) (1.2 equivalents) and CH₂Cl₂ (5 ml) was stirred at room temperature for 1 hour. To the obtained mixture a solution of (-)-*cis-*myrtanylamine (2 equivalents) in CH₂Cl₂ (5 ml) was then dropwise added. The thus obtained solution was stirred at room temperature for 14 hours and then concentrated under reduced pressure. The residue was purified by flash chromatography (oil ligroin/AcOEt 8.5:1.5) to obtain the compound *N*-myrtanyl-6-methyl-1-(4'-methylbenzyl)-1,4-dihydrothieno [3',2':4,5]-cyclopenta[1,2-*c*] pyrazol-3-carboxamide with a 78% yield. Rf = 0.27 (oil ligroin/AcOEt 8.5:1.5); IR (nujol) (λ= cm⁻¹) 3420, 1660; ¹H-NMR (CDCl₃) δ 0.92 (d, 1H, J = 9.2 Hz); 1.09 (s, 3H); 1.21 (s, 3H); 1.46-1.72 (m, 1H); 1.72-2.13 (m, 7H); 2.23-2.45 (m, 1H); 2.34 (s, 3H); 2.48 (s, 3H); 3.26-3.63 (m, 1H); 3.57 (s, 2H); 5.32 (s, 2H); 6.76 (s, 1H); 6.85-7.00 (m, 1H); 7. 10-7.22 (m, 4H). Anal. calc. for C₂₈H₃₃N₃OS: C, 73.16; H, 7.24; N, 9.14; S, 6.98. Found: C, 73.11; H, 7.23; N, 9.12; S, 6.96.

### EXAMPLE 3.2

### Preparation of N-myrtanyl-1-(2',4'-difluorophenyl)-6-methyl-1,4-dihydrothieno[3',2':4,5]cyclopenta[1,2-c]pyrazol-3-carboxamide

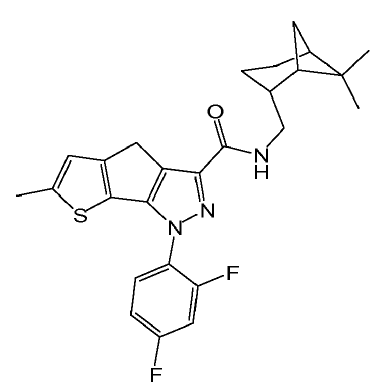

The same procedure of example 3.1 was followed, but using the acid compound of example 2.2 in place of the compound of example 2.1. The yield was 96%. Rf = 0.52 (oil ligroin/AcOEt 8:2); IR (nujol) (λ= cm⁻¹) 3405, 1664; ¹H-NMR (CDCl₃) δ 0.91 (d, 1H, J = 9.6 Hz); 1.09 (s, 3H); 1.21 (s, 3H); 1.52-1.63 (m, 1H); 1.81-2.06 (m, 5H); 2.27-2.39 (m, 2H); 2.52 (s, 3H); 3.36-3.44 (m, 1H); 3.46-3.55 (m, 1H); 3.69 (s, 2H); 6.83 (s, 1H); 6.96 (bt, 1H, J = 5.5 Hz); 7.02-7.12 (m, 2H); 7.62-7.71 (m, 1H). Anal. calc. for C₂₆H₂₇F₂N₃OS: C, 66.79; H, 5.82; F, 8.13; N, 8.99; S, 6.86. Found: C, 66.71; H, 5.81; F, 8.11; N, 8.97; S, 6.85.

### EXAMPLE 3.3

### Preparation of N-myrtanyl-1-(2',4'-dichlorophenyl)-7-methyl-4,5-dihydro-1H-thieno[2,3-g]indazol-3-carboxamide

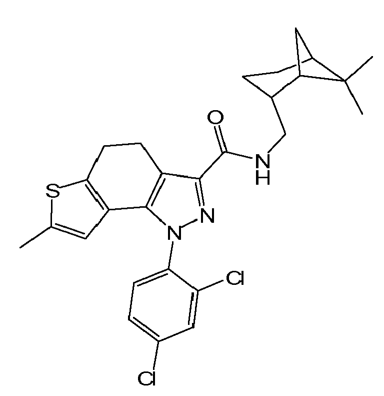

The same procedure described in example 3.1 was followed, but using the acid compound obtained in example 2.3 instead of the compound of example 2.1. The yield is 60%. Rf = 0.37 (oil ligroin/ AcOEt 9:1); IR (nujol) (λ= cm⁻¹) 3410, 1662; ¹H-NMR (CDCl₃) δ 0.89 (d, 1H, J = 9.6 Hz); 1.07 (s, 3H); 1.20 (s, 3H); 1.47-1.68 (m, 1H); 1.77-2.05 (m, 5H); 2.19-2.41 (m, 2H); 2.31 (s, 3H); 3.00 (t, 2H, J = 7.8 Hz); 3.19-3.55 (m, 2H); 3.28 (t, 2H, J = 7.9 Hz); 5.81 (s, 1H); 6.91 (bs, 1H); 7.43 (bs, 2H); 7.62 (s, 1H). Anal. calc. for C₂₇H₂₉Cl₂N₃OS: C, 63.03; H, 5.68; Cl, 13.78; N, 8.17; S, 6.23. Found: C, 62.88; H, 5.67; Cl, 13.75; N, 8.15; S, 6.22.

### EXAMPLE 3.4

### Preparation of N-myrtanyl-7-chloro-1-(2',4'-difluorophenyl)-4,5-dihydro-1H-thieno[2,3-g]indazol-3-carboxamide

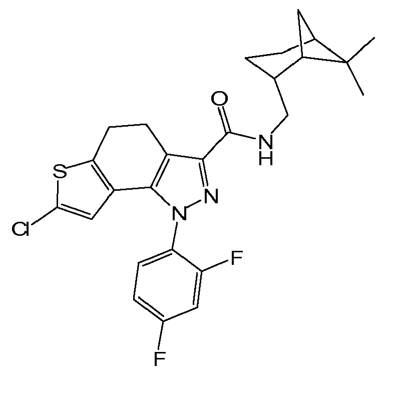

The same procedure described in example 3.1 was followed but using the acid compound of example 2.4 in place of the compound of example 2.1. The yield was 42%. Rf = 0.44 (oil ligroin/AcOEt 9:1 volume/volume); IR (nujol) (λ= cm⁻¹) 3400, 1664; ¹H-NMR (CDCl₃) δ 0.90 (d, 1H, J = 9.5 Hz); 1.07 (s, 3H); 1.20 (s, 3H); 1.47-1.70 (m, 1H); 1.81-2.06 (m, 5H); 2.26-2.41 (m, 2H); 2.99 (t, 2H, J = 8.2 Hz); 3.30 (t, 2H, J = 8.0 Hz); 3.33-3.42 (m, 1H); 3.43-3.52 (m, 1H); 6.12 (s, 1H); 6.91 (bs, 1H); 7.02-7.11 (m, 2H); 7.45-7.55 (m, 1H). Anal. calc. for C₂₆H₂₆ClF₂N₃OS: C, 62.20; H, 5.22; Cl, 7.06; F, 7.57; N, 8.37; S, 6.39. Found: C, 62.11; H, 5.21; Cl, 7.05; F, 7.55; N, 8.36; S, 6.38.

### EXAMPLE 3.5

### Preparation of N-myrtanyl-7-bromo-1-(2',4'-dichlorophenyl)-4,5-dihydro-1H-thieno[2,3-g]indazol-3-carboxamide

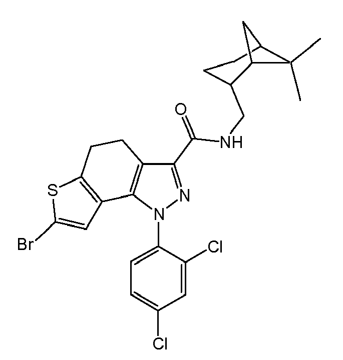

The same procedure described in example 3.1 was followed but using the acid compound of example 2.5 instead of the compound of example 2.1. At the end of the synthesis 0.21 g (56% yield) of the compound *N*-myrtanyl-7-bromo-1-(2',4'-dichlorophenyl)-4,5-dihydro-1H-thieno[2,3-g]indazol-3-carboxamide were obtained. IR (KBr) (λ= cm⁻¹) 3428 (NH), 1661 (C=O); ¹H-NMR (CDCl₃) δ 0.90 (d, 1H, J = 9.6 Hz); 1.06 (s, 3H); 1.20 (s, 3H); 1.51-1.60 (m, 1H); 1.86-2.00 (m, 5H); 2.31-2.37 (m, 2H); 3.09 (t, 2H, J=7.6 Hz); 3.33-3.50 (m, 4H); 6.11 (s, 1H); 6.92 (bt, NH); 7.42-7.48 (m, 2H); 7.64 (d, 1H, J=1.20 Hz). Anal. calc. for C₂₆H₂₆BrCl₂N₃OS: C, 53.90; H, 4.52; N, 7.25; S, 5.53. Found: C, 53.93; H, 4.50; N, 7.22; S, 5.55.

### EXAMPLE 3.6

### Preparation of N-myrtanyl-1-(2',4'-dichlorophenyl)-7-phenyl-4,5-dihydro-1H-thieno[2,3-g]indazol-3-carboxamide

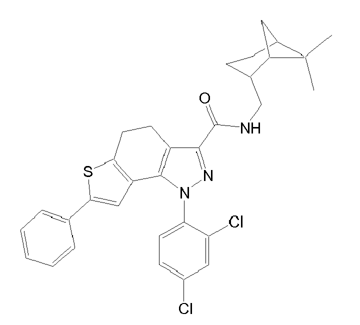

To a mixture in 5 ml of solvent THF/H₂O 4:1 v/v, formed of the following compounds: N-myrtanyl-7-bromo-1-(2',4'-dichloro-phenyl)-4,5-dihydro-1H-thieno[2,3-g]indazol-3-carboxamide (0.2 g; 0.34 mmoles) obtained in example 3.5, phenylboronic acid (0.06 g; 0.52 mmoles) and K₂CO₃, the catalyst [(Ph)₃P]₂PdCl₂ (0.012 g) was added. It is heated then at reflux for 48 hours. At the end it is cooled at room temperature. Then it is diluted with water and extracted with ethyl ether. The organic phases are combined and washed with a saturated NaCl solution. It is dried on sodium sulphate and lastly filtered. The solvent is then removed by evaporation and the residue purified by flash chromatography (oil ligroin/ ethyl acetate 9:1 volume/volume) and recrystallized by oil ligroin/ethyl ether 98:2 v/v. 0.15 g (77% yield) of the compound *N*-myrtanyl-1-(2',4'-dichlorophenyl)-7-phenyl-4,5-di-hydro-1H-thieno[2,3-g]indazol-3-carboxamide were thus obtained as a white solid. IR (KBr) (λ= cm⁻¹) 3430 (NH), 1661 (C=O); ¹H-NMR (CDCl₃) δ 0.90 (d, 1H, J = 9.6 Hz); 1.06 (s, 3H); 1.20 (s, 3H); 1.51-1.60 (m, 1H); 1.86-2.00 (m, 5H); 2.31-2.37 (m, 2H); 3.09 (t, 2H, J=7.6 Hz); 3.33-3.50 (m, 4H); 6.33 (s, 1H); 6.94 (bt, NH); 7.26 (bs, 1H); 7.33-7.34 (m, 4H); 7.45-7.51 (m, 2H); 7.65 (d, 1H, J=1.90 Hz). Anal. calc. for C₃₂H₃₁Cl₂N₃OS: C, 66.66; H, 5.42; N, 7.29; S, 5.56. Found: C, 66.61; H, 5.39; N, 7.28; S, 5.59.

### EXAMPLE 3.7

### Preparation of N-myrtanyl-5-methyl-1-(2',4'-dichlorophenyl)-1,4-dihydro-thieno[3',2':4,5]cyclopenta[1,2-c]pyrazol-3-carboxamide

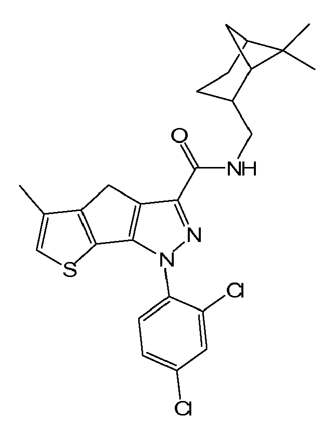

A mixture formed of the acid compound obtained in example 2.6 (0.2 grams), 1.2 equivalents of di *N*-(3-dimethylaminopropyl)-*N'*-ethylcarbodiimide hydrochloride (EDC) and 1.2 equivalents of 1-hydroxybenzotriazole hydrate (HOBt) in CH₂Cl₂ (5 ml) was stirred at room temperature for one hour. To the obtained suspension a solution of (-)-*cis*-myrtanylamine (2 equivalents) in CH₂Cl₂ (5 ml) was then added. Stirring was continued at room temperature for 6 hours. After evaporation of the solvent the residue was purified by flash chromatography (CHCl₃) to give 0.19 grams of *N*-myrtanyl-5-methyl-1-(2',4'-dichlorophenyl)-1,4-dihydro-thieno [3',2':4,5]cyclopenta[1,2-*c*]pyrazol-3-carboxamide (70% yield) as a white solid. M.p.: 112-113°C; IR (nujol) (λ= cm⁻¹) 3430, 1662; ¹H-NMR (CDCl₃) δ 0.91 (d, 1H, J = 9.1 Hz); 1.09 (s, 3H); 1.21 (s, 3H); 1.44-1.73 (m, 1H); 1.74-2.15 (m, 7H); 2.21-2.47 (m, 1H); 2.35 (s, 3H); 3.24-3.55 (m, 1H); 3.72 (s, 2H); 6.96 (s, 1H); 7.39 (dd, 1H, J = 2.1 and 8,7 Hz); 7.49 (d, 1H, J = 8.6 Hz); 7.54 (d, 1H, J = 2.0 Hz). Anal. calc. for C₂₆H₂₇Cl₂N₃OS: C, 62.40; H, 5.44; Cl, 14.17; N, 8.40; S, 6.41. Found: C, 62.31; H, 5.43; Cl, 14.15; N, 8.39; S, 6.40.

### EXAMPLE 3.8

### Preparation of N-myrtanyl-1-(2',4'-dichlorophenyl)-6-methyl-1,4-di hydro-thieno[3',2':4,5]cyclopenta[1,2-c]pyrazole-3-carboxamide

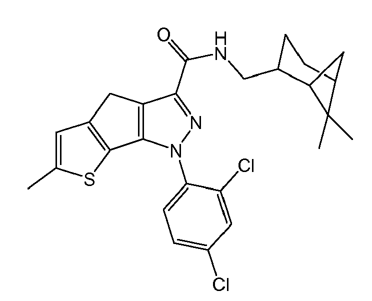

The same procedure described in ex. 3.1 was repeated but the starting compound of the synthesis was the carboxylic acid prepared in ex. 2.7 instead of that prepared in ex. 2.1. Yield 83%. Rf=0.44 (petroleum ether/ethyl acetate 8:2 v/v). IR (nujol) (λ = cm⁻¹) 3405, 1663; ¹H-NMR (CDCl₃) δ 0.91 (d, 1H, J = 9.4 Hz); 1.09 (s, 3H); 1.21 (s, 3H); 1.50-1.65 (m, 1H); 1.80-2.07 (m, 5H); 2.26-2.40 (m, 2H); 2.51 (s, 3H); 3.35-3.45 (m, 1H); 3.44-3.56 (m, 1H); 3.70 (s, 2H); 6.82 (s, 1H); 6.90 (bt, 1H, J = 5.4 Hz); 7.40 (dd, 1H, J = 2.2 and 8.5 Hz); 7.51 (d, 1H, J = 8.5 Hz); 7.61 (d, 1H, J = 2.2 Hz). Anal. calc. for C₂₆H₂₇Cl₂N₃OS: C, 62.40; H, 5.44; Cl, 14.17; N, 8.40; S, 6.41. Found: C, 62.29; H, 5.43; Cl, 14.15; N, 8.39; S, 6.40.

### EXAMPLE 3.9

### Preparation of N-tetrahydrofurfuryl-6-bromo-1-(2',4'-difluoro phenyl)-1,4-dihydro-thieno[3',2':4,5]cyclopenta[1,2-c]pyrazole-3-carboxamide

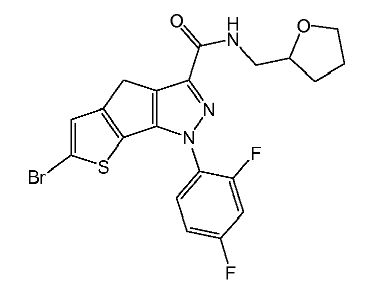

The same procedure described in ex. 3.1 was repeated but the starting compound of the synthesis was the carboxylic acid prepared in ex. 2.8 instead of that prepared in ex. 2.1. Besides, the amine dripped in the reaction mixture was tetrahydrofurfuryl amine, instead of (-)-*cis*-myrtanylamine. Yield 71%. Rf=0.20 (petroleum ether/ethyl acetate 7/3 v/v). IR (nujol) (λ = cm⁻¹) 3409, 1667; ¹H-NMR (CDCl₃) δ 1.59-1.69 (m, 1H); 1.85-1.96 (m, 2H); 1.97-2.07 (m, 1H); 3.36-3.44 (m, 1H); 3.68-3.81 (m, 2H); 3.73 (s, 2H); 3.86-3.94 (m, 1H); 4.04-4.13 (m, 1H); 7.03-7.13 (m, 2H); 7.15 (s, 1H); 7.25 (bt, 1H); 7.64-7.72 (m, 1H). Anal. calc. for C₂₀H₁₆BrF₂N₃O₂S: C, 50.01; H, 3.36; Br, 16.64; F, 7.91; N, 8.75; S, 6.68. Found: C, 49.95; H, 3.35; Br, 16.61; F, 7.89; N, 8.74; S, 6.67.

### EXAMPLE 3.10

### Preparation of N-(1-cyclohexylethyl)-6-bromo-1-(2',4'-difluoro phenyl)-1,4-dihydro-thieno[3',2':4,5]cyclopenta[1,2-c]pyrazole-3-carboxamide

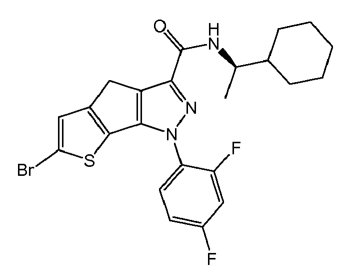

The same procedure described in ex. 3.1 was repeated but the starting compound of the synthesis was the carboxylic acid prepared in ex. 2.8 instead of the compound prepared in ex. 2.1. Besides, the amine dripped in the reaction mixture was R-(-)-1-cyclohexylethylamine instead of (-)-*cis*-myrtanylamine. Yield 57%. Rf=0.66 (petroleum ether/ethyl acetate 8/2 v/v). IR (nujol) (λ = cm⁻¹) 3413, 1668; ¹H-NMR (CDCl₃) δ 0.99-1.29 (m, 5H); 1.21 (d, 3H, J = 6.6 Hz); 1.40-1.50 (m, 1H); 1.62-1.69 (m, 1H); 1.72-1.87 (m, 4H); 3.75 (s, 2H); 4.00-4.11 (m, 1H); 6.76 (d, 1H, J = 9.4 Hz); 7.04-7.13 (m, 2H); 7.16 (s, 1H); 7.65-7.73 (m, 1H). Anal. calc. for C₂₃H₂₂BrF₂N₃OS: C, 54.55; H, 4.38; Br, 15.78; F, 7.50; N, 8.30; S, 6.33. Found: C, 54.48; H, 4.37; Br, 15.75; F, 7.48; N, 8.29; S, 6.32.

### EXAMPLE 3.11

### Preparation of N-cyclohexylmethyl-6-bromo-1-(2',4'-difluorophenyl) -1,4-dihydro-thieno[3',2':4,5]cyclopenta[1,2-c]pyrazole-3-carboxamide

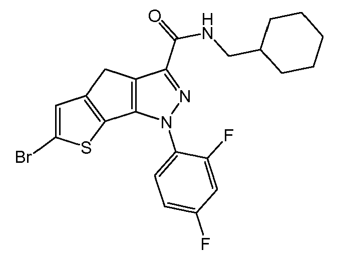

The same procedure described in ex. 3.1 was repeated but the starting compound of the synthesis was the carboxylic acid prepared in ex. 2.8 instead of the compound prepared in ex. 2.1. Besides, the amine dripped in the reaction mixture was cyclohexylmethylamine instead of (-)-*cis*-myrtanylamine. Yield 45%. Rf=0.51 (petroleum ether/ethyl acetate 8/2 v/v). IR (nujol) (λ = cm⁻¹) 3423, 1664; ¹H-NMR (CDCl₃) δ 0.95-1.05 (m, 2H); 1.13-1.30 (m, 3H); 1.55-1.84 (m, 6H); 3.26-3.32 (m, 2H); 3.74 (s, 2H); 6.98 (bt, 1H); 7.04-7.14 (m, 2H); 7.16 (s, 1H); 7.64-7.72 (m, 1H). Anal. calc. for C₂₂H₂₀BrF₂N₃OS: C, 53.66; H, 4.09; Br, 16.23; F, 7.72; N, 8.53; S, 6.51. Found: C, 53.49; H, 4.08; Br, 16.21; F, 7.70; N, 8.51; S, 6.50.

### EXAMPLE 3.12

### Preparation of N-myrtanyl-6-bromo-1-(2',4'-difluorophenyl)-1,4-dihydro-thieno[3',2':4,5]cyclopenta[1,2-c]pyrazole-3-carboxamide

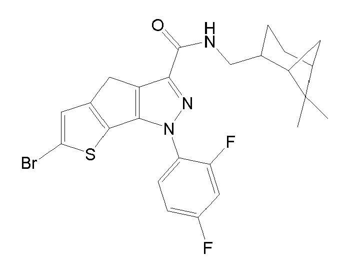

The same procedure described in ex. 3.1 was repeated but the starting compound of the synthesis was the carboxylic acid prepared in ex. 2.8 instead of the compound prepared in ex. 2.1. Yield 41%. Rf=0.41 (petroleum ether/ethyl acetate 8.5/1.5 v/v). IR (nujol) (λ = cm⁻¹) 3409, 1667; ¹H-NMR (CDCl₃) δ 0.92 (d, 1H, J = 9.5 Hz); 1.09 (s, 3H); 1.21 (s, 3H); 1.53-1.73 (m, 1H); 1.82-2.12 (m, 5H); 2.29-2.41 (m, 2H); 3.35-3.54 (m, 2H); 3.74 (s, 2H); 6.94 (bt, 1H); 7.04-7.14 (m, 2H); 7.17 (s, 1H); 7.63-7.73 (m, 1H). Anal. calc. for C₂₅H₂₄BrF₂N₃OS: C, 56.39; H, 4.54; Br, 15.01; F, 7.14; N, 7.89; S, 6.02. Found: C, 56.33; H, 4.53; Br, 14.99; F, 7.12; N, 7.87; S, 6.00.

### EXAMPLE 3.13

### Preparation of N-myrtanyl-1-(2',4'-difluorophenyl)-6-phenyl-1,4-dihydro-thieno[3',2':4,5]cyclopenta[1,2-c]pyrazole-3-carboxamide

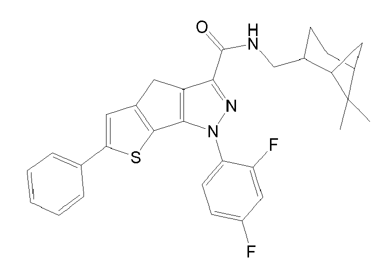

To a mixture formed of *N*-myrtanyl-6-bromo-1-(2',4'-difluoro phenyl)-1,4-dihydro-thieno[3',2':4,5]cyclopenta[1,2-*c*] pyrazole-3-carboxamide obtained in ex. 3.12 (0.25 g, 0.49 mmol), tetrakis(triphenylphosphine)palladium (0.03 g, 0.024 mmol) and phenylboronic acid (0.07 g, 0.59 mmol) in DME (5 ml), a solution of sodium carbonate (0.07 g, 0.64 mmol) in water (2.5 ml) was added. The reaction mixture was heated to reflux for 18 hours, then cooled to room temperature, poured in water, and extracted with CH₂Cl₂. The combined organic extracts were washed with water, dried over anhydrous sodium sulfate, filtered, and evaporated under reduced pressure. The residue thus obtained was purified by flash chromatography (petroleum ether/ethyl acetate 7.5/2.5 v/v) 0.24 g (92% yield) of the compound *N*-myrtanyl-1-(2',4'-difluorophenyl)-6-phenyl-1,4-dihydro-thieno[3',2':4,5]cyclopenta [1,2-*c*]pyrazole-3-carboxamide as a white solid, were recovered. Rf=0.38 (petroleum ether/ethyl acetate 7.5/2.5 v/v). IR (nujol) (λ = cm⁻¹) 3411, 1668; ¹H-NMR (CDCl₃) δ 0.93 (d, 1H, J = 9.5 Hz); 1.10 (s, 3H); 1.22 (s, 3H); 1.53-1.72 (m, 1H); 1.82-2.09 (m, 5H); 2.30-2.42 (m, 2H); 3.38-3.57 (m, 2H); 3.80 (s, 2H); 6.97 (bt, 1H); 7.05-7.18 (m, 2H); 7.24-7.32 (m, 1H); 7.33-7.43 (m, 3H); 7.58 (d, 2H, J = 7.3 Hz); 7.66-7.76 (m, 1H). Anal. calc. for C₃₁H₂₉F₂N₃OS: C, 70.30; H, 5.52; F, 7.17; N, 7.93; S, 6.05. Found: C, 70.25; H, 5.51; F, 7.16; N, 7.91; S, 6.03.

### EXAMPLE 3.14

### Preparation of N-myrtanyl-1-(2',4'-difluorophenyl)-6-(2-thienyl)-1,4-dihydro-thieno[3',2':4,5]cyclopenta[1,2-c]pyrazole-3-carboxamide

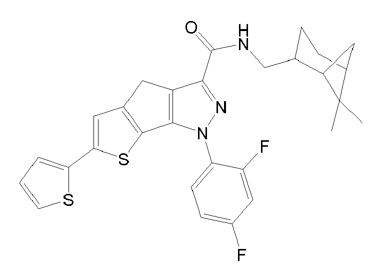

The same procedure described in ex. 3.13 was repeated but substituting phenylboronic acid with 2-thienylboronic acid. Yield 79%. Rf=0.52 (petroleum ether/ethyl acetate 7.5/2.5 v/v). IR (nujol) (λ = cm⁻¹) 3420, 1670; ¹H-NMR (CDCl₃) δ 0.92 (d, 1H, J = 9.9 Hz); 1.10 (s, 3H); 1.22 (s, 3H); 1.54-1.65 (m, 1H); 1.82-2.07 (m, 5H); 2.30-2.41 (m, 2H); 3.36-3.56 (m, 2H); 3.77 (s, 2H); 6.96 (bt, 1H); 6.98-7.04 (m, 1H); 7.04-7.18 (m, 3H); 7.19-7.27 (m, 2H); 7.65-7.74 (m, 1H). Anal. calc. for C₂₉H₂₇F₂N₃OS₂: C, 65.02; H, 5.08; F, 7.09; N, 7.84; S, 11.97. Found: C, 64.96; H, 5.07; F, 7.07; N, 7.81; S, 11.94.

### EXAMPLE 3.15

### Preparation of 1-(2',4'-dichlorophenyl)-6-methyl-3-(1-oxo-2-cyclo hexyleth-1-yl)-1,4-dihydro-thieno[3',2':4,5]cyclopenta[1,2-c] pyrazole

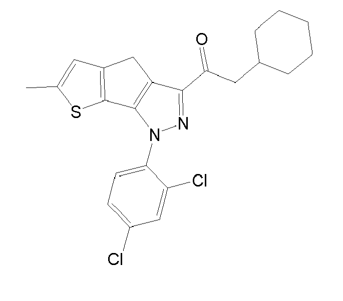

### 3.15a Preparation of N-methoxy-N-methyl-1-(2',4'-dichlorophenyl)-6-methyl-1,4-dihydro-thieno[3',2':4,5]cyclopenta[1,2-c]pyrazole-3-carboxamide

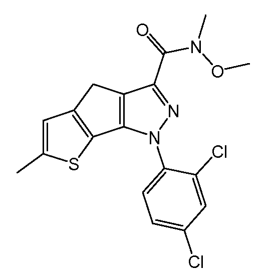

Trimethylaluminum (0.92 ml of a 2 M solution in hexane, 1.84 mmol) was added dropwise to a suspension of dimethylhydroxylamine hydrochloride (0.18 g, 1.84 mmol) in CH₂Cl₂ (3 ml) at 0°C. The reaction mixture was stirred at 0 °C for 45 minutes and then at room temperature for 40 minutes. At the end a solution was obtained. A solution in CH₂Cl₂ (2 ml) of the compound obtained in ex. 1.7 (0.4 g, 0.92 mmol) was added dropwise under stirring. Stirring was continued for further 4 hours at room temperature. The reaction mixture was then cooled to 0 °C, and 10% HCl was carefully added dropwise. The mixture was extracted with CH₂Cl₂, washed with water, brine, dried over Na₂SO₄, and filtered. The residue obtained after evaporation of the solvent under reduced pressure was purified by flash chromatography (petroleum ether/ethyl acetate 7/3 v/v), obtaining the compound *N*-methoxy-*N*-methyl-1-(2',4'-dichlorophenyl)-6-methyl-1,4-dihydro-thieno [3',2' :4,5]cyclopenta[1,2-*c*]pyrazole-3-carboxamide as a white solid. Yield 66%. Rf=0.39 (petroleum ether/ethyl acetate 6/4 v/v). IR (nujol) (λ= cm⁻¹) 1686; ¹H-NMR (CDCl₃) δ2.51 (s, 3H); 3.53 (bs, 3H); 3.63 (s, 2H); 3.82 (s, 3H); 6.82 (s, 1H); 7.41 (dd, 1H, J = 2.2 and 8.6 Hz); 7.53 (d, 1H, J = 8.6 Hz); 7.61 (d, 1H, J = 1.9 Hz). Anal. calc. for C₁₈H₁₅Cl₂N₃O₂S: C, 52.95; H, 3.70; Cl, 17.37; N, 10.29; S, 7.85. Found: C, 52.90; H, 3.69; Cl, 17.35; N, 10.27; S, 7.84.

### 3.15b Preparation of 1-(2',4'-dichlorophenyl)-6-methyl-3-(1-oxo-2-cyclohexyleth-1-yl)-1,4-dihydro-thieno[3',2':4,5]cyclopenta[1,2-c]pyrazole

3.86 ml of a 0.5 M solution of cyclohexylmethylmagnesium bromide in THF were added dropwise at 0°C, under nitrogen, to 6 ml of a THF solution containing 0.29 g (0.64 mmol) of the compound obtained in ex. 3.15a. The reaction mixture was slowly warmed to room temperature and stirred at said temperature for 24 hours. The temperature of the mixture was then lowered to 0°C. 15 ml of a saturated NH₄Cl water solution, maintained at 0°C, were added dropwise. The reaction mixture was again warmed up to room temperature, then it was diluted with ethylacetate (15 ml). The aqueous and the organic phases were separated. The aqueous layer was extracted with ethylacetate (3x10 ml), and the combined organic layers were washed with water, dried (Na₂SO₄), and filtered. The residue obtained after evaporation of the solvent under reduced pressure was purified by flash chromatography (petroleum ether/diethyl ether 9/1 v/v) obtaining the compound 1-(2',4'-dichlorophenyl)-6-methyl-3-(1-oxo-2-cyclohexyleth-1-yl)-1,4-dihydro-thieno[3',2':4,5]cyclopenta[1,2-*c*]pyrazole. Yield 46%. Rf=0.38 (petroleum ether/diethyl ether 9/1 v/v). IR (nujol) (λ = cm⁻¹) 1684; ¹H-NMR (CDCl₃) δ 1.00-1.13 (m, 2H); 1.14-1.38 (m, 4H); 1.60-1.82 (m, 4H); 2.00-2.13 (m, 1H); 2.52 (s, 3H); 2.94 (d, 2H, J = 7.0 Hz); 3.66 (s, 2H); 6.83 (s, 1H); 7.43 (dd, 1H, J = 2.2 and 8.6 Hz); 7.54 (d, 1H, J = 8.6 Hz); 7.63 (d, 1H, J = 2.2 Hz). Anal. calc. for C₂₃H₂₂Cl₂N₂OS: C, 62.02; H, 4.98; Cl, 15.92; N, 6.29; S, 7.20. Found: C, 61.96; H, 4.97; Cl, 15.90; N, 6.28; S, 7.18.

### EXAMPLE 3.16

### Preparation of 1-(2',4'-dichlorophenyl)-6-methyl-3-(1-hydroxy-2-cyclohexyleth-1-yl)-1,4-dihydro-thieno[3',2':4,5]cyclopenta[1,2-c]pyrazole

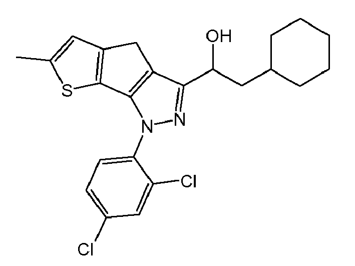

To a suspension of the keto compound prepared in ex. 3.15 (60 mg, 0.12 mmol) in methyl alcohol (3 ml) sodium borohydride (10 mg, 0.25 mmol) was added, and the mixture stirred at room temperature for 2 hours. The reaction mixture was then diluted with CHCl₃ and washed with water. The organic layer was dried over anhydrous sodium sulfate, filtered, and concentrated under reduced pressure. The compound 1-(2',4'-dichlorophenyl)-6-methyl-3-(1-hydroxy-2-cyclohexyleth-1-yl)-1,4-dihydro-thieno[3',2':4,5]cyclopenta[1,2-*c*]pyrazole was obtained. Yield 95%. Rf=0.22 (petroleum ether/ethyl acetate 8/2 v/v). IR (nujol) (λ = cm⁻¹) 3319; ¹H-NMR (CDCl₃) δ 0.95-1.08 (m, 2H); 1.13-1.32 (m, 4H); 1.51-1.61 (m, 1H); 1.61-1.74 (m, 3H); 1.75-1.90 (m, 3H); 2.42 (bs, 1H); 2.51 (s, 3H); 3.42-3.55 (m, 2H); 4.93-5.02 (m, 1H); 6.79 (s, 1H); 7.37 (dd, 1H, J = 2.2 and 8.6 Hz); 7.48 (d, 1H, J = 8.6 Hz); 7.58 (d, 1H, J = 2.2 Hz). Anal. calc. for C₂₃H₂₄Cl₂N₂OS: C, 61.74; H, 5.41; Cl, 15.85; N, 6.26; S, 7.17. Found: C, 61.67; H, 5.40; Cl, 15.83; N, 6.25; S, 7.14.

### EXAMPLE 3.17

### Preparation of N-myrtanyl-6-bromo-1-(2',4'-dichlorophenyl) -1,4-dihydro-thieno[3',2':4,5]cyclopenta[1,2-c]pyrazole-3-carboxamide

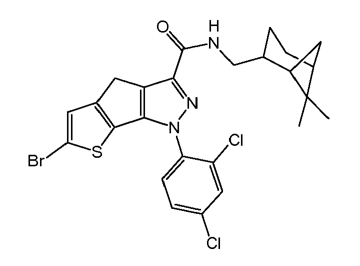

The same procedure described in ex 3.1 was repeated but the starting compound of the synthesis was the carboxylic acid prepared in ex. 2.9 instead of the compound prepared in Ex. 2.1. Yield 74%. Rf=0.48 (petroleum ether/ethyl acetate 8/2 v/v). IR (nujol) (λ = cm⁻¹) 3398, 1665; ¹H-NMR (CDCl₃) δ 0.96 (d, 1H, J = 9.6 Hz); 1. 16 (s, 3H); 1.28 (s, 3H); 1.63-1.68 (m, 1H); 1.92-2.07 (m, 5H); 2.37-2.44 (m, 2H); 3.41-3.61 (m, 2H); 3.83 (s, 2H); 7.01 (bs, 1H); 7.23 (s, 1H); 7.51 (dd, 1H, J= 2.0 and 8.5 Hz); 7.58 (d, 1H, J= 8.5 Hz); 7.70 (d, 1H, J= 2.0 Hz). Anal. calc. for C₂₅H₂₄BrCl₂N₃OS: C, 53.11; H, 4.28; Br, 14.13; Cl, 12.54; N, 7.43; S, 5.67. Found: C, 53.10; H, 4.29; Br, 14.13; Cl, 12.53; N, 7.42; S, 5.68.

### EXAMPLE 3.18

### Preparation of N-myrtanyl-1-(2',4'-dichlorophenyl)-6-(3-amino phenyl)-1,4-dihydro-thieno[3',2':4,5]cyclopenta[1,2-c]pyrazole-3-carboxamide

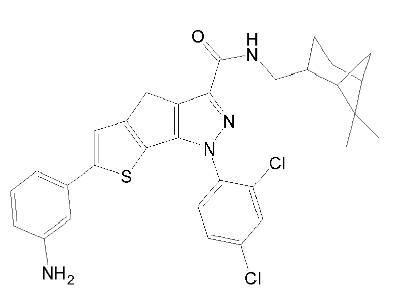

The same procedure described in ex. 3.13 was repeated but with the compound prepared in ex. 3.17 instead of *N*-myrtanyl-6-bromo-1-(2',4'-difluorophenyl)-1,4-dihydrothieno[3',2':4,5]cyclopenta [1,2-*c*]pyrazole-3-carboxamide. Besides, phenylboronic acid was substituted with 3-aminophenylboronic acid. Yield 79%. Rf=0.28 (petroleum ether/ethyl acetate 7/3 v/v). IR (nujol) (λ = cm⁻¹) 3410, 1665; ¹H-NMR (CDCl₃) δ 0.83 (d, 1H, J = 9.3 Hz); 1.06 (s, 3H); 1.16 (s, 3H); 1.50-1.58 (m, 1H); 1.77-1.95 (m, 5H); 2.25-2.35 (m, 2H); 3.11-3.38 (m, 2H); 3.73 (s, 2H); 5.19 (s, 2H); 6.47 (dd, 1H, J= 1.0 and 7.9 Hz); 6.79-6.82 (m, 2H); 6.99-7.04 (m, 1H); 7.52 (s, 1H); 7.71 (dd, 1H, J= 2.3 and 8.5 Hz); 7.81 (d, 1H, J= 8.5 Hz); 8.05 (d, 1H, J= 2.3 Hz); 8.28-8.34 (m, 1H). Anal. calc. for C₃₁H₃₀Cl₂N₄OS: C, 64.47; H, 5.24; Cl, 12.28; N, 9.70; S, 5.55. Found: C, 64.46; H, 5.22; Cl, 12.29; N, 9.79; S, 5.56.

### EXAMPLE 3.19

### Preparation of N-myrtanyl-1-(2',4'-dichlorophenyl)-6-(4-methyl phenyl)-1,4-dihydro-thieno[3',2':4,5]cyclopenta[1,2-c]pyrazole-3-carboxamide

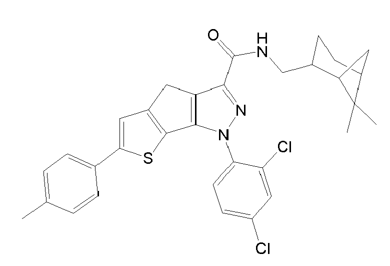

The same procedure described in ex. 3.13 was repeated but with the compound prepared in ex. 3.17 instead of *N*-myrtanyl-6-bromo-1-(2',4'-difluorophenyl)-1,4-dihydro-thieno[3',2':4,5]cyclopenta [1,2-*c*]pyrazole-3-carboxamide. Besides, phenylboronic acid was substituted with 4-methylphenylboronic acid. Yield 51%. Rf=0.24 (petroleum ether/ethyl acetate 8.5/1.5 v/v). IR (nujol) (λ - cm⁻¹) 3412, 1664; ¹H-NMR (CDCl₃) δ 0.98 (d, 1H, J = 9.6 Hz); 1.16 (s, 3H); 1.28 (s, 3H); 1.60-1.63 (m, 1H); 1.92-2.10 (m, 5H); 2.38-2.48 (m, 5H); 3.41-3.61 (m, 2H); 3.87 (s, 2H); 7.03 (bs, 1H); 7.24 (bd, 2H, J= 7.9 Hz); 7.41 (s, 1H); 7.51-7.54 (m, 3H); 7.62 (d, 1H, J= 8.5 Hz); 7.73 (d, 1H, J= 2.2 Hz). Anal. calc. for C₃₂H₃₁Cl₂N₃OS: C, 66.66; H, 5.42; Cl, 12.30; N, 7.29; S, 5.56. Found: C, 66.65; H, 5.40; Cl, 12.29; N, 7.28; S, 5.57.

### EXAMPLE 3.20

### Preparation of N-myrtanyl-1-(2',4'-dichlorophenyl)-6-(2-methoxy phenyl)-1,4-dihydro-thieno[3',2':4,5]cyclopenta[1,2-c]pyrazole-3-carboxamide

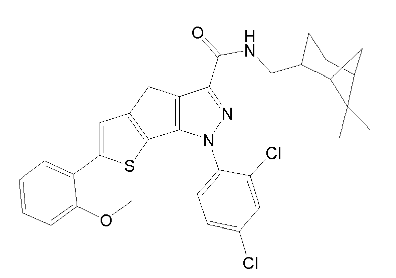

The same procedure described in ex. 3.13 was repeated but with the compound prepared in ex. 3.17 instead of *N*-myrtanyl-6-bromo-1-(2',4'-difluorophenyl)-1,4-dihydro-thieno[3',2':4,5]cyclopenta[1, 2-*c*]pyrazole-3-carboxamide. Besides, phenylboronic acid was substituted with 2-methoxyphenylboronic acid. Yield 51%. Rf=0.24 (petroleum ether/ethyl acetate 8.5/1.5 v/v). IR (nujol) (λ = cm⁻¹) 3420, 1666; ¹H-NMR (CDCl₃) δ 0.91 (d, 1H, J = 9.6 Hz); 1.09 (s, 3H); 1.21 (s, 3H); 1.58-1.64 (m, 1H); 1.86-2.04 (m, 5H); 2.33-2.39 (m, 2H); 3.35-3.55 (m, 2H); 3.80 (s, 2H); 3.93 (s, 3H); 6.94-7.01 (m, 3H); 7.24-7.28 (m, 1H); 7.45 (dd, 1H, J= 2.2 and 8.5 Hz); 7.51-7.57 (m, 2H); 7.61 (dd, 1H, J= 1.7 and 6.5 Hz); 7.65 (d, 1H, J= 2.2 Hz). Anal. calc. for C₃₂H₃₁Cl₂N₃O₂S: C, 64.86; H, 5.27; Cl, 11.97; N, 7.09; S, 5.41. Found: C, 64.85; H, 5.25; Cl, 11.99; N, 7.06; S, 5.43.

### EXAMPLE 4

### Affinity of the compounds of the invention towards the CB1 and CB2 cannabinoidergic receptors

The affinity of the synthesized compounds towards the CB1 and CB2 cannabinoidergic receptors was evaluated in vitro by radioreceptor binding studies using the method reported hereinunder.

The technique of the receptor binding allows to establish if, and with which affinity and specificity, a specific compound binds to a particular receptor. To evaluate the affinity of a specific compound to a particular receptor it is necessary to challenge in a particular tissue preparation wherein those specific receptors are present the compound to be tested with a radioactive labelled compound whose affinity for the same receptors is known. The ability of the compound under test to displace the radioactive compound from the receptor site gives an index of the affinity of the compound under test for that specific receptor. The amount of radioactivity present in the receptor-compound complex allows furthermore to estimate with great accuracy the amount of compound bound to the receptor. By said method it is therefore possible to stablish quickly the affinity of a new compound towards a specific receptor and thus to determine its pharmacological activity. With the same experimental protocol it is possible to evaluate the affinity of the compound towards other receptors and thus establish its specificity degree toward said other receptors.

The receptor binding technique, besides being used for the screening of new molecules with pharmacological activity, can give useful information on possible changes at receptor level, related for example to a prolonged exposure to drugs and/or to particular pathologies. In these conditions, indeed, changes in the amount of the receptors, or conformational changes can take place that alter the binding affinity of the agonists or antagonists, therefore affecting the functionality of the receptors themselves.

The experimentation has been carried out according to the guide lines of the European Community for the animal experimentation (EEC n. 86/609), by using laboratory animals (mice) lodged twenty per cage, under standard stabulation conditions (temperature 22±2°C, relative humidity 60%, artificial lighting with light/dark cycle of 12 hours). The food and water were ad libitum.

The procedure adopted, based on the use of the compound [³H]-CP-55,940 (New England Nuclear, Boston, MA, USA), requires the use of the mouse brain as biological tissue for the evaluation of the affinity towards the CB1 receptors and of the mouse spleen for the affinity assay for the CB2 receptors.

The animals were sacrificed by cervical dislocation and the complete brain (excluding the cerebellum) and the spleen were quickly dissected and kept in ice.

The tissue was homogeneized in 15 volumes (weight/volume) of TME buffer (50 Mm Tris, 1 mM EDTA and 3 mM MgCl₂, pH 7.4) by an Ultra-Turrax and subsequently centrifuged for 10 minutes at 1086 x g in a centrifuge refrigerated at 4°C. The recovered supernatant was centrifuged at 45,000 x g for 30 minutes at 4°C by using a Beckman SW41 rotor and the obtained pellet was resuspended in 50 volumes of TME.

The thus obtained membranes (50-80 µg of proteins) were incubated in the presence of 1 nM of [³H]-CP55.940 for 1 hour at 30°C in a final volume of 0.5 ml of TME buffer containing 5 mg/ml of bovine serum albumin (BSA). The non specific binding was measured in the presence of CP55.940 at a 1 µM concentration.

All the experiments were carried out in polypropylene test tubes pretreated with Sigma-Cote (Sigma Chemical Co. Ltd., Poole, UK) for reducing non specific binding.

In order to determine the competitive inhibition binding curves, eight different concentrations of each compound were used. As reference compounds, SR141716A was used for the CB1 receptors and the compound SR144528 was used for the CB2 receptors.

Incubation was stopped by addition of TME buffer (at 4°C) containing 5 mg/ml of BSA, and subsequent filtration under vacuum by Whatman GFC filters pretreated with 0.5% of polyethylamine (PEI) and by using a filtering device (Brandell, Gaithersburg, MD, USA). The filters were washed 3 times with 5 ml of Tris HCl buffer (pH 7.4, 4°C) containing 1 mg/ml of BSA and separately placed in plastic vials containing 4 ml of scintillating liquid (Ultima Gold MV, Packard).

The radioactivity present in the filters was determined by a scintillator spectrophotometer (Tricarb^{®} 2100, Packard, Meridien, USA).

Protein determination was carried out by the Bradford method by using the protocol and the reactants supplied by Bio-Rad (Milano, Italy).

The experiments were carried out in triplicate and the results confirmed in five independent experiments.

The affinity of the compounds towards the CB1 and CB2 receptors has been expressed in Ki terms.

The Ki values, obtained with the compounds of the present invention in the test in vitro, are reported in Table 1. By comparison, in the Table, the affinity values of the reference compounds SR144528 and SR141716A (Rimonobant^{®}) are reported.

The Table shows that the compounds of the present invention have activity on the CB1 and/or CB2 receptors comparable with that of the reference compounds.

### EXAMPLE 5

### Hypothermia tests in vivo

It is known that the compounds having a cannabomimetic activity show in vivo the following effects: hypoactivity, hypothermia, analgesia and catalepsy (B.R.Martin et al., Pharmacol. Biochem. Behav.; 1991, 40, 471-478; P.B. Smith et al.; J. Pharmacol. Exp. Ther. ; 1994, 270, 219-227). In order to display thermoregulating activity, the compounds acting towards the cannabinoidergic receptors must be able to pass the haematoencephalic barrier since the central site of said temperature regulating receptors is positioned in the preoptical front core of the hypothalamus (S.M. Rawls et al.; J. Pharmacol. Exp. Ther.; 2002, 303, 395-402). After treatment with CB1 agonist compounds passing the haemato-encephalic barrier, the cannabimimetic activity is evidenced by a reduction of the body temperature. CB1 antagonist compounds that pass the haemato-encephalic barrier, have no influence on the body temperature and show an antagonist activity towards the reference CB1 agonists as WIN 55.212-2, thus hindering the hypothermia induced by the latter.

In order to evaluate the property of the compounds of general formula (I) to cross the haematoencephalic barrier, tests have been carried out in order to check whether said compounds induced hypothermia. The tests have been carried out in the experimental animal (mice) according to the indications of M.Rinaldi-Carmona et al. in FEBS Letters; 1994, 350, 240-244. Rectal temperature in mice was determined by an electronic thermometer inserted at a 2 mm depth. The measurements were carried out on mice acclimatized for one hour. The rectal temperature was determined before and after (from 30 to 120 minutes) i.p. the administration of the compound to be under test.

The experiment was carried out with the compound of example 3.1. Said compound was dispersed in a carrier formed of a physiological solution added with Tween 80 (3 drops). The so prepared samples were used for treating the animals at a dose of the compound of 40 mg/kg of body weight. The blank was the carrier.

Each test was repeated on ten animals: the results obtained are therefore the average of the determinations made on ten animals.

The results are reported in Table 2 and show that the compounds of the invention having affinity for the CB1 receptors (see example 4) are capable to pass the haematoencephalic barrier, as they induce hypothermia in the experimental animals.

In fact the compound of example 3.1 at the administered dose of 40 mg/Kg was able to significantly reduce the body temperature in the confront of the carrier. The compound of example 3.1 is therefore effective on the CB1 agonist central nervous system.

### EXAMPLE 6

### Evaluation of the activity of the compounds of the invention in the reduction of the ocular pressure

The main risk factor in the glaucoma is represented by the high intraocular pressure (IOP). See for instance: Libby R.T. et al., Vis Neurosci. 2005 Sep-Oct, 22(5):637-48 and Tomida I. et al., Br. J. Ophthalmol. 2004 May, 88(5):708-13. Among the various experimental animal models wherein a high intraocular pressure correlated to glaucoma is obtained, the animal model based on the use of old DBA/2J mice (6-10 months age) with high IOP is considered one the most effective for selecting potential therapeutic agents for clinical use, since the mouse eye physiology is similar to that of the human eye (Zhong et al., Invest. Ophthalmol. Vis. Sci. 2007 March, 48(3); F.Schuettauff et al., Acta Neuropathol. (2004) 107 : 352-358; S.W.M.John et al., Invest. Ophthalmol. Vis. Sci. 1998 May, 39(6):951-962 e F.Schuettauf et al., Vision Res. 2002 September, 42 (20) :2333-2337) .

Pharmacological and histological studies have shown the direct involvement of the cannabinoid receptors in the IOP reduction. Porcella et al. have found high levels of mRNA CB1 in the ciliary body (Porcella et al., Brain Res. Mol. Brain Res., 1998 July 15, 58(1-2):240-245) while Lu and colleagues have pointed out the presence of mRNA CB2 in the retina (Q.Lu et al., Vis. Neurosci. 2000 January-February, 17(1):91-95). The activation of the CB1 and/or CB2 receptors appears therefore a pharmacological target of interest for the modulation of the intraocular pressure, for the therapeutical benefits in the glaucoma treatment. Very positive results have been for example reported by Porcella et al. in European Journal of Neuroscience, 2001, Vol. 13, 409-412, wherein it is mentioned the significant IOP reduction in men after local administration in the eye of the cannabinoidergic compound WIN 55212-2.

By using the animal model of old DBA/2J mice (see above) it has been evaluated the efficacy of the compound obtained in example 3.1 in reducing the IOP. As said, this compound has a good affinity towards the CB1 receptors in vitro (example 4) and a CB1 agonist action in vivo (example 5). The intraocular pressure has been determined by a TONOLab Tonometer (Tiolat Oy), as reported by M.E.Pease et al. in J. Glaucoma, 2006 December, 15(6):512-519. The compound was dispersed in the commercial emulsion Tocrisolve^{™}, (Tocris), at a concentration of 5 mg/ml and applied to the eye of old DBA/2J mice (8-10 month age; basal IOP comprised between 19.0 and 22.5 mmHg). The applied emulsion volumes were such that the compound doses resulted, respectively, of 100 µg or 50 µg. WIN 55212-2 was dispersed in Tocrisolve^{™} at the concentration of 5 mg/ml and was the reference cannabinoidergic compound. The determination of the ocular pressure was carried out every 30 minutes up to two hours starting from the administration. The obtained results (average of 6 measurements at each time for experimental groups of 8-10 animals for group/dose) are reported in Table 3. IOP decrease was expressed in per cent with respect to the basal value.

The results show that the commercial emulsion Tocrisolve^{™} (20 and 40 µl) has no effect on the IOP. Furthermore, at the dose of 100 µg the compound of example 3.1 was as effective to reduce eye pressure as the reference compound WIN 55212-2. Further, at the dose of 50 µg the compound of the present invention was more effective in reducing IOP than the reference compound.

### EXAMPLE 7

### Evaluation of the activity of the compounds of the invention in an ex-vivo model based on the use of the vas deferens

The use of the isolated deferent vessel organ, as a highly sensitive basic system in an ex-vivo model for evaluating the activity of agonist CB1 compounds, has been described by Pertwee et al. in Br. J. Pharmacol., 105:980-4, 1992. This model has been used also to evaluate the properties of antagonist CB1 compounds and to distinguish antagonist CB1 derivatives from inverse agonist CB1 compounds (Rinaldi-Carmona et al., FEBS Lett., 350:240-4, 1994; Pertwee et al., Eur. J. Pharmacol., 456:99-106, 2002). The model is based on the activity of the agonist CB1 or partial agonist CB1 compounds of decreasing in a dose-depending way the amplitude of the contractions electrically induced on the mouse deferent vessel. This effect is correlated to the action on the prejunctional neuronal CB1 receptors with the consequent release inhibition, induced by electric stimuli, of contractile neurotransmitters (noradrenalin and ATP) (Schlicker and Kathmann, Trends Pharmacol. Sci., 22:565-72, 2001). The inhibition of the contractions induced by the electric stimulus on the deferent vessel in the presence of agonist (or partial agonist) CB1 compounds are expressed as % of contraction inhibition. Said % contraction inhibition is calculated by the ratio between the contraction amplitude determined in the presence of a given concentration of the compound and that detected in its absence (basal value). The curve of the "% of contraction inhibition" as a function of the logarithm of the concentration of agonist (or partial agonist) CB1 compounds, is sigmoidal. The use of the model for the evaluation of antagonist CB1 compounds is based on the evaluation of their efficacy in inhibiting the action of the deferent vessel of agonist (or partial agonist) CB1 compounds. The inhibition is expressed by a shifting towards the right of the curves % of inhibition of contractions/logarithm of the concentration of the agonist CB1 compound, with increasing the concentration of the antagonist CB1 compound.

As reported in the literature, the experimentation has been carried out by using albino CD1 mice weighing 30-40 grams. The mice deferent vessel was recovered and transferred into an instrument for separated organs supplied by the Firm Ugo Basile (Comerio, Italy). The initial tension was of 0.5 g. The procedure adopted is reported in Pertwee et al. in Br. J. Pharmacol., 110:1483-1490, 1993. In particular the procedure and instruments as described by S.Ruiu et al. in J.P.E.T., Vol. 306, 2003, 363-370, have been used.

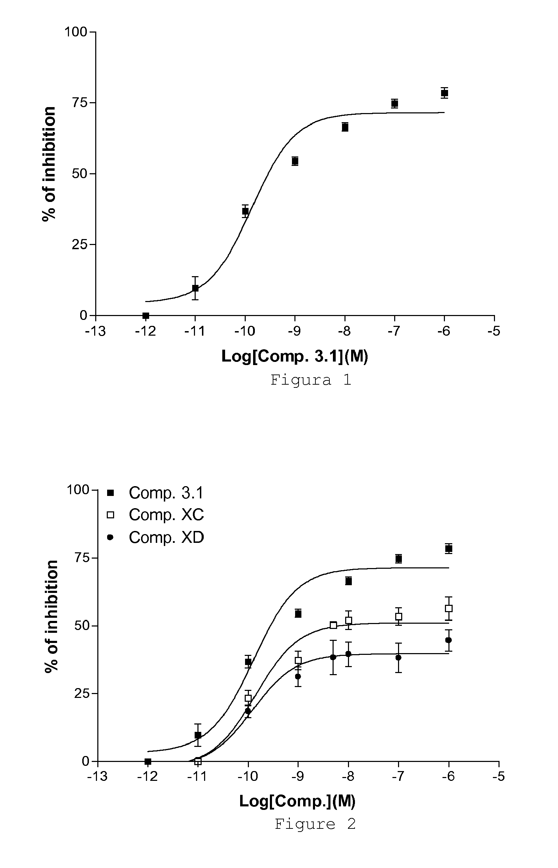

Figure 1 shows the results expressed as an average of seven different experiments, and shows that the compound under tes was effective in inhibiting the contractions induced by an electric stimulus.

In a subsequent experiment it was shown that the compound activity was antagonized by the antagonist CB1 compound *N-*piperidinyl-[8-chloro-1-(2,4-dichlorophenyl)-1,4,5,6-tetrahydrobenzo[6,7] cyclohepta[1,2-*c*]pyrazol-3-carboxamide], described by S.Ruiu et al. in J.P.E.T., Vol. 306, 2003, 363-370. The experiment was carried out by adding the antagonist (concentration in bath 1 nM) 20 minutes before the first addition of the compound of the invention. This experimental model shows that the compound of example 3.1 is a CB1 agonist.

### EXAMPLE 8.1 (Comparative)

### Preparation of ethyl α-(4-oxo-4,5,6,7-tetrahydro-benzo-thiophen-5-yl)-α-oxo-acetate

The same procedure described in example 1.1a was followed but using 5*H*-6,7-dihydro-benzo[*b*]thiophen-4-one (13.14 mmoles) instead of 2-methyl-4*H*-5,6-dihydro-cyclopenta[*b*]thiophen-6-one. The yield is quantitative. Rf = 0.20 (AcOEt/oil ligroin 8:2); IR (nujol) (λ= cm⁻¹) 1725, 1684; ¹H-NMR (CDCl₃) δ 1,42 (t, 3H, J = 6.6 Hz); 2.33 (t, 2H, J = 7.2 Hz); 2.58 (t, 2H, J = 7.3 Hz); 4.41 (q, 2H, J = 6.8 Hz); 6.53 (d, 1H, J = 5.4 Hz); 7.03 (d, 1H, J = 5.2 Hz); 12.77 (bs, 1H). Anal. calc. for C₁₂H₁₂O₄S: C, 57.13; H, 4.79; S, 12.71. Found: C, 57.03; H, 4.77; S, 12.66.

### Preparation of 1-(4'-sulphonamidophenyl)-4,5-dihydro-1H-thieno [2,3-g]indazol-3-ethyl carboxylate

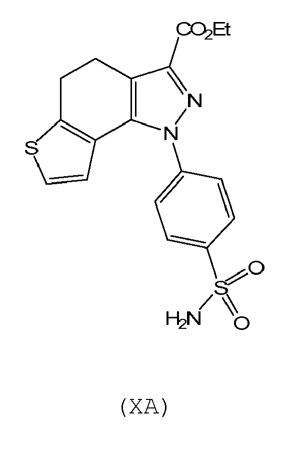

The same procedure described in example 1.1b was used by reacting the compound α-(4-oxo-4,5,6,7-tetrahydro-benzo-thiophen-5-yl)-α-oxo-ethyl acetate (3.95 mmoles) with 4-sulphonamidophenylhydrazine hydrochloride (4.55 mmoles). Yield 90%; Rf = 0.44 (AcOEt/oil ligroin 1:1); IR (nujol) (λ= cm⁻¹) 1725, 1320, 1210; ¹H-NMR (CDCl₃) δ 1.43 (t, 3H, J = 6.9 Hz); 3.05-3.22 (m, 4H); 4.44 (q, 2H, J = 6.9 Hz); 6.53 (d, 1H, J = 5.4 Hz); 6.75 (bs, 2H); 7.04 (d, 1H, J = 5.2 Hz); 7.70 (d, 2H, J = 8.7 Hz); 8.08 (d, 2H, J = 8.5 Hz). Anal. calc. for C₁₈H₁₇N₃O₄S₂: C, 53.58; H, 4.25; N, 10.41; S, 15.89. Found: C, 53.49; H, 4.24; N, 10.39; S, 15.86.

### EXAMPLE 8.2 (Comparative)

### Preparation of 1-(4'-sulphonamidophenyl)-4,5-dihydro-1H-thieno [2,3-g]indazol-3-carboxamide

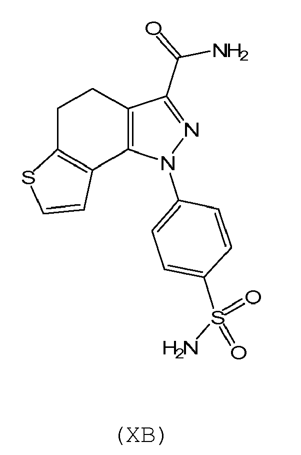

A mixture of the ester 1-(4'-sulphonamidophenyl)-4,5-dihydro-1*H*-thieno[2,3-*g*]indazol-3-ethyl carboxylate (1.3 g, corresponding to 3.22 mmoles) and of NH₄OH 33% w.(60 ml) in methanol (30 ml) was stirred at room temperature for 72 hours. The obtained dispersion was filtered. A solid was isolated, washed with water and dried in the air obtaining 0.88 g (73% yield) of the compound 1-(4'-sulphonamidophenyl)-4,5-dihydro-1*H*-theno[2,3-*g*]indazol-3-carboxamide. Rf = 0.20 (AcOEt/oil ligroin 7:3); IR (nujol) (λ= cm⁻¹) 1684, 1322, 1215; ¹H-NMR (CDCl₃) δ 3.05-3.24 (m, 4H); 3.30 (bs, 2H); 6.56 (d, 1H, J = 5.4 Hz); 7.07 (bs, 2H); 7.13 (d, 1H, J = 5.4 Hz); 7.71 (d, 2H, J = 8.4 Hz); 8.06 (d, 2H, J = 8.7 Hz). Anal. calc. for C₁₆H₁₄N₄O₃S₂: C, 51.32; H, 3.77; N, 14.96; S, 17.13. Found: C, 51.25; H, 3.76; N, 14.94; S, 17.11.

### EXAMPLE 8.3 (Comparative)

### Affinity towards the CB1 and CB2 cannabinoidergic receptors

By using the experimental model described in example 4, the affinity of the comparative compounds (XA) and (XB) towards the CB1 and CB2 receptors has been evaluated. Differently from what found with the compounds of the invention in example 4, both compound (XA) and compound (XB) did not show any significant affinity for both cannabinoid receptors. Ki values higher than 5000 nM were obtained both for CB1 receptors and for CB2 receptors.

### EXAMPLE 8.4 (Comparative)

### Evaluation of the compound activity in the ocular pressure reduction

The compounds (XA) and (XB) were dispersed in Tocrisolve^{™} and evaluated in the same experimental model described in example 6. The applied doses were the same.

Differently from what found with the compounds of the invention, both the comparative compounds (XA) and (XB), at the doses of 50 µg and 100 µg were not able to significantly reduce the intraocular pressure. See Table 4.

### EXAMPLE 8.5 (Comparative)

### Evaluation of the activity of the compounds in a model ex-vivo based on the use of the deferent vessel (vas deferens)

The experimental model described in example 7 was used. The compounds (XA) and (XB) were evaluated at concentrations lower than or equal to 1 x 10⁻⁶ M. Both compounds did not significantly inhibit the contractions induced on the deferent vessel through the electric stimulus. Besides, for concentrations lower than or equal to 1 x 10⁻⁵ M, both the comparative compounds did not significantly modify the slope of the curve % of inhibition of the contractions/logarithm of the WIN 55212-2 concentration.

The results obtained in this test show that condensed pyrazole tricyclic compounds having substituent groups -C(O)-CH₂-CH₃ or -C(O)-NH₂ at position 3 of the pyrazole ring, therefore different from those defined for R' in formula (I), have no affinity for the CB1 and/or CB2 cannabinoid receptors. Said compounds not only are not effective in reducing intraocular pressure, but they also show no activity in the test ex-vivo based on the use of the deferent vessel defined above.

### EXAMPLE 9.1 (Comparative)

### Synthesis of N-menthyl-6-methyl-1-(4'-methylbenzyl)-1,4-dihydro-thieno[3',2':4,5]cyclopenta[1,2-c]pyrazol-3-carboxamide (US 2005/0282798)

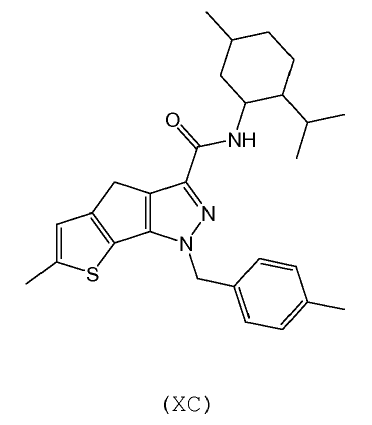

The same procedure indicated in example 3.1 was followed but substituting (-)-*cis*-myrtanylamine with methylamine. By chromatography (CHCl₃ solvent) of the reaction crude the compound, *N*-menthyl-6-methyl-1-(4'-methylbenzyl)-1,4-dihydro-thieno[3',2':4,5]cyclopenta[1,2-*c*] pyrazol-3-carboxamide was separated with a 28% yield. IR (nujol) (λ= cm⁻¹) 3400 (NH), 1670 (C=O); ¹H-NMR (CDCl₃) δ 0.79-0.95 (m, 9H); 1.00-1.27 (m, 5H); 1.63-1.80 (m, 2H); 1.92-2.19 (m, 2H); 2.34 (s, 3H); 2.48 (s, 3H); 3.58 (s, 2H); 3.80-4.06 (m, 1H); 5.33 (s, 2H); 6.62 (bd, 1H, J = 9.0 Hz); 6.76 (s, 1H); 7.11-7.28 (m, 4H). Anal. calc. for C₂₈H₃₅N₃OS: C, 72.85; H, 7.64; N, 9.10; S, 6.95. Found: C, 72.80; H, 7.62; N, 9.09; S, 6.94.

### EXAMPLE 9.2 (Comparative)

### Preparation of N- fenchyl-1-(2',4'-dichlorophenyl)-6-methyl-1,4-dihydro-thieno[3',2':4,5]cyclopenta[1,2-c]pyrazol-3-carboxamide (US 2005/0282798)

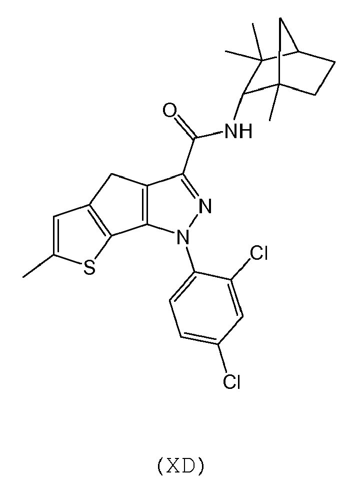

The same procedure mentioned in example 3.3 was followed but substituting (-)-*cis*-myrtanylamine with fenchylamine. The purification by chromatography (solvent CHCl₃) of the product obtained at the end of the reaction yielded the compound *N-*fenchyl-1-(2',4'-dichlorophenyl)-6-methyl-1,4-dihydro-thieno[3',2':4,5]cyclopenta [1,2-*c*]pyrazol-3-carbo-xamide (58% yield). IR (nujol) (λ= cm⁻¹) 3250 (NH), 1646 (C=O); ¹H-NMR (CDCl₃) δ 0.88 (s, 3H); 1.12 (s, 3H); 1.09-1.20 (m, 1H); 1.18 (s, 3H); 1.30-1.50 (m, 2H); 1.60-1.83 (m, 4H); 2.52 (s, 3H); 3.70 (s, 2H); 3.81 (d, 1H, J = 10.0 Hz); 6.83 (s, 1H); 7.04 (d, 1H, J = 9.8 Hz); 7.43 (dd, 1H, J = 2.2 and 8.4 Hz); 7.55 (d, 1H, J = 8.6 Hz); 7.62 (d, 1H, J = 2.0 Hz). Anal. calc. for C₂₆H₂₇Cl₂N₃OS: C, 62.40; H, 5.44; Cl, 14.17; N, 8.40; S, 6.41. Found: C, 62.26; H, 5.43; Cl, 14.14; N, 8.38; S, 6.39.

### EXAMPLE 9.3 (Comparative)

### Affinity towards the cannabinoidergic CB1 and CB2 receptors

The affinity of the comparative compounds (XC) and (XD) towards the CB1 and CB2 receptors was evaluated with the experimental model described in example 4. These compounds showed a good affinity for the CB1 receptors. The obtained Ki values (values expressed as nM) are the following:
Compound (XC): 33.0±8.2 (CB1) and 2.5±0.3 (CB2);
Compound (XD): 43.3±5.5 (CB1) and 15.0±3.2 (CB2).

### EXAMPLE 9.4 (Comparative)

### Evaluation of the activity of the compounds in the reduction of the ocular pressure

The comparative compounds (XC) and (XD) were dispersed in Tocrisolve^{™} and evaluated by the same experimental model described in example 6. The used doses were the same.

It was found that, differently from the compounds of the invention, the comparative compounds (XC) and (XD) were able to significantly reduce the intraocular pressure in old DBA/2J mice (8-10 months age) only at the highest tested dose of 100 µg. See Table 5.

### EXAMPLE 9.5 (Comparative)

### Evaluation of the activity of the compounds in a model ex-vivo based on the use of the deferent vessel (vas deferens)

The experimental model of example 7 was repeated. The comparative compounds (XC) and (XD) were evaluated at concentrations lower than or equal to 1 x 10⁻⁶ M. Both compounds were able to significantly inhibit the contractions induced in the deferent vessel by the electric stimulus. As shown in Fig. 2, the activity of the comparative compounds (XC) and (XD) resulted however lower than that of the compounds of the invention.

The results of this experiment show that the condensed pyrazole tricyclic compounds described in US 2005/0282798, having in position 3 of the pyrazole ring substituent groups different from those defined for R' in formula (I) of the compounds of the invention, and that show affinity for the CB1 and/or CB2 cannabinoid receptors, and are besides unable to pass the haemato-encephalic barrier, are less effective than the compounds of the invention both in reducing the intraocular pressure and in the experiment ex-vivo of the deferent vessel.

### EXAMPLE 10

### Preparation of a microemulsion containing the compounds of the invention

17.7 mg of the compound obtained in example 3.1 were solubilized in a mixture of: ethanol(44.2 mg) Miglyol^{®}810N (88.4 mg), Imvitor^{®}308 (88.4 mg). To the obtained solution 663.1 mg of the nonionic surfactant Solutol^{®}HS15 and 98.2 mg of physiological solution were added under stirring.

A composition in the form of a microemulsion was obtained. At temperatures comprised between 25°C and 37°C said composition is in fact liquid and isotropic.

The microemulsion compoosition (% by weight) is the following:

| | |
|---|---|
| Ethanol | 4.42 |
| Miglyol^{®}810N | 8.84 |
| Imvitor^{®}308 | 8.84 |
| Solutol^{®}HS15 | 66.31 |
| Compound ex. 3.1 | 1.77 |
| Physiological solution | 9.82 |

### EXAMPLE 11

### Preparation of a microemulsion containing the compounds of the invention

The microemulsion of example 10 was diluted with physiological solution up to a final composition containing the following components (% by weight):

| | |
|---|---|
| Ethanol | 2.23 |
| Miglyol^{®}810N | 4.45 |
| Imvitor^{®}308 | 4.45 |
| Solutol^{®}HS15 | 33.34 |
| Compound ex. 3.1 | 0.89 |
| Physiological solution | 54,54 |

It was found that the obtained formulation at temperatures comprised between 25°C and 37°C is liquid and isotropic and is therefore a microemulsion.

### EXAMPLE 12

### Preparation of an emulsion containing the compounds of the invention

The microemulsion of example 10 was diluted with physiological solution up to a final composition containing the following components (% by weight):

| | |
|---|---|
| Ethanol | 0.49 |
| Miglyol^{®}810N | 0.98 |
| Imvitor^{®}308 | 0.98 |
| Solutol^{®}HS15 | 7.35 |
| Compound ex. 3.1 | 0.20 |
| Physiological solution | 90.00 |

It was found that the obtained formulation at temperatures comprised between 25°C and 37°C is an opalescent emulsion.

### EXAMPLE 13

### Preparation of a microemulsion containing the compounds of the invention

99.1 mg of the compound obtained in example 3.1 were solubilized in ethanol (54.1 mg) and Miglyol^{®}810N (108.1 mg). To the obtained solution 648.6 mg of the nonionic surfactant Solutol^{®}HS15 and 90.1 mg of physiological solution were added under stirring.

A composition in the form of a microemulsion was obtained, which at temperatures between 25°C and 37°C is therefore liquid and isotropic.

The microemulsion composition (% by weight) is the following:

| | |
|---|---|
| Ethanol | 5.41 |
| Miglyol®810N | 10.81 |
| Solutol®HS15 | 64.86 |
| Compound ex. 3.1 | 9.91 |
| Physiological solution | 9.01 |

### EXAMPLE 14

### Preparation of a microemulsion containing the compounds of the invention

The microemulsion of example 13 was diluted with physiological solution up to a final composition containing the following components (% by weight):

| | |
|---|---|
| Ethanol | 0.65 |
| Miglyol®810N | 1.29 |
| Solutol®HS15 | 7.77 |
| Compound ex. 3.1 | 1.19 |
| Physiological solution | 89.10 |

It was found that the obtained composition is in the form of a microemulsion which at temperatures comprised between 25°C and 37°C results liquid and isotropic.

**TABLE 1**

| Example 4 : in vitro activity of the compounds of the invention and of the reference compounds on CB1 and CB2 receptors | | |
|---|---|---|
| Compound Example | CB1 (brain) Ki (nM) | CB2 (spleen) Ki (nM) |
| 3.1 | 83.6±14.6 | 48.3±2.6 |
| 3.2 | 223±25 | 5.8±1.0 |
| 3.6 | 86.4±20 | 4353±251 |
| 3.7 | 919±226 | 4.5±0.8 |
| 3.8 | 400±71 | 30.6±7.2 |
| 3.12 | 160±2.5 | 9.3±1.5 |
| 3.15 | 720±139 | 5.4±1.4 |
| SR144528 (comp) | 70±10 | 0.28±0.04 |
| SR141716A (comp) | 1.8±0.075 | 514±30 |

**TABLE 2**

| Example 5: body temperature variation after administration in mice of the compound of example 3.1 (dose 40 mg/kg) or of the carrier. The data are the average of the results obtained with ten animals. | | |
|---|---|---|
| Time from administration (minutes) | Body Temperature (°C) | |
| | Compound Example 3.1 (40 mg/kg) | Carrier |
| 0 | 37.9 | 38.0 |
| 15 | 36.1 | 37.9 |
| 30 | 34.8 | 38.1 |
| 60 | 35.0 | 38.0 |
| 90 | 36.7 | 37.9 |
| 120 | 37.2 | 37.8 |

**TABLE 3**

| Example 6: intraocular pressure (IOP) variation in old DBA/2J mice after administration of the reference compound and of the compound of example 3.1. The results are expressed as per cent decrease of the IOP with respect to the animal basal IOP value. | | | | | | |
|---|---|---|---|---|---|---|
| Time from administration (minutes) | Intraoculare Pressure Decrease (%) | | | | | |
| | Carrier | | WIN 55212-2 | | Compound Example 3.1 | |
| | 20 µl | 40 µl | 50 µg | 100 µg | 50 µg | 100 µg |
| 30 | 0.1 | 0.3 | 4.3 | 23.1 | 15.9 | 23.9 |
| 60 | -0.1 | 0.0 | 3.0 | 21.3 | 15.0 | 22.8 |
| 90 | 0.2 | -0.2 | 2.0 | 18.1 | 12.6 | 19.6 |
| 120 | 0.0 | 0.0 | 1.9 | 13.2 | 11.7 | 21.7 |

**TABLE 4**

| Example 8.4 comparative: intraocular pressure variation in old DBA/2J mice after administration of the compounds (XA) (ex. 8.1) and (XB) (ex. 8.2). Results are expressed as in Table 3. | | | | |
|---|---|---|---|---|
| Time from administration (minutes) | Intraoculare Pressure Decrease (%) | | | |
| | Compound (XA) comp. | | Compound (XB) comp. | |
| | 50 µg | 100 µg | 50 µg | 100 µg |
| 30 | -0.3 | 0.0 | -0.2 | -0.1 |
| 60 | -0.2 | -0.2 | -0.1 | 0.4 |
| 90 | 0.0 | -0.1 | 0.0 | -0.3 |
| 120 | -0.1 | 0.1 | -0.1 | 0.0 |

**TABLE 5**

| Example 9.4 comparative: intraocular pressure variation in old DBA/2J mice after administration of the compound (XC) according to US 2005/0282798 (ex. 9.1) and of compound (XD) according to US 2005/0282798 (ex. 9.2). Results are expressed as in Table 3. | | | | |
|---|---|---|---|---|
| Time from administration (minutes) | Intraocular pressure decrease (%) | | | |
| | Compound (XC) comp. | | Compound (XD) comp. | |
| | 50 µg | 100 µg | 50 µg | 100 µg |
| 30 | 2.9 | 22.2 | 3.0 | 16.6 |
| 60 | 2.2 | 22.4 | 2.0 | 14.0 |
| 90 | 0.8 | 25.0 | 0.5 | 11.2 |
| 120 | 1.5 | 16.7 | 1.0 | 11.1 |

## Claims

1. Condensed tricyclic pyrazole compounds having affinity for the CB1 and/or CB2 cannabinoidergic receptors, with activity both on the peripheral and central nervous system, of formula (I): 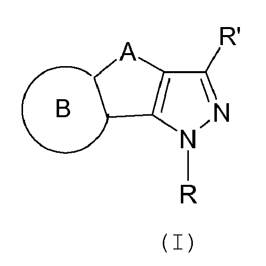 wherein:
A represents a group selected from -(CH₂)ₜ-, -(CH₂)ᵣ-O-(CH₂)ₛ- and -(CH₂)ᵣ-S(O)ₚ-(CH₂)ₛ-
wherein
t is an integer equal to 1, 2 or 3,
p is an integer equal to 0, 1 or 2,
r and s, equal to or different from each other, are integers equal to 0, 1 or 2 with the proviso that r+s is equal to 0, 1, 2 or 3,
B is an heteroaryl,
R is a group selected from heteroaryl, heteroarylalkyl, aryl, arylalkyl, arylalkenyl or bivalent C₁-C₁₀ aliphatic chain, linear or branched when possible, wherein the chain end not linked to the nitrogen atom is linked to W, W being a group selected from hydrogen, halogen, isothiocyanate, CN, OH, OCH₃, NH₂, SO₂NH₂ or -CH=CH₂,
R' is a group selected from the following:
R'₁: a substituent bearing a keto group of formula -C(O)-(Z')ᵥ-Z"
wherein Z' is a bivalent C₁-C₈ aliphatic chain, Z" is selected from C₃-C₁₅ cycloalkyl, saturated or unsaturated heterocycle, aryl, heteroaryl,
v is an integer equal to 1,
when A is -(CH₂)ᵣ-O-(CH₂)ₛ- v=0, r and s being as defined above,
R'₂: a substituent bearing an hydroxylic group, of formula -CH(OH)-(Z')ᵥ-Z",
Z', v and Z" being as defined above,
R'₃: an amide substituent of formula
-C(O)-NH-(Z')ᵥ-T',
Z' and v being as defined above and T' a group selected from:
- C₃-C₁₅ cycloaklyl,
- NR₁R₂,
wherein R₁ and R₂, equal to or different from each other, have the following meanings: hydrogen, C₁-C₇ alkyl, C₁-C₇ haloalkyl, heteroaryl, heteroarylalkyl, aryl, arylalkyl or arylalkenyl,
or
R₁ and R₂ with the nitrogen atom form a saturated or unsaturated heterocycle, containing from 5 to 10 atoms,
- C₃-C₁₅ heterocycloalkyl containing one or more heteroatoms, equal to or different from each other selected from N, O, S, with the proviso that Z' is linked to one carbon atom of the heterocycloalkyl ring, and excluding when Z' is C₁-C₃ linear alkylene chain and T' has the following formula (IB): 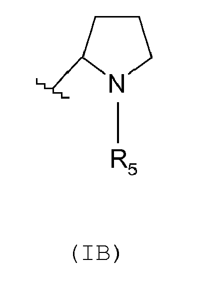 wherein R₅ represents a linear or branched when possible C₁-C₃ alkyl,
- aryl or heteroaryl, with the proviso that A is
- (CH₂)ᵣ-O-(CH₂)ₛ-, r and s being as defined above.

2. Condensed tricyclic pyrazole compounds according to claim 1, wherein the following compound is excluded from formula (I):

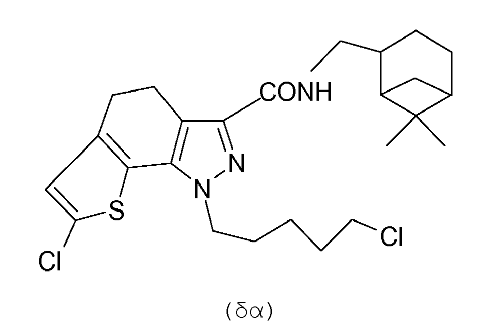

3. Tricyclic pyrazole derivatives according to claims 1-2, wherein B is substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, SO₂NH₂, cyano, nitro, isothiocyanate, phenyl, cycloalkyl, saturated or unsaturated heterocycle, heteroaryl, amino optionally mono- or bisubstituted with a C₁-C₇ alkyl chain, said phenyl, cycloalkyl, saturated or unsaturated heterocycle and heteroaryl being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, SO₂NH₂, cyano, isothiocyanate, phenyl, cycloalkyl, saturated or unsaturated heterocycle, heteroaryl, nitro, amino optionally mono- or bisubstituted with a C₁-C₇ alkyl chain.

4. Tricyclic pyrazole compounds according to claims 1-3, wherein R is heteroaryl, heteroarylalkyl, aryl, arylalkyl or arylalkenyl, and is substituted with one or more substituents, equal to or different from each other, selected from halogen, C₁-C₇ alkyl, C₁-C₇ alkylthio, C₁-C₇ alkoxy, C₁-C₇ haloalkyl, C₁-C₇ haloalkoxy, SO₂NH₂, cyano, nitro, phenyl, saturated or unsaturated heterocycle, amino optionally mono- or bisubstituted with a C₁-C₇ alkyl chain.

5. Tricyclic pyrazole compounds according to claims 1-4, wherein Z" is substituted with one or more substituents, equal to or different from each other, selected from halogen, SO₂NH₂, C₁-C₇ alkyl, C₁-C₇ haloalkyl, C₁-C₇ haloalkoxy, C₁-C₇ alkylthio, C₁-C₇ alkoxy.

6. Tricyclic pyrazole compounds according to claims 1-5, wherein T' is aryl, heteroaryl, C₃-C₁₅ cycloalkyl or C₃-C₁₅ heterocycloalkyl, and is substituted with one or more groups, equal to different from each other, selected from halogen, SO₂NH₂, C₁-C₇ alkyl, C₁-C₇ haloalkyl, C₁-C₇ haloalkoxy, C₁-C₇ alkylthio, C₁-C₇ alkoxy, phenyl, benzyl, said phenyl and benzyl substituents are optionally substituted with one or more groups, equal to or different from each other, selected from halogen, SO₂NH₂, C₁-C₇ alkyl, C₁-C₇ alkylthio, C₁-C₇ alkoxy, C₁-C₇ haloalkyl, C₁-C₇ haloalkoxy, cyano, nitro, isothiocyanate, phenyl, cycloalkyl, saturated or unsaturated heterocycle, amino optionally mono- or bisubstituted with C₁-C₇ alkyl.

7. Tricyclic pyrazole compounds according to claims 1-6, wherein R₁ and R₂ are heteroaryl, heteroarylalkyl, aryl, arylalkyl or arylalkenyl, or R₁ and R₂ with the nitrogen atom form one heterocycle, the aromatic rings and the heterocycle being substituted with one or more groups equal to or different from each other, selected from halogen, S₂NH₂, C₁-C₇ alkyl, C₁-C₇ haloalkyl, C₁-C₇ haloalkoxy, C₁-C₇ alkylthio, C₁-C₇ alkoxy, phenyl, benzyl, cyano, nitro, amino optionally mono- or bisubstituted with a C₁-C₇ alkyl chain, said phenyl and benzyl substituents are optionally substituted with one or more groups, equal to or different from each other, selected from halogen, SO₂NH₂, C₁-C₇ alkyl, C₁-C₇ alkylthio, C₁-C₇ alkoxy, C₁-C₇ haloalkyl, C₁-C₇ haloalkoxy, cyano, isothiocyanate, phenyl, cycloalkyl, saturated or unsaturated heterocycle, heteroaryl, nitro, amino optionally mono- or bisubstituted with a C₁-C₇ alkyl chain.

8. Tricyclic pyrazole compounds according to claims 1-7, wherein A, R and R' are as defined above and B is a monocyclic heteroaryl.

9. Tricyclic pyrazole compounds according to claims 1-8, wherein A is as defined above, B is an heteroaryl having a 5 or 6 atom ring, optionally substituted with one or more groups, equal to or different from each other, selected from the following: halogen, C₁-C₇ alkyl, C₁-C₇ alkylthio, C₁-C₇ alkoxy, C₁-C₇ haloalkyl, C₁-C₇ haloalkoxy, SO₂NH₂, phenyl, heteroaryl, wherein said phenyl and heteroaryl substituents are optionally substituted with one or more groups, equal to or different from each other, selected from halogen, C₁-C₇ alkyl, C₁-C₇ alkoxy, SO₂NH₂, phenyl, heteroaryl,
R is selected from the following groups:
- monocyclic heteroaryl, heteroarylalkyl, aryl, arylalkyl,
- bivalent C₁-C₁₀ aliphatic chain, linear or branched when possible, wherein the chain end not linked to the nitrogen atom is linked to W, W being as defined above, R' is a R'₁ or R'₃ group as defined above.

10. Tricyclic pyrazole compounds according to claims 1-9, wherein:
A is as defined above but excluding the meaning -(CH₂)ᵣ-S(O)ₚ-(CH₂)ₛ-,
B is an heteroaryl selected from the following: thiophene, pyridine, furan, oxazole, thiazole, imidazole, pyrazole, isoxazole, isothiazole, triazole, pyridazine, pyrimidine, pyrazine, triazine, pyrrole, said heteroaryls optionally substituted with one or more groups, equal to or different from each other, selected from: halogen, C₁-C₃ alkyl, C₁-C₃ alkylthio, C₁-C₃ alkoxy, C₁-C₃ haloalkyl, C₁-C₃ haloalkoxy, SO₂NH₂, phenyl, heteroaryl, amino optionally mono- or bisubstituted with a C₁-C₃ alkyl chain, said phenyl and heteroaryl are optionally substituted with one or more groups, equal to or different from each other, selected from halogen, C₁-C₃ alkyl, C₁-C₃ alkoxy, SO₂NH₂, phenyl, heteroaryl,
R is selected from the following groups:
- monocyclic aryl or monocyclic arylalkyl, optionally substituted with one or more substituents, equal to or different from each other, selected from halogen, C₁-C₃ alkyl, SO₂NH₂, C₁-C₃ alkoxy, C₁-C₃ alkylthio, C₁-C₃ haloalkyl, C₁-C₃ haloalkoxy, amino optionally mono- or bisubstituted with a C₁-C₃ alkyl chain,
- bivalent C₄-C₁₀ aliphatic chain, linear or branched, wherein the chain end not linked to the nitrogen atom is linked to W, W being as defined above,
R' is selected from R'₁ and R'₃ with the proviso that Z' is a bivalent C₁-C₃ aliphatic chain, Z" a group selected from C₃-C₁₅ cycloalkyl, saturated or unsaturated heterocycle, monocyclic aryl or monocyclic heteroaryl, v and T' are as defined above.

11. Tricyclic pyrazole compounds according to claims 1-10, wherein:
A is as defined above but excluding the meaning -(CH₂)ᵣ-S(O)ₚ-(CH₂)ₛ-,
B is thiophene, optionally substituted with one or more groups equal to or different from each other, selected from the following: halogen, C₁-C₃ alkyl, C₁-C₃ alkylthio, C₁-C₃ alkoxy, C₁-C₃ haloalkyl, C₁-C₃ haloalkoxy, SO₂NH₂, phenyl, heteroaryl, amino optionally mono- or bisubstituted with a C₁-C₃ alkyl chain, wherein phenyl and heteroaryl are optionally substituted with one or more groups, equal to or different from each other, selected from halogen, C₁-C₃ alkyl, C₁-C₃ alkoxy, SO₂NH₂, phenyl, heteroaryl,
R is selected from the following groups:
- phenyl or benzyl, optionally substituted with one or more substituents, equal to or different from each other, selected from halogen, C₁-C₃ alkyl, SO₂NH₂, C₁-C₃ alkoxy, C₁-C₃ alkylthio, C₁-C₃ haloalkyl, C₁-C₃ haloalkoxy, amino optionally mono- or bisubstituted with a C₁-C₃ alkyl chain,
- bivalent C₄-C₁₀ aliphatic chain, linear or branched, wherein the chain end not linked to the nitrogen atom is linked to W, W being as defined above,
R' is selected between R'₁ and R'₃ with the proviso that:
Z' is selected between -CH₂- or -CH(CH₃)-,
Z" is a group selected from C₃-C₁₅ cycloalkyl, saturated or unsaturated heterocycle, monocyclic aryl, monocyclic heteroaryl,
T' and v are as defined above.

12. Tricyclic pyrazole compounds according to claims 1-11 having the following formulae (X')-(XXXIII'): 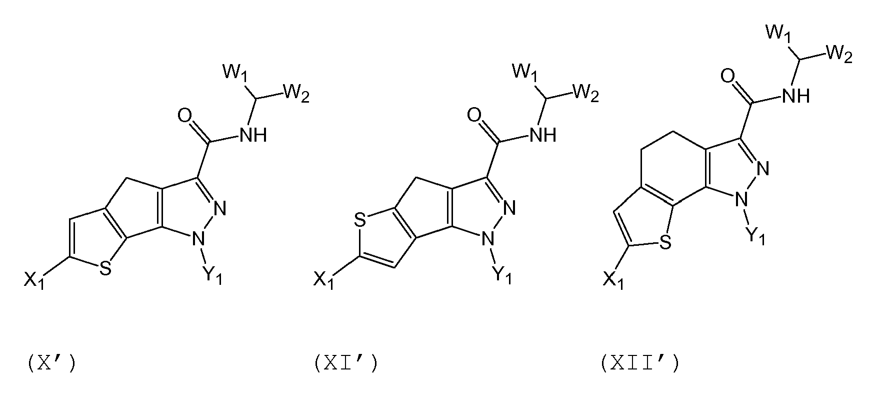 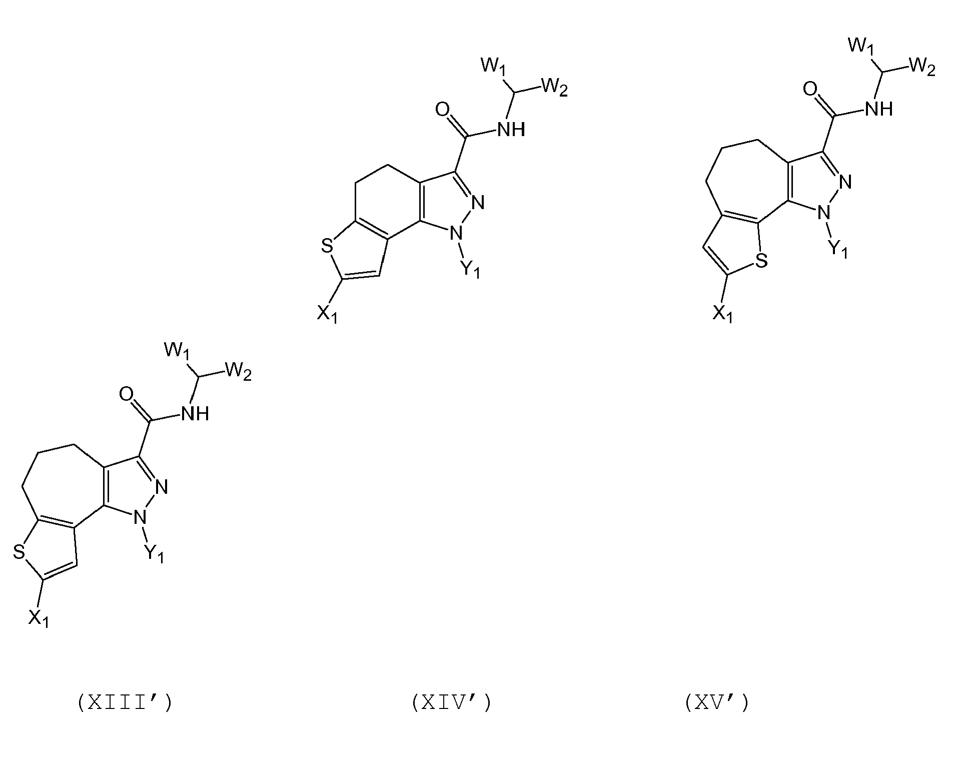 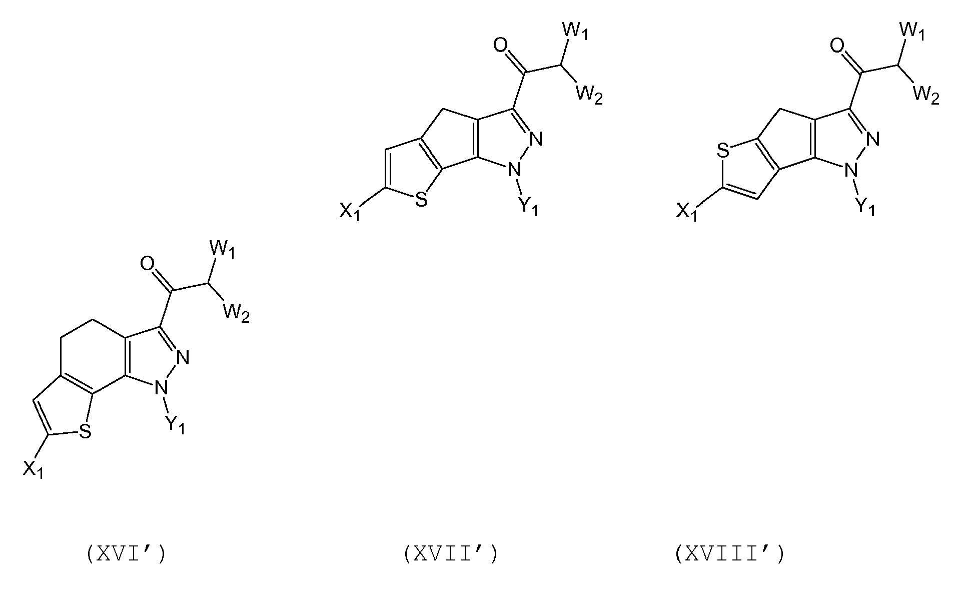 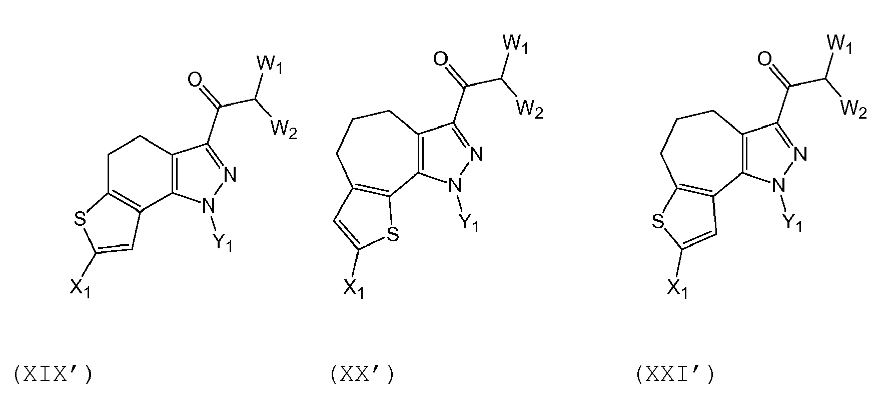 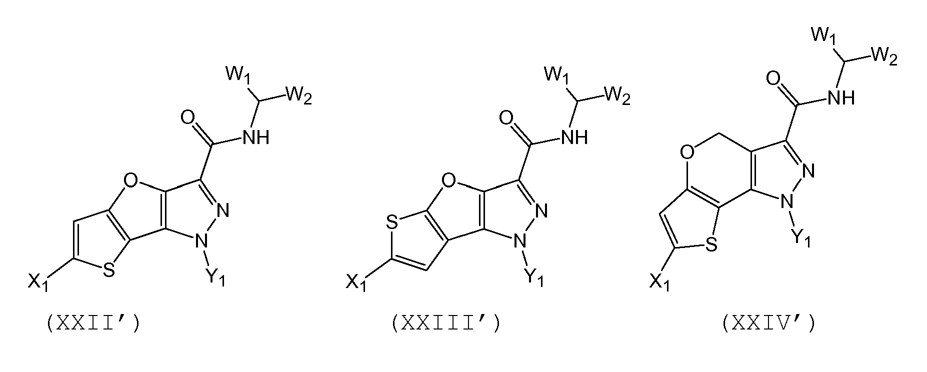 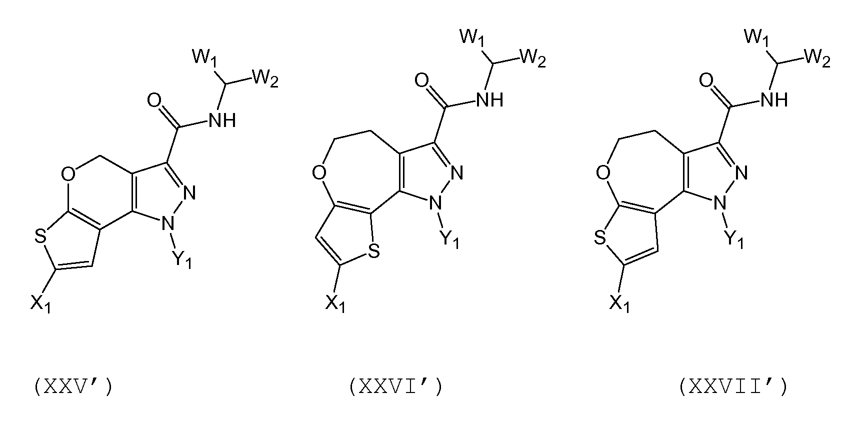 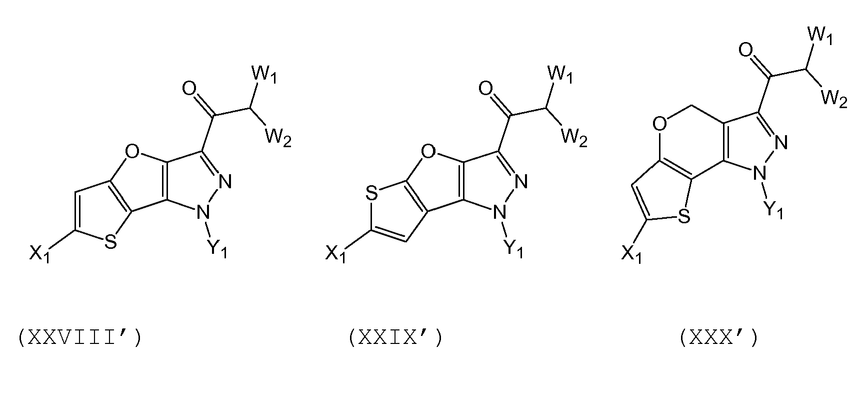 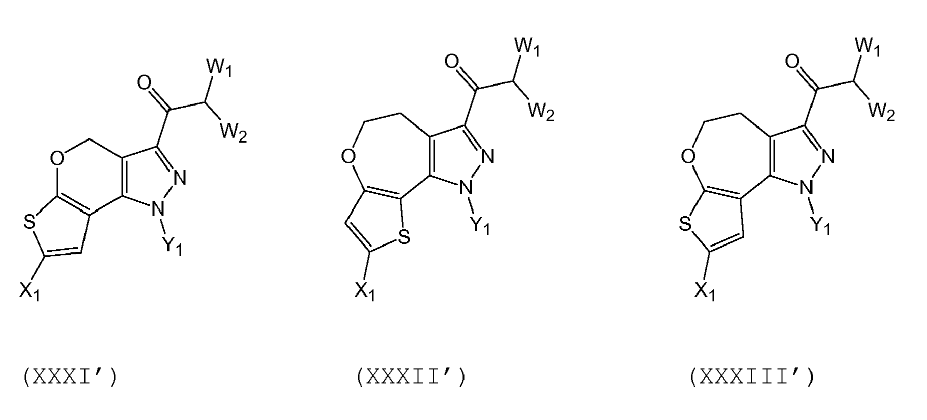 wherein:
Y₁ is selected from: 2,4-dichlorophenyl, 2,4-difluorophenyl, 4-methylbenzyl or 5-chloropentyl,
X₁ is selected from: methyl, chlorine, bromine, fluorine, phenyl, 4-methylphenyl, 4-chlorophenyl, 4-bromophenyl, 4-fluorophenyl or thiophene,
W₁ is hydrogen or methyl,
W₂ is a group selected from the following:

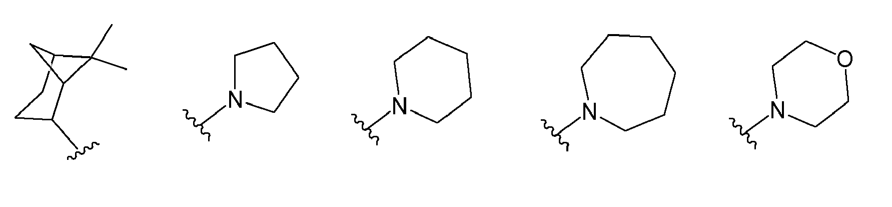

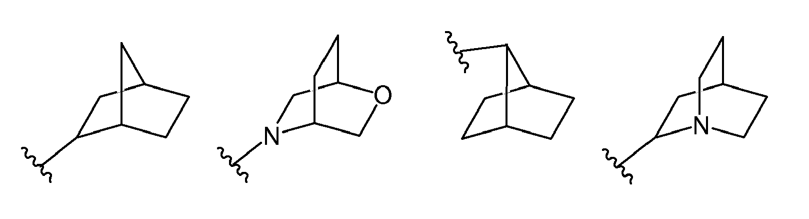

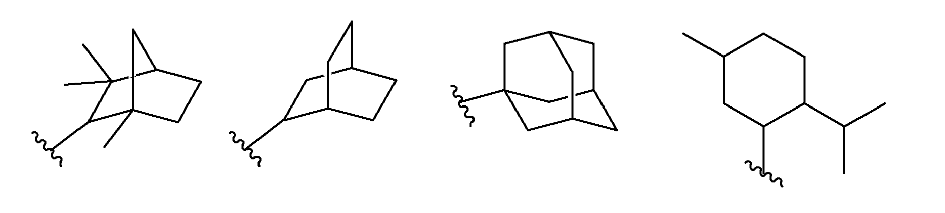

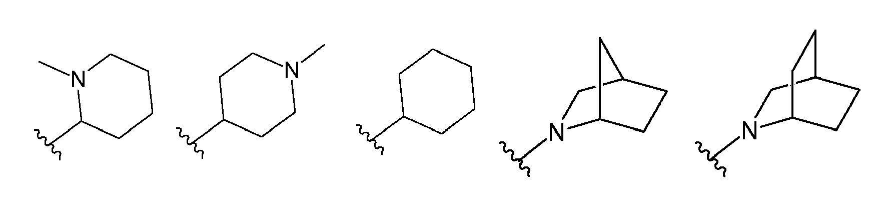

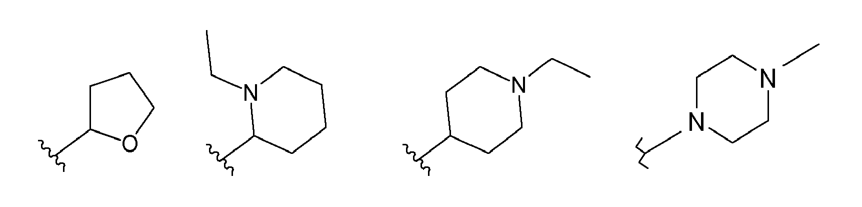

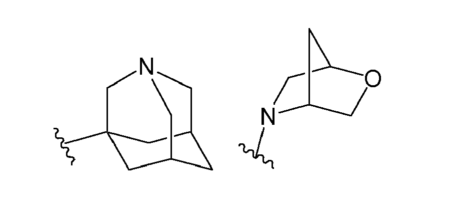

13. Tricyclic pyrazole compounds according to claims 1-12 having the following formulae (XX")-(LXIX"):

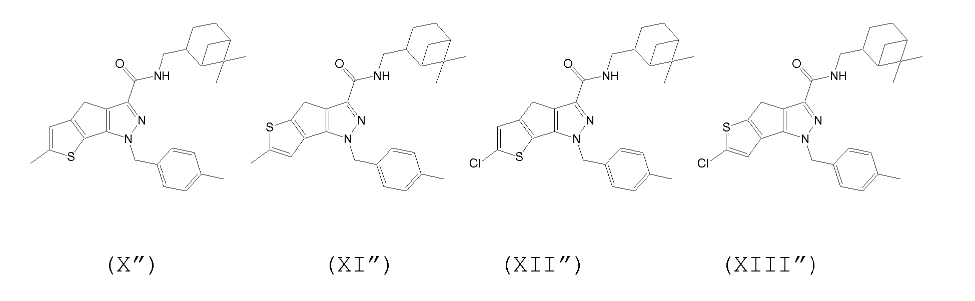

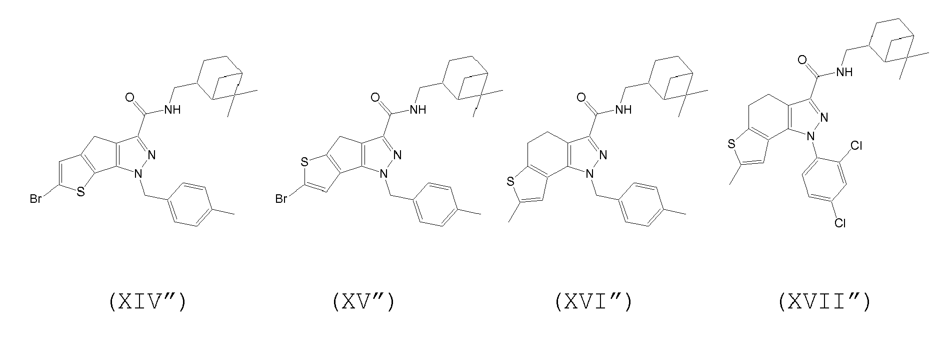

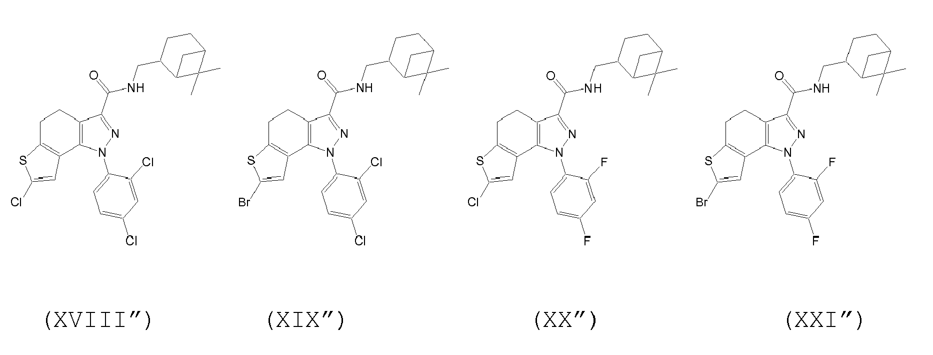

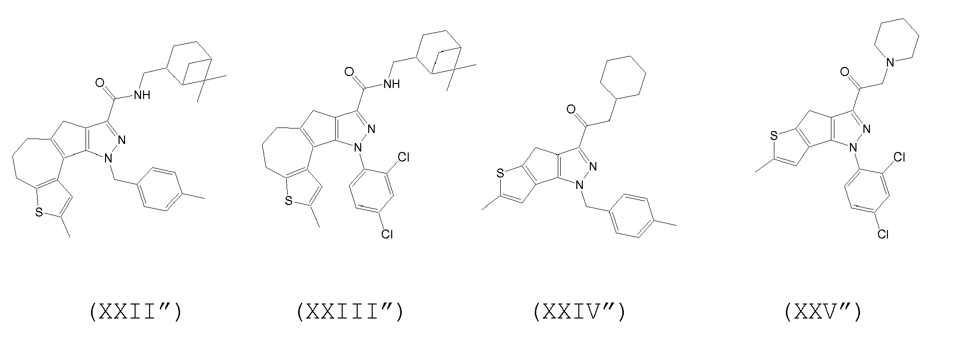

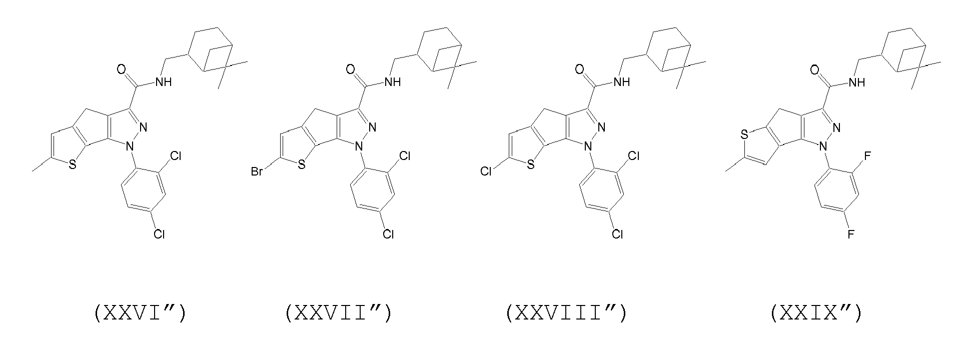

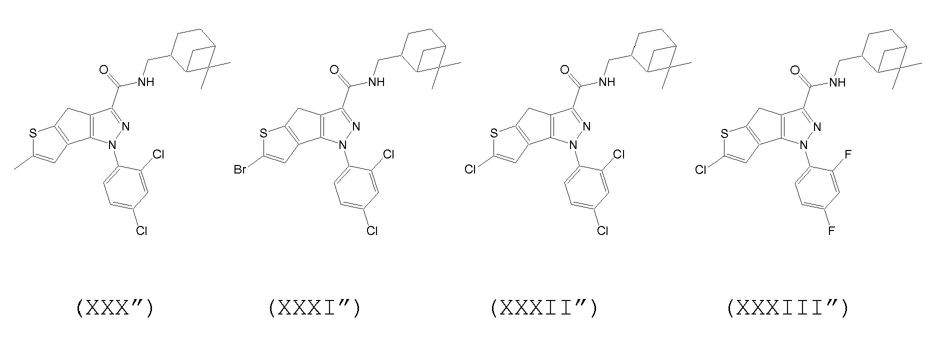

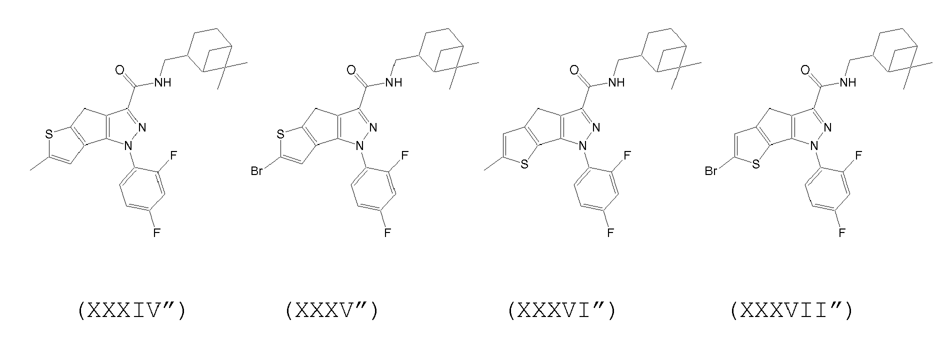

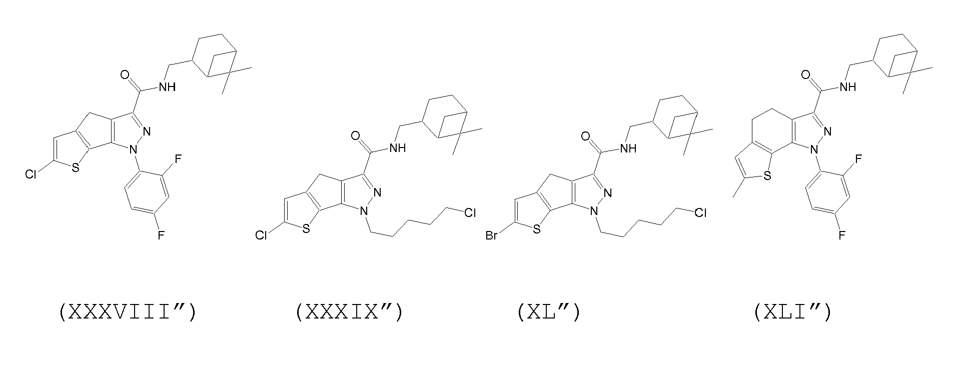

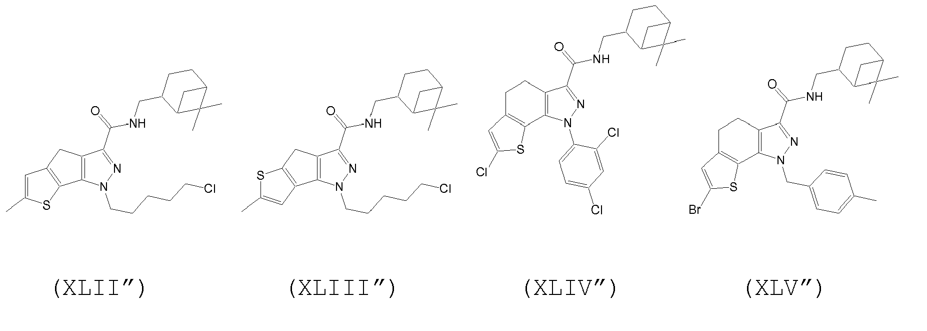

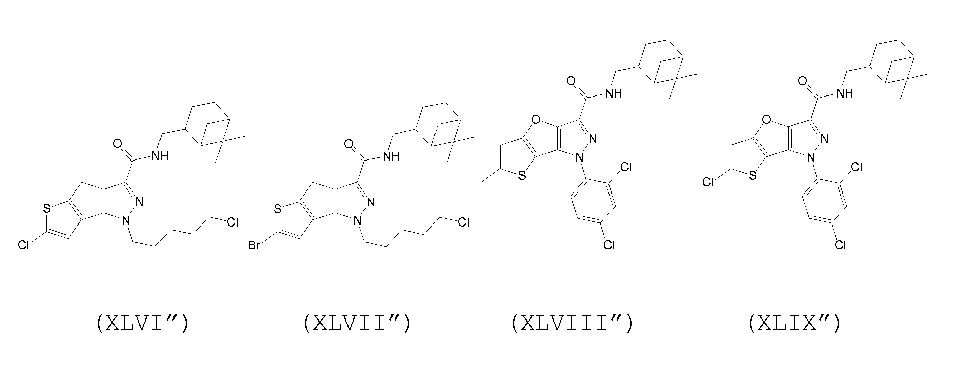

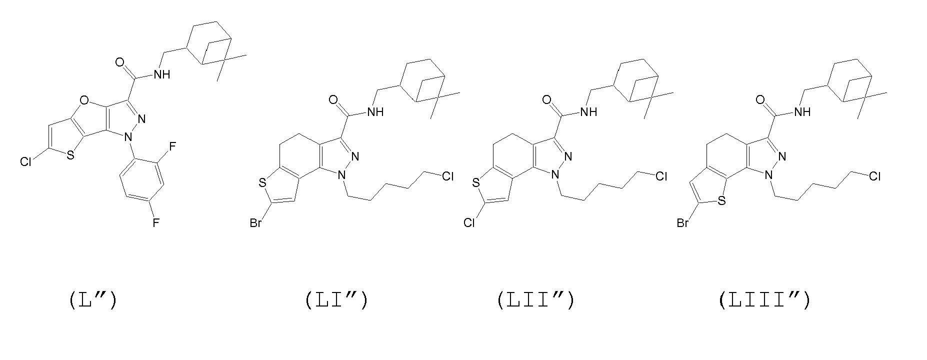

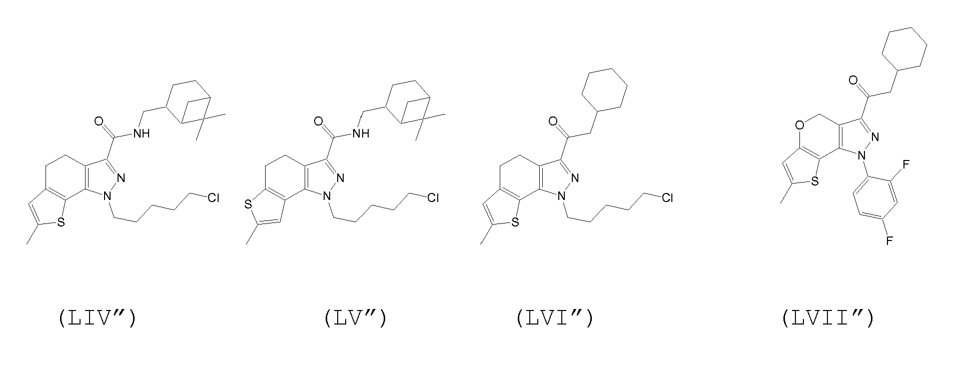

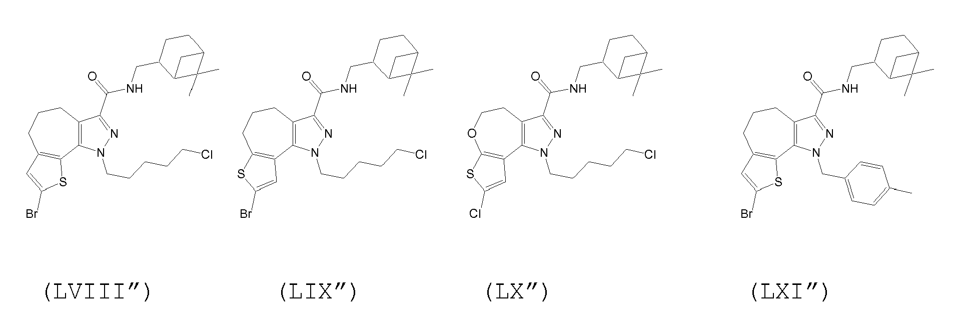

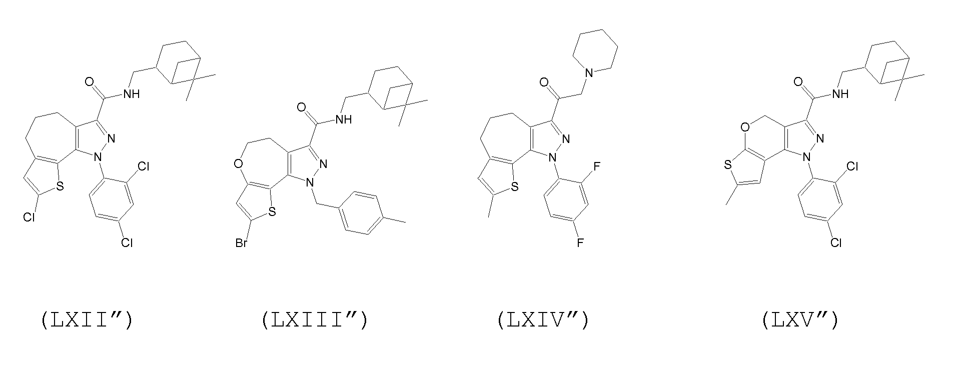

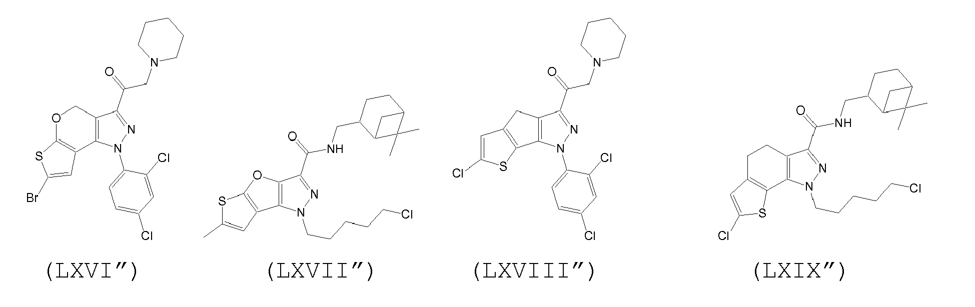

14. Tricyclic pyrazole compounds according to claims 1-13 containing in their structure chiral centres.

15. Tricyclic pyrazole compounds according to claims 1-14, wherein the compounds are geometric isomers or stereoisomers.

16. Tricyclic pyrazole compounds according to claims 1-15 in the form of the corresponding hydrates, solvates and pharmaceutically acceptable salts.

17. A process for preparing the tricyclic pyrazole compounds according to claims 1-16 comprising:
i) synthesis of the acid of general formula (II), or optionally of one reactive derivative thereof: 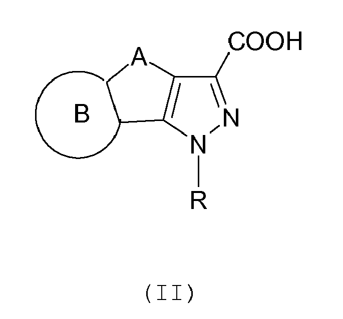 comprising the following steps:
- synthesis of α-hydroxy-γ- ketoesters of formula (IV), wherein A and B are as previously defined, by reacting a compound of formula (III) with sodium alkoxide RONa and diethyloxalate in a C₁-C₃ alcoholic solvent at reflux: 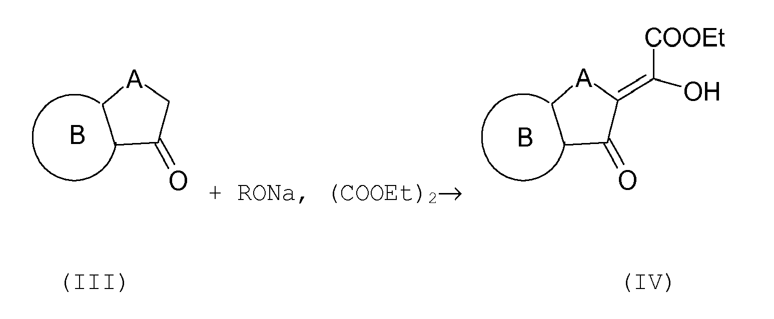
- reaction of the compounds of formula (IV) with an hydrazine of formula (V), wherein R is as previously defined, in an alcoholic solvent or in acetic acid, at reflux, obtaining the tricyclic compound (VI):
(IV) + NH2-NH-R → (V) 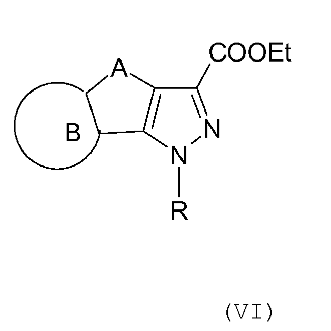
- hydrolysis with alkaline hydroxides in an hydroalcoholic solution of the compound of formula (VI) at reflux, to obtain the acid of formula (II);
- optionally, formation of a reactive derivative of the acid of general formula (II),
ii) when R'=R'₁ as defined above, the compounds of formula (I) are prepared according to one of the following two processes:
- reaction of an ester of the acid of formula (II), with trialkylaluminum and with the hydrochloride salt of an ammine in a solvent inert under the reaction conditions until the ester has completely reacted, and subsequent addition to the reaction mixture of [Z"-(Z')ᵥ]MgBr, wherein Z', v and Z" are as defined above, and following reaction at room temperature, or
- reaction of the acid of formula (II), or a reactive derivative thereof, with a metallorganic salt of formula [Z"-(Z')ᵥ]⁻ Me⁺, wherein Me⁺ is an alkaline metal cation, in a solvent inert under the reaction conditions,
iii) when in formula (I) R'= R'₂ the compounds of formula (I) are obtained by the following reactions:
- formation of the compound of formula (I) wherein R'=R'₁ by using one of the two reactions described in ii);
- reduction at room temperature of the compound of formula (I) wherein R'=R'₁,
iiii) when in formula (I) R'= R'₃ the compounds of formula (I) are prepared by reaction of the acid (II), in the form of a reactive derivative thereof, with a compound of general formula:
H₂N-(Z')ᵥ-T' (VII)
wherein Z', v and T' are as defined above, in a solvent inert under the reaction conditions, by operating at room temperature.

18. A process according to claim 17, wherein in i), when R has the meaning of bivalent C₁-C₁₀ aliphatic chain having at one end the substituent W, compound (VI) is prepared by reacting the compound (IV) with hydrated hydrazine in an alcoholic solvent, at reflux, obtaining the compound (IV'): 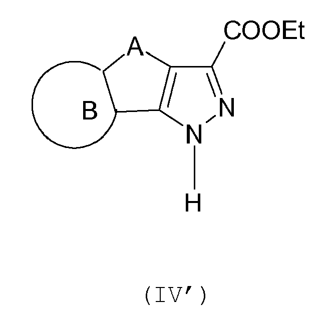 and subsequent alkylation of (IV') by using the compound W-R-T" in an inert solvent at reflux, W and R being as defined above and T" a leaving group.

19. Pharmaceutical compositions comprising the tricyclic pyrazole compounds of claims 1-16.

20. Pharmaceutical compositions according to claim 19 formed of or comprising emulsions or microemulsions.

21. Pharmaceutical compositions according to claim 20, wherein the emulsions or microemulsions comprise the following components (% by weight) the sum of the components of the emulsions or microemulsions being 100%:
S) from 0.01 to 95% of one or more pharmaceutically acceptable compounds, selected from the following classes:
- surfactants selected from non-ionic, anionic, cationic and amphotheric, optionally containing fluorine atoms,
- polymers forming organized structures such as aggregates, micelles, liquid crystals, vesicles, in the liquid in which they are solubilized,
O) from 0 to 95% of one or more oils selected from the following classes of pharmaceutically acceptable compounds:
- esters of C₄-C₃₂ acids, optionally containing one or more unsaturations of ethylene type,
- C₄-C₃₂ acids optionally containing one or more unsaturations of ethylene type, included when the final composition has a pH such that the acid is not converted into the salt thereof,
PA) from 0.001 to 90% of the tricyclic pyrazole derivatives of claims 1-16,
AD) from 0 to 60% by weight of one or more compounds selected from the following classes:
- modifiers of the water and/or oil polarity,
- modifiers of the curvature of the film of component S),
- co-surfactants,
WA) from 0.001 to 99.9% of water or of an aqueous saline solution, optionally buffered.

22. Pharmaceutical compositions according to claims 20-21 having the following composition:
- Component S) from 0.01 to 90%,
- Component O) from 0 to 90%,
- Component PA) from 0.001 to 50%,
- Component AD) from 0 to 30%,
- Component WA) from 0.1 to 99.9%,
the sum of the components being 100%.

23. Pharmaceutical compositions according to claims 20-22 having the following composition:
- Component S) from 0.01 to 80%,
- Component O) from 0 to 70%,
- Component PA) from 0.05 to 40%,
- Component AD) from 0 to 20%,
- Component WA) from 10 to 99.9%,
the sum of the components being 100%.

24. Pharmaceutical compositions according to claims 20-23 having the following composition:
- Component S) from 0.01 to 70%,
- Component O) from 0 to 50%,
- Component PA) from 0.05 to 30%,
- Component AD) from 0 to 15%,
- Component WA) from 20 to 99.9%,
the sum of the components being 100%.

25. A process for preparing the pharmaceutical compositions in the form of microemulsions comprising the following steps:
| | |
|---|---|
| (IP) | optionally, solubilization of component PA) in the oil, |
| (IIP) | addition of component S) to component PA) or to the corresponding oily solution obtained in (IP), |
| (IIIP) | optionally, addition of component AD) to the liquid phase obtained in (IIP), |
| (IVP) | addition, under stirring, of water or of aqueous saline solution to the liquid phases obtained in (IIP) or in the optional step (IIIP), obtaining a limpid solution. |

26. Pharmaceutical formulations according to claim 19 for oral administration comprising (% by weight):
0.5-20% of one or more tricyclic pyrazole compounds of claims 1-16,
0.05-0.5% of sodium alkylsulphate or another surfactant, 2.5-10% of a disgregating agent,
the difference to 100% being conventional adjuvants used in oral dosage forms.

27. Pharmaceutical compositions according to claim 21 for oral and intraocular administration comprising one or more tricyclic pyrazole compounds of claims 1-16 together with hydroxypropylmethylcellulose, the latter being in the core of the pharmaceutical formulation and/or in the shell, when the latter is present in the formulation.

28. Tricyclic pyrazole derivatives of claims 1-16 for use as medicament.

29. Use of the tricyclic pyrazole derivatives of claims 1-16 and of the pharmaceutical compositions thereof for preparing a medicament for the treatment in mammals and in an individual of diseases and disorders involving the CB1 and/or CB2 receptors.

30. Use according to claim 29, wherein the diseases and disorders are the following: diseases in which immune system cells are involved, immune disorders, osteoporosis, renal ischaemia, pain, neuropathic pain, post-surgery pain, inflammatory conditions, lateral amyotrophic schlerosis, diseases associated with organ transplants, therapies for preventing allogenic transplant rejection, treatment of transplant rejection in patients undergoing other immunosuppressive therapies, treatment and prophylaxis of GVHD (Graft Versus Host Disease), lupus systemic erithematous, ankilosant spondylitis, reumathoid polyarthritis, haemolytic autoimmune anaemia, Behcet disease, Sjögren syndrome, undifferentiated spondylarthritis, reactive arthritis, dermatomyositis.

31. Use according to claim 29, wherein the diseases and the disorders are the following: glaucoma or ocular hypertension, pulmonary diseases, asthma, chronic bronchitis, allergies, allergic reactions, allergic rhinitis, contact dermatitis, allergic conjunctivitis, inflammations, arthitis, pain, anxiety, behaviour disorders, delirium conditions, psychotic disorders in general, schizophrenia, depression, treatment of drug and/or alcohol abuse, tabagism, vomit, nausea, vertigoes, neuropathies, hemicrania, stress, psychosomatic origin diseases, epilepsy, Tourette syndrome, Parkinson disease, Huntington disease, Alzheimer disease, senile dementia, in the case of recognition disorders and memory loss, appetite disorders, obesity, bulimia, pathologies of the gastrointestinal tract and of the bladder, cardiovascular diseases, urinary disorders, fertility and erectile disorders, neuroinflammatory pathologies, multiple sclerosis, Guillain-Barré syndrome, viral encefalitis, syndrome associated to demineralization, osteoporosis, in reducing metabolic and/or cardiovascular risk factors, also in patients with metabolic syndrome and/or dyslipidemia and in patients with type 2 diabetes, eye inflammatory conditions, eye autoimmune diseases, uveitis, uveoretinitis and retina neurodegeneration.

32. Radiolabelled tricyclic pyrazole compounds of claims 1-16.

33. Use of the compounds of claim 32 for identifying and labelling CB1 or CB2 cannabinoidergic receptors in mammals and in human beings.
